(19) Europäisches Patentamt
European Patent Office
Office européen des brevets

(11) Veröffentlichungsnummer: **0 497 851 B1**

(12) **EUROPÄISCHE PATENTSCHRIFT**

(45) Veröffentlichungstag der Patentschrift: **04.01.95**

(51) Int. Cl.[6]: **C07D 239/34**, C07D 239/52, C07D 239/30, C07D 401/12, C07D 403/12, C07D 405/12, C07D 409/12, A01N 43/54, C07D 213/34

(21) Anmeldenummer: **90916278.6**

(22) Anmeldetag: **18.10.90**

(86) Internationale Anmeldenummer: **PCT/EP90/01768**

(87) Internationale Veröffentlichungsnummer: **WO 91/06541 (16.05.91 91/11)**

# (54) SULFONIERTE HETEROCYCLISCHE CARBOXAMIDE ALS HERBIZIDE, WACHSTUMSREGULATOREN ODER FUNGIZIDE.

(30) Priorität: **24.10.89 DE 3935277**

(43) Veröffentlichungstag der Anmeldung: **12.08.92 Patentblatt 92/33**

(45) Bekanntmachung des Hinweises auf die Patenterteilung: **04.01.95 Patentblatt 95/01**

(84) Benannte Vertragsstaaten: **DE FR GB**

(56) Entgegenhaltungen:
**EP-A- 0 162 538**
**EP-A- 0 353 640**
**DE-A- 1 445 155**
**DE-A- 3 243 590**
**US-A- 2 876 224**

(73) Patentinhaber: **Hoechst Schering AgrEvo GmbH**
**Gerichtstrasse 27**
**D-13342 Berlin (DE)**

(72) Erfinder: **ORT, Oswald**
**Gundelhardtstrasse 41**
**D-6233 Kelkheim (DE)**
Erfinder: **WILLMS, Lothar**
**Lindenstrasse 17**
**D-5416 Hillscheid (DE)**
Erfinder: **BAUER, Klaus**
**Doorner Strasse 53d**
**D-6450 Hanau (DE)**
Erfinder: **BIERINGER, Hermann**
**Eichenweg 26**
**D-6239 Eppstein (DE)**
Erfinder: **SCHULZ, Arno**
**Stauffenstrasse 22**
**D-6234 Hattersheim am Main (DE)**
Erfinder: **SACHSE, Burkhard**
**An der Ziegelei 30**
**D-6233 Kelkheim (DE)**
Erfinder: **BRAUN, Peter**
**Pfarrer-Dorn-Strasse 13**
**D-6500 Mainz (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Es ist bereits bekannt, daß bestimmte sulfonylierte bi- oder tricyclische heteroaromatische Carbonsäureamide herbizide und wachstumsregulatorische Eigenschaften besitzen (EP-A-244 166). Sulfonylierte monocyclische Pyridincarbonsäureamide werden als Fungizide und Mikrobiozide in der Landwirtschaft beschrieben (Chem. Abstr. 108 (19): 167298p; Chem. Abstr. 108 (15): 131590p). Eine Herbizid-Wirkung dieser Verbindungen ist nicht bekannt geworden.

Es wurde bereits vorgeschlagen, sulfonierte 2-Pyrimidinylcarbonsäureamide und 2-Triazinylcarbonsäureamide als Herbizide und Wachstumsregulatoren einzusetzen (siehe Deutsche Patentanmeldung P 38 26 230.4 oder EP-A-353 640).

Es wurde nun gefunden, daß sulfonierte 4-Pyrimidinylcarbonsäureamide vorteilhafte herbizide, wachstumsregulatorische und fungizide Eigenschaften besitzen.

Gegenstand der vorliegenden Erfindung sind Verbindungen der Formel I oder deren Salze

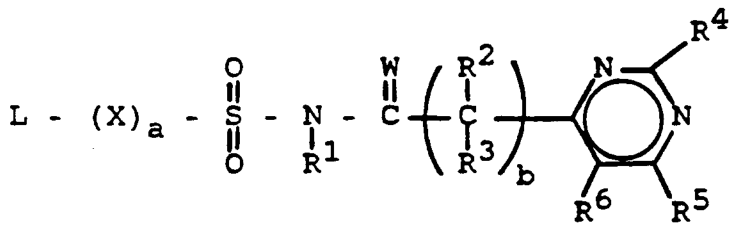

(I)

worin

| | |
|---|---|
| $R^1$ | Wasserstoff, $(C_1$-$C_4)$Alkyl, $(C_2$-$C_6)$Alkenyl oder $(C_2$-$C_6)$Alkinyl; |
| $R^2$, $R^3$ | unabhängig voneinander Wasserstoff, $(C_1$-$C_3)$Alkyl oder Phenyl; |
| W | O, S, $NR^7$ oder $NOR^7$; |
| X | $CHR^2$, O, $NR^7$ oder $NOR^7$; |
| $R^4$, $R^5$ | unabhängig voneinander Wasserstoff, Hydroxy, Halogen, $(C_1$-$C_6)$Alkyl, $(C_1$-$C_6)$Alkoxy oder $(C_1$-$C_6)$Alkylthio, wobei die drei vorgenannten Reste ein- oder mehrfach durch Halogen oder ein- oder zweifach durch $(C_1$-$C_4)$Alkoxy oder $(C_1$-$C_4)$Alkylthio substituiert sein können; $-NR^8R^9$, $(C_3$-$C_6)$Cycloalkyl, $-OCHR^8CO_2R^{10}$, $(C_2$-$C_5)$Alkenyl, $(C_2$-$C_4)$Alkinyl, $(C_3$-$C_5)$Alkenyloxy oder $(C_3$-$C_5)$Alkinyloxy; |
| $R^6$ | Wasserstoff, Halogen, $(C_1$-$C_3)$Alkyl, $(C_1$-$C_3)$Alkoxy, -CN, $-NO_2$, $-CO-R^{11}$, $CO_2R^{12}$, $(C_1$-$C_3)$-Alkylthio, $-SO-R^{12}$, $-SO_2-R^{12}$ oder $-CO-NR^8R^9$; |
| $R^7$ | Wasserstoff, $(C_1$-$C_3)$Alkyl, $(C_1$-$C_3)$Haloalkyl oder Phenyl; |
| $R^8$ | Wasserstoff oder $(C_1$-$C_4)$Alkyl oder $R^8$ und $R^9$ zusammen $-(CH_2)_2(CH_2)_c(CH_2)_2-$ oder $-(CH_2)_2O(CH_2)_2-$; |
| $R^9$ | Wasserstoff, $(C_1$-$C_4)$Alkyl, $(C_1$-$C_4)$Alkoxy, $(C_2$-$C_4)$Alkenyl oder $R^8$ und $R^9$ zusammen $-(CH_2)_2(CH_2)_c(CH_2)_2-$ oder $-(CH_2)_2O(CH_2)_2-$; |
| $R^{10}$ | Wasserstoff, $(C_1$-$C_4)$Alkyl, das unsubstituiert oder ein- oder mehrfach durch Halogen oder ein- bis zweifach durch CN, $CO_2-R^{12}$, $-NR^8R^9$, $OR^{12}$ oder $Si(CH_3)_3$ substituiert ist; $(C_3$-$C_4)$Alkinyl oder $(C_3$-$C_4)$Alkenyl, wobei die zwei vorgenannten Reste unsubstituiert oder durch $CH_3$ oder $-Si(CH_3)_3$ substituiert sind; $(C_3$-$C_6)$Cycloalkyl, das unsubstituiert oder durch $(C_1$-$C_4)$Alkyl oder $(C_1$-$C_4)$Alkoxy substituiert ist; oder $Si(CH_3)_3$; |
| $R^{11}$ | Wasserstoff, $(C_1$-$C_4)$Alkyl, $(C_1$-$C_4)$Alkyl, das ein- oder mehrfach durch Halogen oder $(C_1$-$C_3)$Alkoxy substituiert ist; $(C_3$-$C_6)$Cycloalkyl, das unsubstituiert oder durch Halogen, wie z. B. F, Cl und Br, oder $CH_3$ ein- oder mehrfach substitutiert ist, $(C_2$-$C_4)$Alkenyl, $(C_2$-$C_4)$-Alkinyl; |
| $R^{12}$ | $(C_1$-$C_3)$Alkyl; |
| L | einen hetero- oder isocyclischen Rest der Formeln (L1) bis (L5) |

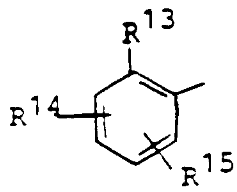

(L1)

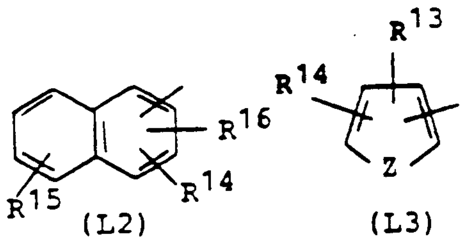

(L2) (L3)

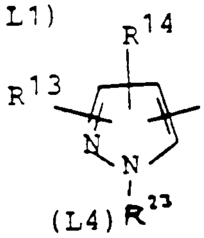

(L4)

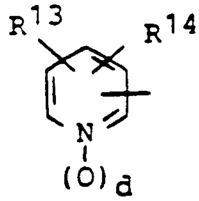

(L5)

| | |
|---|---|
| $R^{13}$ | Wasserstoff, Halogen, $NO_2$, CN, $(C_1\text{-}C_4)$Alkyl, das unsubstituiert oder ein- oder mehrfach durch Halogen, wie z. B. F, Cl und Br, oder einfach durch CN, $OCH_3$ oder $SCH_3$ substituiert ist; $(C_2\text{-}C_4)$Alkenyl, das unsubstituiert oder ein- oder mehrfach durch Halogen, wie z. B. F, Cl und Br, oder einfach durch $OCH_3$ substituiert ist; $(C_2\text{-}C_4)$Alkinyl, das unsubstituiert oder ein- oder mehrfach durch Halogen, wie Z. B. F, Cl und Br, oder einfach durch $OCH_3$ oder $Si(CH_3)_3$ substituiert ist; $(C_3\text{-}C_6)$Cycloalkyl, das unsubstituiert oder ein- oder mehrfach durch Halogen, wie z. B. F und Cl, oder $CH_3$ substituiert ist; $-CO\text{-}R^{11}$, $-OCH_2CH_2OR^{11}$, -OH, $-C(R^{11})(OR^{17})(OR^{18})$; $-CO_2R^{10}$, $-CO\text{-}NR^8R^9$, $-N_3$, $SO_2NR^8R^9$, $-SO_3R^{19}$, $-OSO_2R^{20}$; Phenyl, das unsubstituiert oder ein- oder mehrfach durch Halogen, die z. B. F, Cl und Br, $CH_3$ oder $OCH_3$ substituiert ist; $-E\text{-}R^{21}$ oder $-(CH_2)_e\text{-}G$; |
| $R^{14}$ | Wasserstoff, Halogen; CN, $NO_2$, $(C_1\text{-}C_4)$-Alkyl, das unsubstituiert oder ein- oder mehrfach durch Halogen oder ein- bis zweifach durch $-CO_2R^{10}$, $-SO_2NR^8R^9$, $-NR^8R^9$, $(C_1\text{-}C_2)$-Alkoxy, $-E\text{-}R^{22}$, $(C_1\text{-}C_2)$Haloalkoxy, $(C_1\text{-}C_2)$Alkylthio, $(C_1\text{-}C_2)$Haloalkylthio, -CN, -OH oder SH substituiert ist; $-CO_2R^{10}$, $-SO_2NR^8R^9$, $-NR^8R^9$ oder $-E\text{-}R^{22}$; |
| $R^{15}$ | Wasserstoff, $(C_1\text{-}C_4)$Alkyl, $(C_1\text{-}C_4)$Haloalkyl, $(C_2\text{-}C_4)$Alkenyl, Phenyl oder Phenyl, das ein- oder mehrfach durch Halogen oder $-E\text{-}R^{22}$ substituiert ist; $-E\text{-}R^{22}$ oder Halogen; |
| $R^{16}$ | Wasserstoff, $(C_1\text{-}C_4)$Alkyl, $(C_1\text{-}C_4)$Haloalkyl, Halogen, $-CO_2R^{10}$, $-SO_2NR^8R^9$, $-OSO_2R^{20}$, $-E\text{-}R^{22}$, -CN oder $-NO_2$; |
| E, Z | unabhängig voneinander O oder $S(O)_f$; |
| a, b, c, d, e | unabhängig voneinander 0 oder 1; |
| f | 0, 1 oder 2; |
| $R^{17}$, $R^{18}$ | unabhängig voneinander $(C_1\text{-}C_4)$Alkyl, oder $R^{17}$ und $R^{18}$ zusammen $-CH_2CH_2\text{-}$, $-CH_2OCH_2\text{-}$ oder $-CH_2\text{-}C(CH_3)_2CH_2\text{-}$; |
| $R^{19}$ | $(C_1\text{-}C_4)$Alkyl oder $(C_1\text{-}C_4)$Haloalkyl, |
| $R^{20}$ | $(C_1\text{-}C_4)$Alkyl, $-NR^8R^9$ oder $(C_1\text{-}C_4)$Haloalkyl, |
| $R^{21}$ | $(C_1\text{-}C_4)$Alkyl, $(C_1\text{-}C_4)$Haloalkyl, $(C_2\text{-}C_4)$Alkoxyalkyl, $(C_2\text{-}C_4)$Alkenyl, $(C_3\text{-}C_4)$Alkinyl, Phenyl oder Phenyl, das ein- oder mehrfach durch Halogen $(C_1\text{-}C_3)$Alkyl, $(C_1\text{-}C_3)$Alkoxy oder $(C_1\text{-}C_3)$Haloalkyl substituiert ist; $(C_2\text{-}C_4)$Haloalkenyl; |
| $R^{22}$ | $(C_1\text{-}C_4)$Alkyl oder $(C_1\text{-}C_4)$Alkyl, das ein- oder mehrfach durch Halogen, wie z.B. F, Cl, oder einfach durch $OR^{19}$ substituiert ist; |
| $R^{23}$ | Wasserstoff, $(C_1\text{-}C_4)$Alkyl, das unsubstituiert oder ein- oder mehrfach durch Halogen oder einfach durch Phenyl substituiert ist, $(C_2\text{-}C_4)$Alkenyl, Phenyl oder Phenyl, das ein- oder mehrfach durch Halogen, $(C_1\text{-}C_4)$Alkyl, $(C_1\text{-}C_4)$Haloalkyl, $-NO_2$, -CN oder $(C_1\text{-}C_4)$Alkoxy substituiert ist, |
| G | einen heterocyclischen Rest aus der Gruppe der Reste der Formeln (G1)-(G25); |

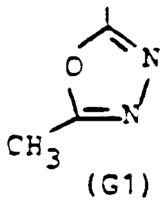
(G1)

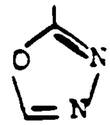
(G2)

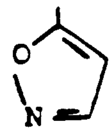
(G3)

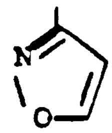
(G4)

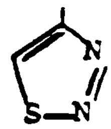
(G5)

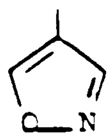
(G6)

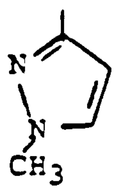
(G7)

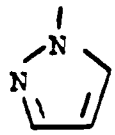
(G8)

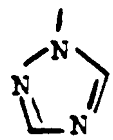
(G9)

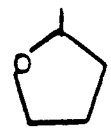
(G10)

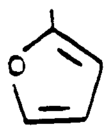
(G11)

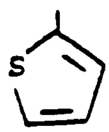
(G12)

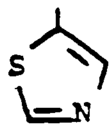
(G13)

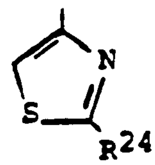
(G14)

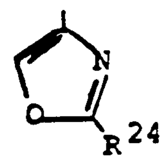
(G15)

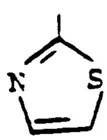
(G16)

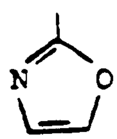
(G17)

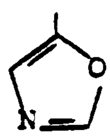
(G18)

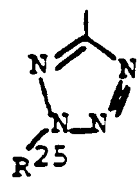
(G19)

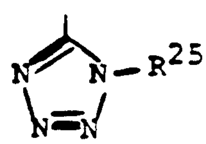
(G20)

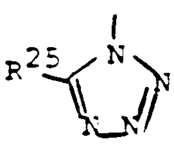
(G21)

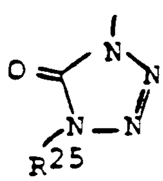
(G22)

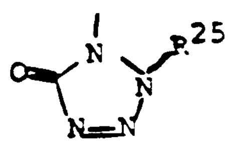
(G23)

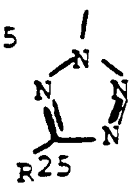
(G24)

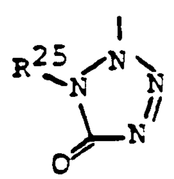
(G25)

und

$R^{24}$ Wasserstoff oder $(C_1$-$C_4)$Alkyl und

$R^{25}$ Wasserstoff, $(C_1$-$C_3)$Alkyl oder $(C_2$-$C_4)$Alkenyl

bedeuten.

Die Verbindungen der Formel I können ein oder mehrere Chiralitätszentren enthalten und liegen dann als Diastereomeren- oder Enantiomerengemische vor. Die Erfindung umfaßt sowohl die reinen Enantiomeren oder Diastereomeren als auch deren Gemische.

Die Verbindungen der Formel I können im Falle $R^1$ = H Salze bilden, bei denen der Wasserstoff der $-SO_2$-NH-Gruppe durch ein für die Landwirtschaft geeignetes Kation ersetzt wird. Diese Salze sind im

allgemeinen Metall-, insbesondere Alkali-, Erdalkali-, gegebenenfalls alkylierte Ammonium- oder organische Aminsalze. Sie werden vorzugsweise in inerten Lösungsmitteln wie z. B. Wasser, Methanol oder Aceton bei Temperaturen von 0 - 100 °C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Die Verbindungen der Formel (I), welche stickstoffhaltige Heterocyclen enthalten, können außerdem mit anorganischen oder organischen Säuren Salze bilden. Geeignete Säuren zur Herstellung der erfindungsgemäßen Säureadditionssalze sind z. B. anorganische Säuren wie HCl, $H_2SO_4$ und $H_3PO_4$ oder organische Säuren wie Ameisensäure, Essigsäure, Palmitinsäure, Trichloressigsäure oder 4-Toluolsulfonsäure.

In den obengenannten Definitionen bedeutet der Ausdruck "Alkyl", alleinstehend oder in zusammengesetzten Worten wie "Alkylthio", "Haloalkyl", "Alkylamino" oder "Bisalkylamino" jeweils geradkettiges oder verzweigtes Alkyl.

Ebenso bedeutet Alkenyl gerad- oder verzweigtkettiges Alkenyl, z.B. 1-Propenyl, 2-Propenyl, 3-Propenyl. Alkinyl bedeutet gerad- oder verzweigtkettiges Alkinyl z.B. Ethinyl, 1-Propinyl, 2-Propinyl oder die verschiedenen Butinyl-Isomere. Alkylsulfonyl bedeutet beispielsweise Methylsulfonyl, Ethylsulfonyl oder die verschiedenen Propylsulfonyl-Isomere.

Der Ausdruck "Halogen" allein oder in zusammengesetzten Wörtern wie "Haloalkyl" bedeutet Fluor, Chlor, Brom oder Jod. Weiterhin bedeutet in zusammengesetzten Wörtern wie "Haloalkyl", daß besagtes Alkyl teilweise oder vollständig halogeniert ist. Beispiele für Haloalkyl sind $CHF_2$, $CH_2CH_2F$, $CF_2CF_3$ und $CH_2CHFCl$.

Bevorzugt von den Verbindungen der allgemeinen Formel I sind solche, worin

| | |
|---|---|
| L | einen Rest der Formeln (L1), (L3), (L4) oder (L5); |
| X | $CH_2$, $CH(CH_3)$, O, NH, $NCH_3$, $NC_2H_5$, $NOCH_3$, insbesondere $CH_2$, O oder NH; |
| W | Sauerstoff; |
| $R^1$ | Wasserstoff; |
| $R^2$, $R^3$ | unabhängig voneinander Wasserstoff oder $(C_1-C_3)$Alkyl, insbesonders Wasserstoff; |
| $R^4$, $R^5$ | unabhängig voneinander Wasserstoff, $(C_1-C_2)$Alkyl, $(C_1-C_2)$Alkoxy, $-OCH_2OCH_3$, $-CH_2OCH_3$, F, Cl, Br, I, $-CH_2OCH_2CH_3$, $-NHCH_3$, $-N(CH_3)_2$, $-N(CH_3)OCH_3$, $-CF_3$, $-SCH_3$, $-CH(OCH_3)_2$, $-OCH_2CH=CH_2$, $-OCH_2C{\equiv}CH$, $-OCH_2CH_2OCH_3$, $-CH_2SCH_3$, $-OCHF_2$, $-SCHF_2$, Cyclopropyl, $-C{\equiv}CH$ oder $-C{\equiv}C-CH_3$; |
| $R^6$ | Wasserstoff, Halogen, $-CH_3$, $-OCH_3$, $-NO_2$, -CN, -CHO, $-CO_2CH_3$, $CO_2C_2H_5$ oder $-SCH_3$, insbesondere Wasserstoff, Halogen, $-NO_2$, -CN, -CHO, $-CO_2CH_3$ oder $-CO_2C_2H_5$; |
| $R^8$ | Wasserstoff oder $-CH_3$ oder $R^8$ und $R^9$ zusammen $-(CH_2)_4-$, $-(CH_2)_5-$ oder $-(CH_2)_2O(CH_2)_2-$; |
| $R^9$ | $-CH_3$, $-CH_2CH_3$, $-OCH_3$ oder $R^8$ und $R^9$ zusammen $-(CH_2)_4-$, $-(CH_2)_5-$ oder $-(CH_2)_2O(CH_2)_2-$; |
| $R^{10}$ | $(C_1-C_3)$Alkyl, $(C_3)$Alkenyl, $(C_3)$Alkinyl, $-CH_2CH_2F$, $-CH_2CH_2Cl$, $-CH_2CH_2OCH_3$, $-CH_2CH_2Si(CH_3)_3$ oder Cyclopropylmethyl; |
| $R^{11}$ | $(C_1-C_3)$Alkyl, Wasserstoff, Cyclopropyl oder $(C_3)$Alkenyl; |
| $R^{12}$ | $-CH_3$, $-CH_2CH_3$, $-CH_2CH_2CH_3$ oder $-CH(CH_3)_2$; |
| $R^{13}$ | Halogen, $-NO_2$, -CN, $(C_1-C_3)$Alkyl, das unsubstituiert oder durch F, Cl, Br, CN, $OCH_3$ oder $SCH_3$ substituiert ist; $(C_3)$Alkenyl, das unsubstituiert oder durch F, Cl oder Br substituiert ist, $(C_3)$Alkinyl, Cyclopropyl, das unsubstituiert oder durch F, Cl oder $CH_3$ substituiert ist; $-C(O)R^{11}$, $-OCH_2CH_2OR^{11}$, -OH, $C(R^{11})(OR^{17})(OR^{18})$, $-CO_2R^{10}$, $-CO-NR^8R^9$, $-N_3$, $-SO_2NR^8R^9$, $-OSO_2R^{20}$, $-E-R^{21}$ oder $-(CH_2)_eG$; |
| $R^{14}$ | Wasserstoff, Halogen, -CN, $-NO_2$, $-CH_3$, $-CF_3$, $-E-R^{22}$, oder $(C_1-C_2)$Alkoxy-$(C_1-C_2)$alkyl, $-CO_2R^{10}$ oder $SO_2NR^8R^9$; |
| $R^{15}$ | Wasserstoff; |
| $R^{17}$, $R^{18}$ | unabhängig voneinander $(C_1-C_2)$Alkyl oder $R^{17}$ und $R^{18}$ zusammen $-CH_2CH_2-$; |
| $R^{20}$ | $(C_1-C_3)$Alkyl, $(C_1-C_3)$Haloalkyl; |
| $R^{21}$ | $(C_1-C_3)$Alkyl, $(C_1-C_3)$Haloalkyl, $(C_2-C_3)$Alkoxyalkyl, Allyl, Propargyl, $(C_2-C_3)$-Haloalkenyl; |
| $R^{22}$ | $(C_1-C_2)$Alkyl, das unsubstituiert oder durch F, Cl oder $OCH_3$ substituiert ist; |
| $R^{23}$ | Wasserstoff, $(C_1-C_3)$Alkyl, Phenyl oder Phenyl, das ein- oder mehrfach durch Halogen, $-NO_2$, $(C_1-C_3)$Alkyl, $(C_1-C_3)$Haloalkyl oder $(C_1-C_3)$Alkoxy substituiert ist; |
| $R^{24}$ | Wasserstoff oder $(C_1-C_3)$Alkyl; |

| | |
|---|---|
| $R^{25}$ | Wasserstoff oder $(C_1-C_3)$Alkyl; |
| Z | S; |
| d | 0 und |
| e | 0 bedeuten |
| und E, a, b, f und G | wie oben definiert sind. |

Für $R^4$ und $R^5$ sind unabhängig voneinander besonders bevorzugt die Reste Wasserstoff, $-CH_3$, $-OCH_3$, $-CH_2CH_3$, $-OCH_2CH_3$, $-OCHF_2$, $-OCH_2OCH_3$, $-CH_2OCH_3$, $-NHCH_3$, $-CH(OCH_3)_2$, -Cl oder Cyclopropyl.

Gegenstand der vorliegenden Erfindung sind auch Verfahren zur Herstellung der Verbindung der Formel I und deren Salze, dadurch gekennzeichnet, daß man

a) zur Herstellung von Verbindungen mit W = O

$a_1$) eine Verbindung der Formel II mit

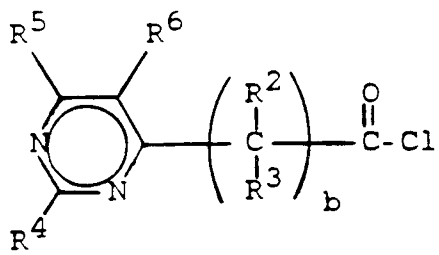

(II)

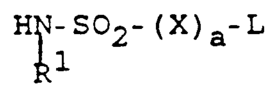

(III)

einer Verbindung der Formel III in Gegenwart einer Base umsetzt oder

$a_2$) eine Verbindung der Formel (IIa)

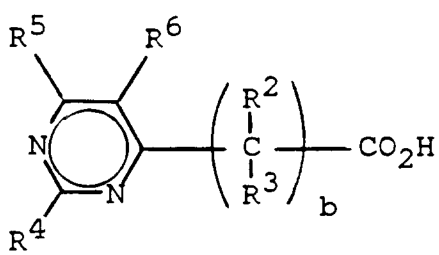

(IIa)

in Gegenwart von aktivierenden Reagentien, wie 2-Chlor-1-methylpyridinium-chlorid (IVa), Dicyclohexylcarbodiimid (IVb) oder 1,1-Carbonyldiimidazol (IVc), und gegebenenfalls in Gegenwart einer Base mit einer Verbindung der Formel III umsetzt oder

$a_3$) zur Herstellung von Verbindungen mit W = O und $R^1$ = H eine Verbindung der Formel (V),

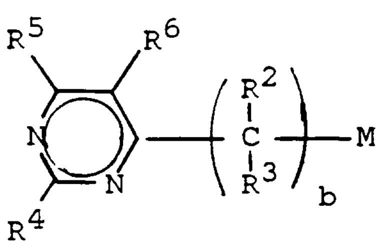

(V)

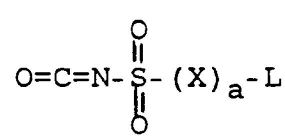

(VI)

worin M Wasserstoff (für b = 0) oder Lithium bedeutet, mit einer Verbindung der Formel (VI) umsetzt oder

b) zur Herstellung von Verbindungen mit W = S eine unter a) erhaltene Verbindung der Formel I mit $P_4S_{10}$ (VIIa) oder der Verbindung der Formel VIIb umsetzt

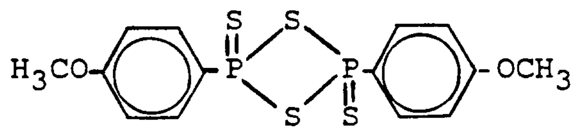

(VIIb)

c) zur Herstellung von Verbindungen mit W = $NR^7$ und $R^1$ = H eine Verbindung der Formel VIII

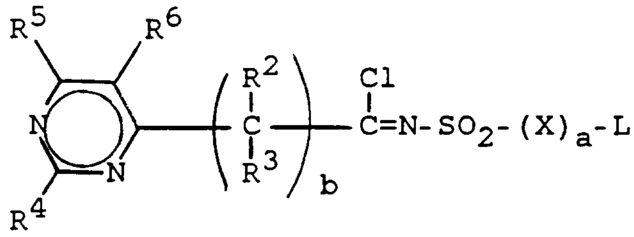

mit einem Amin der Formel $H_2N\text{-}R^7$ umsetzt,

d) zur Herstellung von Verbindungen mit W = $NOR^7$ und $R^1$ = H

$d_1$) im Falle $R^7$ = H eine Verbindung der Formel IX mit

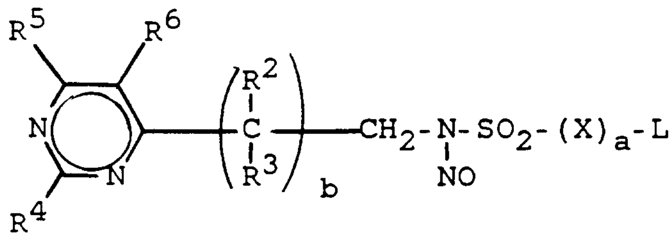

(IX)

einem Alkali- oder Erdalkalihydroxid oder Ammoniumhydroxid umsetzt oder

$d_2$) im Falle daß $R^7$ wie in Anpruch 1 definiert ist, eine Verbindung der Formel VIII in Gegenwart einer Base mit einem Hydroxylamin der Formel $H_2NOR^7$ umsetzt oder

e) zur Herstellung von Verbindungen der Formel I, worin $R^1$ ungleich Wasserstoff ist, eine Verbindung der Formel I mit $R^1$ = H in Gegenwart einer Base mit einem Alkylhalogenid der Formel $R^{1'}\text{-}X^1$, worin $R^{1'}$ die Bedeutung von $R^1$ außer Wasserstoff besitzt und $X^1$ Chlor, Brom oder Iod bedeutet, umsetzt.

Das beim Verfahren $a_1$) eingesetzte Säurechlorid der Formel II läßt sich in bekannter Weise aus der entsprechenden Carbonsäure der Formel IIa oder deren Salz mit anorganischen Säurechloriden wie Thionylchlorid oder mit Oxalylchlorid in Gegenwart geeigneter Basen, insbesondere organischer Stickstoffbasen, wie Pyridin, 2,6-Lutidin oder Triethylamin und/oder Katalysatoren wie N,N-4-Dimethylaminopyridin oder Dimethylformamid herstellen und wird zweckmäßigerweise direkt ohne Zwischenisolierung in Gegenwart der genannten Base mit der Komponente III umgesetzt. Überschüssiges Thionylchlorid oder Oxalylchlorid wird vor der Reaktion des Säurechlorids II mit dem Sulfonamid III abdestilliert.

Die Umsetzungen gemäß Verfahrensvariante $a_2$) sind an sich bekannt. Sie werden vorzugsweise in einem inerten aprotischen Solvens, wie Dichlormethan, 1,2-Dichlorethan, Acetonitril oder Glykoldimethylether bei Temperaturen zwischen -30 °C bis 83 °C durchgeführt. Als Basen werden beispielsweise organische Stickstoffbasen wie Pyridin, Triethylamin etc. verwendet. Im Falle des Carbonyldiimidazols ist kein Basenzusatz erforderlich.

Das Verfahren $a_3$) ist an sich bekannt; es wird bevorzugt in einem inerten, aprotischen Solvens wie Dichlormethan, Tetrahydrofuran, 1,2-Dichlorethan oder Glykoldimethylether im Temperaturbereich von -78 °C bis 85 °C durchgeführt.

Das Verfahren gemäß Variante b) ist ebenfalls an sich bekannt; es wird vorzugsweise in einem inerten aprotischen Solvens wie Toluol oder Xylol bei Temperaturen zwischen 0 °C und 145 °C durchgeführt. In Fällen, in denen die Produkte im Reaktionsmedium unlöslich sind, lassen sie sich durch einfache Filtration isolieren. Sind die Reaktionsprodukte löslich, lassen sie sich nach Abdampfen des Solvens durch Kristallisation oder Chromatographie des Rückstandes erhalten.

Die Verfahrensvarianten c) und d) sind im Prinzip bekannt. Verfahren c) und $d_2$) werden bevorzugt in einem inerten aprotischen Solvens wie Dichlorethan oder Toluol im Temperaturbereich zwischen 0 ° C und 110 ° C durchgeführt.

Die Imidoylchloride der Formel VIII können aus einer gemäß Variante a) hergestellten Verbindung der Formel I durch Umsetzung mit Triphenylphosphin/Tetrachlormethan oder $PCl_5/POCl_3$ erhalten werden. Das Verfahren wird vorzugsweise in $CCl_4$ oder $POCl_3$ bei Temperaturen zwischen 0 ° C und 105 ° C durchgeführt, vgl. Houben-Weyl, Bd. E5/1, S. 628-632 (1985); Bd 5/3 S. 916-922 und Bd 8 S. 346, 673-76.

Das an sich bekannte Verfahren e) wird bevorzugt in einem inerten, aprotischen Solvens wie Dichlormethan, Tetrahydrofuran, 1,2-Dichlorethan oder Glykoldimethylether im Temperaturintervall von -78 ° C bis 85 ° C durchgeführt.

Die den Säurechloriden der Formel (II) entsprechenden Carbonsäuren der Formel (IIa) können nach verschiedenen Methoden hergestellt werden. Möglich ist z.B. die alkalische Verseifung der entsprechenden Carbonsäureester (J. March "Advanced Organic Chemistry", 3rd edition, John Wiley & Sons, NY 1985, Seite 334-338), oder die Umsetzung der Esterverbindungen mit Lithiumiodid in Pyridin oder anderen Aminen bzw. mit Trimethylsilylchlorid und Natriumiodid (vgl. J. March, "Advanced Organic Chemistry" 3rd edition, John Wiley & Sons, NY 1985, Seite 386) oder durch Umsetzung mit Jodtrimethylsilan, s. Olah, Narany, Tetrahedron 38, 2225-2277 (1982) oder durch Reaktion mit Bromwasserstoffsäure.

Die Carbonsäuren (IIa) sind ferner herstellbar aus den entsprechenden Nitrilen durch Hydrolyse, s. Monatsh. Chem. 87, 526-35 (1956) oder Überführen in die Imidoether und nachfolgende Hydrolyse, s. T. Sakamoto; Chem. Pharm. Bull. 28, 3362-8 (1980).

Ferner können die Carbonsäuren (IIa) aus den entsprechenden Benzylestern durch Hydrierung in Gegenwart eines Katalysators wie Palladium hergestellt werden, s. Houben-Weyl, "Methoden der organischen Chemie", Bd. 4/1c (1980), S. 379-387.

Alternativ dazu können die Carbonsäuren der Formel IIa aus den halogenierten Heterocyclen der Formel (IIb)

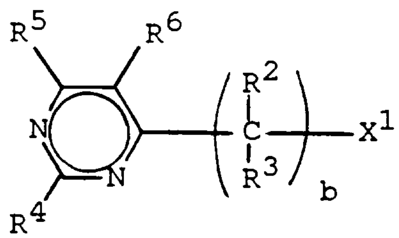

(IIb)

durch Reaktion mit Kohlendioxid direkt - oder indirekt über metallorganische Zwischenstufen erhalten werden, s. Volpin und Kolomnikov, Organomet. React. 5, 313-386 (1975); Langley, J. Am. Chem. Soc. 78, 2136 (1956); Sneeden, in Patai "The Chemistry of Carboxylic Acids and Esters", S. 137-173, Interscience, NY 1969; sowie Kharasch und Reinmuth, "Grignard Reactions of Nonmetallic Substances", S. 913-948, Prentice-Hall, Englewood Cliffs, N.Y. 1954.

Die den Carbonsäuren IIa zugrundeliegenden Nitrile sind darstellbar aus den Verbindungen der Formel (IIc)

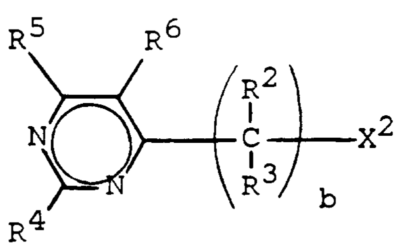

(IIc)

durch Umsetzung mit Alkali- oder Tetraalkylammoniumcyaniden. In Formel (IIc) bedeutet $X^2$ eine Abgangsgruppe wie Chlor, Brom, Jod, p-Tosyl, $OSO_2CH_3$ oder Tetraalkylammonium, s. W. Klötzer, Monatsh. Chem. 78, 526-35 (1956); Sakamoto, Chem. Pharm. Bull. 28, 3362-8 (1980); J. March, "Advanced Organic

Chemistry", 3rd edition, John Wiley & Sons, NY 1985, S. 594-595; Houben-Weyl, Bd. E5/2, S. 1447-1474 (1985); K. Hermann, Liebigs Ann. Chem. 1981, 333-41; Hurst, D.T.; Heterocycles 6, 2005-15 (1977)
Alternativ dazu sind die Nitril-Verbindungen durch Umsetzung heterocyclischer N-Oxide mit Trimethylsilylcyanid darstellbar, s. H. Yamanaka, Synthesis 1984, 681-3; und H. Vorbrüggen, Synthesis 1983, 316-9; Houben-Weyl, Bd. E5/2, S. 1444-1446 (1985).

Ferner können die Nitrile aus den entsprechenden Aminopyrimidinen gemäß Sandmeyer-Reaktion, d.h. durch Diazotierung und anschließender Umsetzung mit Kupfer(II)cyanid hergestellt werden.

Die halogenierten Heterocyclen der Formel (IIb) lassen sich durch Halogenierung der entsprechenden alkylierten heterocyclischen N-Oxide herstellen, s. Craig, J. Org. Chem. 1970 (35), 1721; Bauer, J. Org. Chem. 1963 (28), 1323; Hunt, J. Chem. Soc. 525-530 (1959) und Matsumura, Nippon Kagaku Zasshi 74, 363 (1953).

Ferner können die halogenierten Heterocyclen der Formel IIb aus den entsprechenden Hydroxyverbindungen der Formel IId durch Umsetzung mit halogenierenden Reagenzien, wie Thionylchlorid oder Phosphoroxichlorid erhalten werden, s. Angerstein, Ber.dtsch.Chem.Ges. 34, 3956 (1901); d'Atri, J. Med. Chem. 1984 (27), 1621-1629 oder Sakamoto, Chem. Pharm. Bull. 28, 3362-8 (1980),

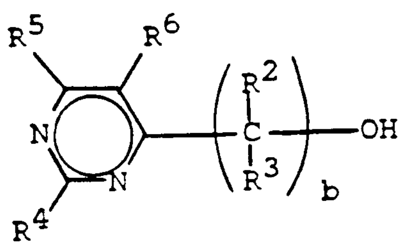

(IId)

oder im Falle b = O durch Umsetzung der entsprechenden Aminopyrimidine in einer Sandmeyer-ähnlichen Reaktion, s. Tagaki, Chem. Pharm. Bull. 11, 1382 (1963); Houben-Weyl, "Methoden der organischen Chemie", Bd. 10/3, 53 ff, Georg Thieme Verlag, Stuttgart 1965, Bd. 5/3, 846 ff und Bd. 5/4, 437 ff.

Die heterocyclischen Amine sind bekannt oder lassen sich nach im Prinzip bekannten Verfahren herstellen, s. "The Chemistry of Heterocyclic Compounds", Bd. XVI, (1962), Interscience Publ. NY & London, und Supplement I (1970) dieses Handbuchs.

Als Zwischenprodukte verwendbare Pyrimidincarbonsäurederivate der allgemeinen Formel IIe

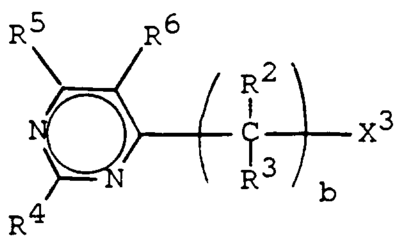

(IIe)

worin
$R^4$ und/oder $R^5$ $(C_1-C_6)$Alkoxy, $(C_1-C_6)$Alkylthio, $NR^8R^9$, Hydroxy, oder Halogen und $X^3$ -CO-Hal, CN, $CO_2H$, $CO_2R^{10}$ oder $CO_2CH_2C_6H_5$ bedeuten und $R^2$, $R^3$, $R^6$, $R^8$, $R^9$, $R^{10}$ und b die bereits genannten Bedeutungen haben, lassen sich z.B. folgendermaßen herstellen:
Alkoxy-, alkylthio- und aminosubstituierte Derivate (Formel IIe, $R^4$ und/oder $R^5$ = $(C_1-C_6)$Alkoxy, $(C_1-C_6)$-Alkylthio oder $NR^8R^9$) lassen sich aus den entsprechenden halogenierten Pyrimidincarbonsäurederivaten (Formel IIe, $R^4$ und/oder $R^5$ = Halogen) durch Umsetzung mit den entsprechenden $(C_1-C_6)$Alkoholen, $(C_1-C_6)$Alkylmercaptanen bzw. Aminen $HNR^8R^9$ in Gegenwart oder Abwesenheit äquivalenter Mengen Alkoholat, Mercaptid oder Amid oder einer anderen geeigneten Hilfsbase darstellen; s. H. Gershon, J. Org. Chem. 27, S. 3507-3510 (1962); G.D. Daves, J. Org. Chem. 26, S. 2755-2763 (1961).

Die vorstehend erwähnten halogenierten Pyrimidincarbonsäurederivate (Formel IIe, $R^4$ und/oder $R^5$ = Halogen; und $X^3$, $R^2$, $R^3$, $R^6$ und b mit den genannten Bedeutungen) lassen sich durch Umsetzung der entsprechenden Hydroxypyrimidine (Formel IIe; $R^4$ und/oder $R^5$ = Hydroxy) mit halogenierenden Reagenzien wie z.B. $POCl_3$, $PCl_5$ oder $SOCl_2$, gegebenenfalls in Gegenwart einer Hilfsbase wie z.B. Pyridin oder

einem N,N-Dialkylamin darstellen; s. H. Gershon, J. Org. Chem. 27, S. 3507-2510 (1962); G.D. Daves, J. Org. Chem. 26, S. 2755-2763 (1961); Z. Budesinsky, Coll. Czechoslov. Chem. Commun. 26, 2871-2885 (1961). Alternativ können die Verbindungen der Formel IIe mit $R^4$ und/oder $R^5$ = Halogen durch Umsetzung der entsprechenden Aminopyrimidine (Formel IIe) mit $R^4$ und/oder $R^5$ = Amino in einer Sandmeyer-ähnlichen Reaktion enthalten werden; s. Tagaki, Chem. Pharm. Bull. 11, S. 1382-8 (1963).

Alkoxysubstituierte Pyrimidincarbonsäurederivate (Formel IIe, $R^4$ und/oder $R^5$ = $C_1$-$C_6$-Alkoxy) lassen sich auch aus den o.g. entsprechenden Hydroxypyrimidinen (Formel IIe, $R^4$ und/oder $R^5$ = OH) durch Umsetzung mit alkylierenden Reagenzien wie Dialkylsulfat, Alkylhalogeniden, Alkylsulfonaten oder Trialkyloxoniumsalzen (Meerweinsalzen) darstellen.

Die genannten Hydroxypyrimidine (Formel IIe, $R^4$ und/oder $R^5$ = OH) lassen sich darstellen durch Kondensation von Verbindungen der Formel

$R^{5'}$-CO-$CHR^6$-CO-$(CR^2R^3)_b$-$X^3$

oder

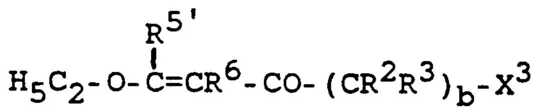

worin
$R^{5'}$ Alkoxy oder Alkyl bedeutet und b, $X^3$ und $R^6$ die genannten Bedeutungen haben, vorzugsweise b = 0 und $X^3$ = $CO_2R^{10}$ oder$CO_2CH_2C_6H_5$, mit Amidinen oder Harnstoffen der Formel

$HN = C(R^{4'})NH_2$

worin
$R^{4'}$ Alkyl, Alkoxy oder Hydroxy bedeutet, in Gegenwart katalytischer oder äquivalenter Menge einer Säure, wie z.B. Salzsäure, oder Base, wie z.B. Natronlauge in wäßrigen oder alkoholischen Medium, s. Budesinsky, Collect. Czechoslov. Chem. Commun. 26, 2871 (1961); A. Pinner, Chem. Ber. 25, 1414 (1892).

Alternativ dazu lassen sich bestimmte Hydroxypyrimidine (Formel IIe, $R^5$ = OH, $R^4$ Wasserstoff oder Alkyl, $R^6$ Wasserstoff oder Halogen, $X^3$ $CO_2R^{10}$ oder $CO_2CH_2C_6H_5$ und b = 0) durch oxidative Cyclisierung von Alkylidenasparaginen der Formel

$H_2NCOCH_2CH(COOH)N = CHR^4$

mit Hypohalogenit oder Kaliumpermanganat erhalten, s. Cherbuliez, Helv. Chim. Acta 5, 267 (1922).

Die Verbindungen der Formel (II), (IIa), (IIb), (IIc), (IId) und (IIe) und ihre in den oben beschriebenen Verfahren verwendeten Vorstufen sind zum Teil neu und entsprechen insbesondere der Formel (II')

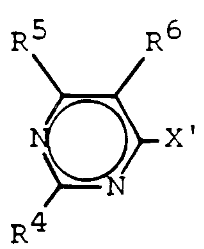

(II')

worin
X' CO-Halogen, $CO_2R^{10}$, $COO^-Me^+$, worin $Me^+$ das Äquivalent eines Alkali- oder Erdalkalimetalls ist, oder $CO_2CH_2C_6H_5$ oder CN bedeutet und $R^6$, $R^5$, $R^4$ und $R^{10}$ die bereits genannten Bedeutungen haben.

Spezielle heterocyclische Vorstufen für die Herstellung der Vorbindungen der Formel I sind in den folgenden Publikationen beschrieben:
US-A-2 876 224, EP-A-162 538, DE-A-1 445 155, DE-A-3 243 590.

Reiner, Eugster; Helv. Chem. Acta 50, 128 (1967); (2-Methylpyrimidin-4-carbonsäure).
Holy, Collect. Czech. Chem. Commun. 40, 738 (1975); (2,6-Dimethoxypyrimidin-4-carbonsäuremethylester).
Budesinsky, Collect. Czech. Chem. Commun. 37, 1721 (1972); (2-Methoxy-5-brom-4-pyrimidincarbonsäure und 2-Methoxy-5-chlor-4-pyrimidincarbonsäure).
Cherkasov, Chem. Heterocycl. Compd. (Engl. Transl.) 8, 509 (1972);
(6-Methoxy-4-pyrimidincarbonsäure, 6-Chlor-4-pyrimidincarbonsäure).
Sakasai, Heterocycles 13, 235 (1979); Brown, Aust. J. Chem. 27, 2251 (1974); (2,6-Dimethylpyrimidin-4-carbonsäuremethylester, 6-Methylpyrimidin-4-carbonsäuremethylester, 2-Methylpyrimidin-4-carbonsäuremethylester).
Budesinsky, Collect. Czech. Chem. Commun. 27,2250 (1962); (6-Chlor-5-fluor-2-methylpyrimidin-4-carbonsäureethylester).
Schwan, J. Heterocycl. Chem. 2, 202 (1965); (5-Cyan-2-methylpyrimidin-4-carbonsäuremethylester).
Budesinsky, Collect. Czech. Chem. Commun. 26, 2871 (1961); (2-Chlor-6-methylpyrimidin-4-carbonsäuremethylester, 2-Chlor-6-methylpyrimidin-4-carbonsäure, 2,6-Dimethylpyrimidin-4-carbonsäure).
Daves, J. Heterocycl. Chem. 1, 130 (1964); (6-Chlor-4-pyrimidincarbonsäuremethylester).
Klötzer, Monatsh. Chem. 87, 526 (1956); Beak, J. Am. Chem. Soc. 98, 3601 (1976);
(2,6-Dimethoxypyrimidin-4-carbonsäure, 2,6-Dimethylpyrimidin-4-carbonsäure).
Yamanaka, Chem. Pharm. Bull. 6, 638 (1958); (2,6-Dimethylpyrimidin-4-carbonsäureethylester).
Budesinsky, Collect. Czech. Chem. Commun. 14, 224 (1949); (5-Brom-2-methylpyrimidin-4-carbonsäureethylester, 5-Chlor-2-methylpyrimidin-4-carbonsäureethylester, 5-Brom-2-methylpyrimidin-4-carbonsäure, 5-Chlor-2-methylpyrimidin-4-carbonsäure).
Angerstein, Chem. Ber. 34, 3958 (1901); (6-Methylpyrimidin-4-carbonsäure).
Bhatt, J. Heterocycl. Chem. 18, 771 (1981); (5-Iod-2,6-dimethoxypyrimidin-4-carbonsäuremethylester).

Gegenstand der Erfindung sind deshalb auch als Zwischenprodukte verwendbare Carbonsäuren der Formel II' (X' = COOH), ihre Salze, ihre Ester,
Benzylester und Carbonsäurehalogenide, mit Ausnahme der bekannten Verbindungen (s. Tabelle; bekannte Verbindungen sind durch Angabe der CAS-Registry-Number, ⟨Reg.-No.⟩,gekennzeichnet):

**Tabelle 1**

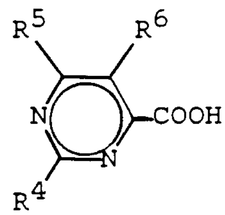

| $R^6$ | $R^5$ | $R^4$ | CAS <Reg. No> bzw. Schmp. [°C] | Ester CAS <Reg.-No> bzw. Schmp. [°C] |
|---|---|---|---|---|
| H | $OCH_3$ | $OCH_3$ | <59864-30-1> | Methylester <55878-45-0> |
| F | $OCH_3$ | $OCH_3$ | | |
| Cl | $OCH_3$ | $OCH_3$ | 177-119 | Methylester 98-100 |
| Br | $OCH_3$ | $OCH_3$ | | |
| I | $OCH_3$ | $OCH_3$ | 125-126 | Methylester <79115-66-5> |
| CN | $OCH_3$ | $OCH_3$ | | |
| $NO_2$ | $OCH_3$ | $OCH_3$ | | |
| $CO_2CH_3$ | $OCH_3$ | $OCH_3$ | | |
| $CO_2C_2H_5$ | $OCH_3$ | $OCH_3$ | | |
| H | $OCH_3$ | $CH_3$ | 184-188 | Methylester 90-93 |
| F | $OCH_3$ | $CH_3$ | | |
| Cl | $OCH_3$ | $CH_3$ | | |
| Br | $OCH_3$ | $CH_3$ | | |
| I | $OCH_3$ | $CH_3$ | | |
| CN | $OCH_3$ | $CH_3$ | | |
| $NO_2$ | $OCH_3$ | $CH_3$ | | |
| $CO_2CH_3$ | $OCH_3$ | $CH_3$ | | |
| $CO_2C_2H_5$ | $OCH_3$ | $CH_3$ | | |
| H | $CH_3$ | $OCH_3$ | 161-163 | Methylester 101-103 |
| F | $CH_3$ | $OCH_3$ | | |

Fortsetzung Tabelle 1

| $R^6$ | $R^5$ | $R^4$ | CAS <Reg. No> bzw. Schmp. [°C] | Ester CAS <Reg.-No> bzw. Schmp. [°C] |
|---|---|---|---|---|
| Cl | $CH_3$ | $OCH_3$ | | |
| Br | $CH_3$ | $OCH_3$ | | |
| I | $CH_3$ | $OCH_3$ | | |
| CN | $CH_3$ | $OCH_3$ | | |
| $NO_2$ | $CH_3$ | $OCH_3$ | | |
| $CO_2CH_3$ | $CH_3$ | $OCH_3$ | | |
| $CO_2C_2H_5$ | $CH_3$ | $OCH_3$ | | |
| H | $CH_3$ | $CH_3$ | <54198-74-2> | Ethylester <103796-11-8>, Methylester <54198-73-1> |
| F | $CH_3$ | $CH_3$ | | |
| Cl | $CH_3$ | $CH_3$ | | |
| Br | $CH_3$ | $CH_3$ | | |
| I | $CH_3$ | $CH_3$ | | |
| CN | $CH_3$ | $CH_3$ | | |
| $NO_2$ | $CH_3$ | $CH_3$ | | |
| $CO_2CH_3$ | $CH_3$ | $CH_3$ | | |
| $CO_2C_2H_5$ | $CH_3$ | $CH_3$ | | |
| H | Cl | $CH_3$ | ab 132 | |
| F | Cl | $CH_3$ | | Ethylester <712-22-1> |
| Cl | Cl | $CH_3$ | | |
| Br | Cl | $CH_3$ | | |
| I | Cl | $CH_3$ | | |
| CN | Cl | $CH_3$ | | |
| $NO_2$ | Cl | $CH_3$ | | |
| $CO_2CH_3$ | Cl | $CH_3$ | | |
| $CO_2C_2H_5$ | Cl | $CH_3$ | | |

**Fortsetzung Tabelle 1**

| $R^6$ | $R^5$ | $R^4$ | CAS <Reg. No> bzw. Schmp. [°C] | Ester CAS <Reg.-No> bzw. Schmp. [°C] |
|---|---|---|---|---|
| H | $CH_3$ | Cl | <89581-58-8> | Methylester <89793-11-3> |
| F | $CH_3$ | Cl | | |
| Cl | $CH_3$ | Cl | | |
| Br | $CH_3$ | Cl | | |
| I | $CH_3$ | Cl | | |
| CN | $CH_3$ | Cl | | |
| $NO_2$ | $CH_3$ | Cl | | |
| $CO_2CH_3$ | $CH_3$ | Cl | | |
| $CO_2C_2H_5$ | $CH_3$ | Cl | | |
| H | Cl | $OCH_3$ | | |
| F | Cl | $OCH_3$ | | |
| Cl | Cl | $OCH_3$ | | |
| Br | Cl | $OCH_3$ | | |
| I | Cl | $OCH_3$ | | |
| CN | Cl | $OCH_3$ | | |
| $NO_2$ | Cl | $OCH_3$ | | |
| $CO_2CH_3$ | Cl | $OCH_3$ | | |
| $CO_2C_2H_5$ | Cl | $OCH_3$ | | |
| H | $OCH_3$ | Cl | | |
| F | $OCH_3$ | Cl | | |
| Cl | $OCH_3$ | Cl | | |
| Br | $OCH_3$ | Cl | | |
| I | $OCH_3$ | Cl | | |
| CN | $OCH_3$ | Cl | | |
| $NO_2$ | $OCH_3$ | Cl | | |
| $CO_2CH_3$ | $OCH_3$ | Cl | | |
| $CO_2C_2H_5$ | $OCH_3$ | Cl | | |

Die als Zwischenprodukte in den Verfahren a 1) und a 2) verwendeten Sulfonamide der Formel III werden im Falle a = 0 aus den entsprechenden Anilinen durch Diazotierung und Austausch der Diazogruppe mit Schwefeldioxid in Gegenwart eines Katalysators wie Kupfer(I)chlorid in Salzsäure oder Essigsäure und Umsetzen des entstandenen Sulfonylchlorids mit Ammoniak erhalten, vgl. Meerwein, Chem. Ber. 1957, 90, 841-852.

Die Zwischenprodukte der Formel III (mit X = O und a = 1 werden aus den entsprechenden Phenolen durch Umsetzung mit Chlorsulfonylisocyanat und anschließender Hydrolyse erhalten, vgl. Lohaus, Chem. Ber. 1972, 105, 2791-2799.

Die Verbindungen der Formel III mit X = $NR^7$ und a = 1 werden aus den entsprechenden Anilinen durch Reaktion mit Amidosulfochlorid in Gegenwart einer Hilfsbase wie Triethylamin in bekannter Weise hergestellt.

Die Sulfonamide der Formel III mit X = $CHR^2$ und a = 1 werden aus den entsprechenden Benzylhalogeniden durch Reaktion mit Thioharnstoff, anschließender Oxidation mit Chlor und nachfolgender Reaktion mit Ammoniak nach üblichen Methoden erhalten. Die Sulfonamide der Formel III mit X = $CHR^2$ und a = 1 werden auch aus den entsprechenden Benzylhalogeniden nach Überführung in das Grignard-Reagenz durch Reaktion mit $SO_2$ und nachfolgender Reaktion des entstandenen Sulfinatsalzes mit Hydroxylamin-O-sulfonsäure in gepufferter wäßriger Säure erhalten, vgl. S.L. Graham, Synthesis 1986, 1031, 1032.

Alternativ können Sulfonamide der allgemeinen Formel (III), worin bevorzugt L = L5, X = $CHR^2$ und a = 1 bedeuten, für den Fall der 2-Pyridylderivate durch Metallierung der entsprechenden 2-Methylpyridinverbindungen der allgemeinen Formel (IIIa),

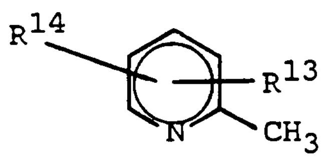

(IIIa)

worin $R^{13}$ und $R^{14}$ die oben angegebene Bedeutung haben, mit geeigneten Basen wie z.B. Butyllithium, nachfolgender Reaktion der Organometallverbindung mit $SO_2$ und Reaktion des entstandenen Sulfinatsalzes mit Hydroxylamin-O-Sulfonsäure erhalten werden. Die Metallierung und die Umsetzung mit $SO_2$ findet dabei bevorzugt in etherischen Solventien und Kohlenwasserstoffen im Temperaturbereich von -120 °C bis +10 °C statt, während die erhaltenen Sulfinatsalze bevorzugt in gepufferter wäßriger Lösung umgesetzt werden.

Ebenfalls sind Sulfonamide der allgemeinen Formel (III), worin bevorzugt L = L5, X = $CHR^2$ und a = 1 bedeuten, durch Substitutionsreaktionen von Pyridinverbindungen der allgemeinen Formel (IIIb),

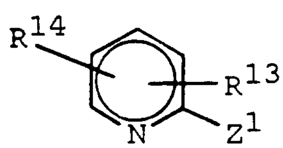

(IIIb)

worin

$Z^1$ eine Abgangsgruppe, wie z.B. Halogen, Methylsulfonyl oder Tetraalkylammonium bedeutet, mit substituierten Alkylsulfonamiden der allgemeinen Formel (X),

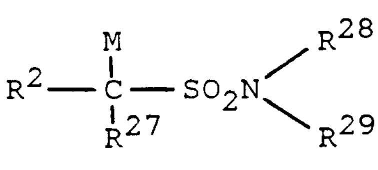

(X)

worin

M Wasserstoff oder ein Metallatom, $R^{27}$ Wasserstoff oder $CO_2(C_1\text{-}C_3)$Alkyl, $R^{28}$ tert.-Butyl oder Trialkylsilyl und $R^{29}$ Wasserstoff, ein Metallatom oder Trialkylsilyl bedeuten, und nachfolgender alkalischer oder bevorzugt saurer Hydrolyse zu erhalten.

Sulfonamide der allgemeinen Formel (III), worin bevorzugt L = L5, X = $CHR^2$ und a = 1 bedeuten, sind ebenfalls darstellbar durch Cyclisierung offenkettiger Zwischenprodukte der allgemeinen Formel (XI) und (XII),

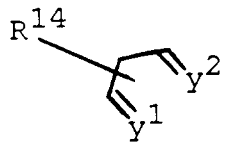

(XI)

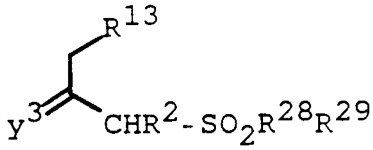

(XII)

worin

$y^1$ Sauerstoff, $(OAlkyl)_2$ oder NH, $y^2$ Sauerstoff oder $(OAlkyl)_2$, $y^3$ Sauerstoff oder NH bedeuten und $R^2$, $R^{13}$, $R^{14}$, $R^{28}$ und $R^{29}$ die oben angegebene Bedeutung haben.

Verbindungen der Formel III, worin $R^1$ die genannte Bedeutung, vorzugsweise H, und X = $CH_2$, a = 1 und L = L5 bedeuten, sind neu und ebenfalls Gegenstand der Erfindung.

Die Herstellung der Sulfonylisocyanate der Formel (VI) erfolgt nach für den Fachmann bekannten Standardverfahren, s. "Newer Methods of Preparative Organic Chemistry", Volume VI, 223-241, Academic Press, New York & London, Lohaus, Chem. Ber. 105 1972), 2791.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z. B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den Perennierenden.

Unter den spezifischen Kulturbedingungen im Reis vorkommenden Unkräuter wie z. B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrüben, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die Verbindungen der Formel (I) oder deren Kombination mit einem oder mehreren der genannten Herbizide bzw. Herbizidgruppen können auf verschiedene Art formuliert werden, je nachdem welche

biologischen und/oder chemischphysikalischen Parameter vorgegeben sind. Als Formulierungsmöglichkeiten kommen beispielsweise in Frage: Spritzpulver (WP), emulgierbare Konzentrate (EC), wäßrige Lösungen (SL), Emulsionen, versprühbare Lösungen, Dispersionen auf Öl- oder Wasserbasis, Suspoemulsionen (SC), Stäubemittel (DP), Beizmittel, Granulate zur Boden- bzw. Streuapplikation, wasserdispergierbare Granulate (WG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Intruduction to Clay Colloid Chemistry"; 2nd Ed., J. Wiley & Sons, N.Y., Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole oder Fettamine, Alkan- oder Alkylbenzolsulfonate und Dispergiermittel, z.B. ligninsulfonsaures Natrium, 2 2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoff der Formel (I), 1 bis 99,9 Gew.-%, insbesondere 5 bis 99,8 Gew.-%, eines festen oder flüssigen Zusatzstoffes und 0 bis 25 Gew.-%, eines Tensides.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 85 Gew.-%, vorzugsweise 5 bis 80 Gew.-% betragen. Staubförmige Formulierungen enthalten etwa 1 bis 25 Gew.-%, meistens 5 bis 20 Gew.-% an Wirkstoff, versprühbare Lösungen etwa 0,2 bis 25 Gew.-%, vorzugsweise 2 bis 20 Gew.-% Wirkstoff. Bei wasserdispergierbaren Granulaten hängt der Wirkstoffgehalt zum Teil davon ab, ob die wirksame Verbindung flüssig oder fest vorliegt. Im allgemeinen liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate, sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u.a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z. B. Insektiziden, Akariziden, Herbiziden, Safenern, Düngemitteln, Wachstumsregulatoren oder Fungiziden sind gegebenenfalls möglich.

Die erfindungsgemäßen Verbindungen der Formel (I) zeichnen sich außerdem durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt verschiedene wirtschaftlich bedeutende, phytopathogener Pilze, wie z. B. Piricularia oryzae, Leptospaeria nodorum, Pyrenophora teres, verschiedene Rostpilze und Botrytis cinerea; besonders hervorzuheben ist die ausgezeichnete Wirkung gegen Echte Mehltaupilze.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch fungizide Mittel, die die Verbindungen der Formel I neben geeigneten Formulierungshilfsmitteln enthalten. Die erfindungsgemäßen fungiziden Mittel enthalten die Wirkstoffe der Formel I im allgemeinen zu 1 bis 95 Gew.-%.

Sie können auf verschiedene Art formuliert werden, je nachdem wie es durch die biologischen und/oder chemischphysikalischen Parameter vorgegeben ist. Als Formulierungsmöglichkeiten kommen im Prinzip alle in Frage, die oben bereits für herbizide Mittel genannt worden sind.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z. B. folgende Produkte zu nennen:
Imazalil, Prochloraz, Fenapanil, SSF 105, Triflumizol, PP 969, Flutriafol, BAY-MEB 6401, Propiconazol, Etaconazol, Diclobutrazol, Bitertanol, Triadimefon, Triadimenol, Fluotrimazol, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol, Bupirimate, Rabenzazole, Tricyclazole, Fluobenzimine, Pyroxyfur, NK-483, PP-389, Pyroquilon, Hymexazole, Fenitropan, UHF-8227, Cymoxanil, Dichlofluanid, Captafol, Captan, Folpet, Tolyfluanid, Chlorothalonil, Etridiazol, Iprodione, Procymidon, Vinclozolin, Metomeclan, Myclozolin, Dichlozolinate, Fluorimide, Drazaxolan, Chinomethionate, Nitrothalisopropyl, Dithianon, Dinocap, Binapacryl, Fentinacetate, Fentinhydroxide, Carboxin, Oxycarboxin, Pyracarbolid, Methfuroxam, Fenfuram, Furmecyclos, Benodanil, Mebenil, Mepronil, Flutalanil, Fuberidazole, Thiabendazole, Carbendazim, Benomyl, Thiophanate, Thiophanate-methyl, CGD-95340 F, IKF-1216, Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cu-oxychlorid, Oxine-Cu, Cu-oxide, Schwefel, Fosethylaluminium, Natrium-dodecylbenzolsulfonat, Natrium-dodecylsulfat, Natrium-C13/C15-alkoholethersulfonat, Natrium-cetostearylphosphatester, Dioctyl-natriumsulfosuccinat, Natrium-isopropylnaphthalinsulfonat, Natrium-methylenbisnaphthalinsulfonat, Cetyl-trimethyl-ammoniumchlorid,
Salze von langkettigen primären, sekundären oder tertiären Aminen, Alkyl-propylenamine, Lauryl-pyridinium-bromid, ethoxilierte quaternierte Fettamine, Alkyl-dimethylbenzylammoniumchlorid und 1-Hydroxyethyl-2-alkyl-imidazolin.

Die obengenannten Kombinationspartner stellen bekannte Wirkstoffe dar, die zum großen Teil in C.R. Worthing, S.B. Walker, "The Pesticide Manual", 7. Auflage (1983) und 8. Auflage (1987), British Crop Protection Council beschrieben sind.

Darüber hinaus können die erfindungsgemäßen Wirkstoffe, insbesondere die der aufgeführten Beispiele, in ihren handelsüblichen Formulierungen sowie in den aus diesen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u. a. Bevorzugte Mischungspartner sind:

1. aus der Gruppe der Phosphorsäureester Azinphos-ethyl, Azinphos-methyl, 1-(p-Chlorphenyl)-4-(O-ethyl, S-propyl)phosphoryl-oxypyrazol (TIA-230), Chlorpyrifos, Coumaphos, Demeton, Demeton-S-methyl, Diazinon, Dichlorvos, Dimethoat, Ethoprophos, Etrimfos, Fenitrothion, Fenthion, Heptenophos, Parathion, Parathion-methyl, Phosalon, Pirimiphos-ethyl, Pirimiphos-methyl, Profenofos, Prothiofos, Sulprofos, Triazophos, Trichlorfon.

2. aus der Gruppe der Carbamate
Aldicarb, Bendiocarb, BPMC (2-(1-Methylpropyl)-phenylmethylcarbamat), Butocarboxim, Butoxicarboxim, Carbaryl, Carbofuran, Carbosulfan, Cloethocarb, Isoprocarb, Methomyl, Oxamyl, Pirimicarb, Promecarb, Propoxur, Thiodicarb.

3. aus der Gruppe der Carbonsäureester
Allethrin, Alphamethrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)-cyclopropancarbonsäure-(alpha-cyano-3-phenyl-2-methyl-benzyl)-ester (FMC 54800), Fenpropathrin, Fenfluthrin, Fenvalerat, Flucythrinate, Flumethrin, Fluvalinate, Permethrin, Resmethrin, Tralomethrin.

4. aus der Gruppe der Formamidine
Amitraz, Chlordimeform

5. aus der Gruppe der Zinnverbindungen
Azocyclotin, Cyhexatin, Fenbutatinoxid

6. Sonstige
Abamektin, Bacillus thuringiensis, Bensultap, Binapacyl, Bromopropylate, Buprofecin, Camphechlor, Cartap, Chlorbenzilate, Chlorfluazuron, 2-(4-Chlorphenyl)-4,5-diphenylthiophen (UBI-T 930), Chlofentezine, Cyclopropancarbonsäure-(2-naphthylmethyl)-ester (Ro 12-0470), Cyromacin, DDT, Dicofol, N-(3,5-Dichlor-4-(1,1,2,2-tetrafluoroethoxy)phenylamino)carbonyl)-2,6-difluorbenzamide (XRD 473), Diflubenzuron, N-(2,3-Dihydro-3-methyl-1,2-thioazol-2-ylidene)-2,4-xylidine, Dinobuton, Dinocap, Endosulfan, Fenoxycarb, Fentiocarb, Flubenzimine, Flufenoxuron, Gamma-HCH, Hexythiazox, Hydramethylnon (AC 217 300), Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinan-3-yl-carbamaldehyde (WL 108 477), Propargite, Teflubenzuron, Tetradifon, Tetrasul, Thicyclam, Triflumaron, Kernpolyeder- und Granuloseviren.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 100 Gew.-% Wirkstoff, vorzugsweise zwischen 0,001 und 1 Gew.-% liegen. Die Anwendung geschieht in einer den Anwendungsformen angepaßten üblichen Weisen.

A. **Formulierungsbeispiele**

a) Ein Stäubmittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösemittel und 10 Gew.-Teilen oxethyliertes Nonylphenol als Emulgator.

e) Ein in Wasser dispergierbares Granulat wird erhalten indem man
75 Gewichtsteile einer Verbindung der Formel I,
10 Gewichtsteile ligninsulfonsaures Calcium,
5 Gewichtsteile Natriumlaurylsulfat,
3 Gewichtsteile Polyvinylalkohol und
7 Gewichtsteile Kaolin
mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man
25 Gewichtsteile einer Verbindung der Formel (I),

5 Gewichtsteile 2,2'-Dinaphthylmethan-6,6'-disulfonsaures Natrium,
2 Gewichtsteile oleoylmethyltaurinsaures Natrium,
1 Gewichtsteile Polyvinylalkohol,
17 Gewichtsteile Calciumcarbonat und
50 Gewichtsteile Wasser
auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

g) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel Ab) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

B. Chemische Beispiele

Beispiel 1: 5-Chloro-2,6-dimethoxypyrimidin-4-carbonsäuremethylester

Zu einer Lösung aus 7,4 g Natrium in 100 ml abs. Methanol gibt man tropfenweise 44 g 2,5,6-Trichlorpyrimidin-4-carbonsäuremethylester in 100 ml abs. Methanol gelöst zu, wobei die Reaktionstemperatur ansteigt. Man rührt noch 1 h bei Raumtemperatur nach, destilliert das Solvens i. Vak. ab und nimmt den Rückstand in Wasser/Dichlormethan auf. Die wäßrige Phase wird mit 2N Salzsäure neutralisiert und zweimal mit Dichlormethan extrahiert. Die vereinigten organischen Phasen werden über Natriumsulfat getrocknet und über ein Kieselgelbett filtriert. Nach Abdestillieren des Lösungsmittels erhält man 32,0 g 5-Chloro-2,6-dimethoxypyrimidin-4-carbonsäuremethylester vom Schmp. 98-100 °C.

Beispiel 2: 5-Chloro-2,6-dimethoxypyrimidin-4-carbonsäure

Zur gerührten Lösung von 5,4 g Natriumhydroxid in 100 ml Methanol und 5 ml Wasser gibt man 29,5 g 5-Chloro-2,6-dimethoxypyrimidin-4-carbonsäuremethylester fest zu. Man rührt bis zum Ende der Verseifung (3 h) bei Raumtemperatur nach, destilliert Methanol ab und nimmt den Rückstand in Wasser auf. Die wäßrige Phase wird zunächst einmal mit Essigsäureethylester extrahiert, dann mit konz. Salzsäure auf pH 2-3 angesäuert und fünfmal mit Dichlormethan extrahiert. Die vereinigten Dichlormethanextrakte werden über Natriumsulfat getrocknet, das Solvens abdestilliert und der Rückstand bei 50 °C im Vakuum getrocknet. Man erhält so 23,0 g 5-Chloro-2,5-dimethoxypyrimidin-4-carbonsäure vom Schmp. 117-119 °C (Zers.).

Beispiel 3: 2,6-Dimethoxy-5-iodo-pyrimidin-4-carbonsäure

Zur gerührten Lösung von 2,4 g Natriumhydroxid in 70 ml Methanol und 4 ml Wasser werden portionsweise 18,0 g 2,6-Dimethoxy-5-iodo-pyrimidin-4-carbonsäuremethylester eingetrag . Man läßt über Nacht bei Raumtemperatur nachrühren, destilliert das Methanol ab und nimmt den Rückstand in Wasser auf. Die wäßrige Phase wird mit Essigester extrahiert, mit konz. Salzsäure auf pH 2-3 angesäuert und mit Dichlormethan extrahiert. Die vereinigten Dichlormethanextrakte werden über Natriumsulfat getrocknet und das Solvens i. Vak. abdestilliert. Man erhält so 10,1 g 2,6-Dimethoxy-5-iodopyrimidin-4-carbonsäure vom Schmp. 125-126 °C.

Beispiel 4: 2-Methoxy-6-methylpyrimidin-4-carbonsäuremethylester

Zu einer Lösung von 4,2 g Natrium in 100 ml Methanol gibt man 30,0 g 2-Chlor-6-methylpyrimidin-4-carbonsäuremethylester zu. Man erhitzt 2 h unter Rückfluß, engt unter vermindertem Druck ein und nimmt den Rückstand in Dichlormethan/Wasser auf. Die organische Phase wird abgetrennt und die wäßrige Lösung noch fünfmal mit Dichlormethan extrahiert. Die vereinigten organischen Extrakte werden über Natriumsulfat getrocknet und das Lösungsmittel unter reduziertem Druck abdestilliert. Man erhält so 25,5 g 2-Methoxy-6-methylpyrimidin-4-carbonsäuremethylester vom Schmp. 101-103 °C.

Beispiel 5: 2-Methoxy-6-methylpyrimidin-4-carbonsäure

Zur gerührten Lösung von 3,6 g Natriumhydroxid in 100 ml Methanol und 10 ml Wasser gibt man 15,5 g 2-Methoxy-6-methylpyrimidin-4-carbonsäuremethylester. Man rührt bis zum Verschwinden des Ausgangsmaterials bei Raumtemperatur nach (DC-Kontrolle; 3 h). Das Methanol wird unter vermindertem Druck abdestilliert, der Rückstand in Wasser aufgenommen und einmal mit Essigsäureethylester extrahiert. Die Wasserphase wird mit konz. Salzsäure auf pH 2 angesäuert, 1 h nachgerührt und das Produkt abgesaugt. Man erhält so 6,6 g 2-Methoxy-6-methylpyrimidin-4-carbonsäure vom Schmp. 161-163 °C. Einengen und Extraktion der Mutterlauge mit Dichlormethan ergibt zusätzliche 3,1 g Produkt vom Schmp. 159-161 °C.

Beispiel 6: 6-Chlor-2-methylpyrimidin-4-carbonsäurechlorid

126,1 g 6-Hydroxy-2-methylpyrimidin-4-carbonsäure-Hydrat werden 4 h bis zum Ende der Gasentwicklung in 500 ml Phosphoroxychlorid zum Sieden erhitzt. Man läßt abkühlen, versetzt mit 145 g Phosphor(V)-chlorid und erhitzt erneut bis zum Ende der Gasentwicklung (5 h) unter Rückfluß. Man kühlt auf Raumtemperatur ab, saugt die ausgeschiedenen anorganischen Salze ab und destilliert überschüssiges Phosphoroxychlorid im Wasserstrahlvakuum ab. Der Rückstand wird fraktioniert. Man erhält so 82,9 g 6-Chlor-2-methylpyrimidin-4-carbonsäurechlorid vom Sdp. 116-119 °C/38 mbar.

Beispiel 7: 6-Methoxy-2-methylpyrimidin-4-carbonsäuremethylester

78,9 g 6-Chlor-2-methylpyrimidin-4-carbonsäurechlorid werden unter Rühren zu 750 ml abs. Methanol getropft. Man rührt 14 h bei Raumtemperatur nach, destilliert das Solvens ab, nimmt den Rückstand in Dichlormethan auf, wäscht einmal mit gesättigter wäßriger Natriumhydrogencarbonatlösung und trocknet die organische Phase über Natriumsulfat. Nach Abdestillieren des Dichlormethan erhält man 65,5 g 6-Methoxy-2-methylpyrimidin-4-carbonsäuremethylester vom Schmp. 90-93 °C (umkristallisiert aus Diisopropylether).

Beispiel 8: 6-Methoxy-2-methylpyrimidin-4-carbonsäure

Zur gerührten Lösung aus 14,4 g Natriumhydroxid in 100 ml Wasser und 350 ml Methanol gibt man 62,6 g 6-Methoxy-2-methylpyrimidin-4-carbonsäuremethylester zu und rührt bis zum Ende des Verseifens nach (DC-Kontrolle). Man versetzt den Reaktionsansatz mit 200 ml Methanol, saugt das Natriumsalz ab und wäscht auf der Nutsche mit Methanol/Diethylether (1:1) nach. Der im Vakuum bei 50 °C getrocknete Filterrückstand (59,3 g; Schmp. über 265 °C) wird portionsweise in eine Lösung aus 24,5 g Acetylchlorid in 300 ml Methanol eingetragen. Man rührt 1/2 h bei Raumtemperatur nach, saugt ab und wäscht den Filterrückstand mit abs. Methanol nach. Das Filtrat wird im Vakuum einrotiert und der Rückstand am Hochvakuum getrocknet. Man erhält so 25,1 g 6-Methoxy-2-methylpyrimidin-4-carbonsäure vom Schmp. 184-188 °C (Zers.).

Beispiel 9: 6-Chlor-2-methylpyrimidin-4-carbonsäure

Zu 150 ml Wasser werden bei 10-15 °C 16 g 6-Chlor-2-methylpyrimidin-4-carbonsäurechlorid zugetropft. Man rührt 1/2 h nach, saugt ab, wäscht den Filterrückstand gut mit Wasser nach und trocknet bei 50 °C im Vakuum. Man erhält so 11,5 g 6-Chlor-2-methylpyrimidin-4-carbonsäure vom Schmp. 132 °C (Zers.).

Beispiel 10: 1-(2-Trifluormethylphenyl)sulfuryldiamid

5,0 g 2-Trifluormethylanilin werden in 50 ml Dichlormethan gelöst und mit 3,5 g Triethylamin versetzt. Dazu tropft man eine Lösung von 4,0 g Amidosulfochlorid in 40 ml Dichlormethan zu, wobei die Reaktionstemperatur leicht ansteigt. Man rührt 2 h bei Raumtemperatur nach, destilliert das Solvens unter reduziertem Druck ab und verrührt den Rückstand mit 50 ml 2N Salzsäure. Das Produkt wird abgesaugt, mit Wasser gewaschen und am Hochvakuum getrocknet. Man erhält so 4,5 g 1-(2-Trifluormethylphenyl)-sulfuryldiamid vom Schmp. 111-113 °C.

Beispiel 11: 2,6-Dichlorphenylsulfamat

Eine Lösung von 24,4 g 2,6-Dichlorphenoxysulfonylisocyanat in 200 ml Tetrachlormethan wird bei 30 °C bis 40 °C tropfenweise mit 2,6 ml Wasser versetzt. Man rührt bis zum Ende der Kohlendioxidentwicklung bei Raumtemperatur nach, kühlt auf 0 °C ab, saugt das Produkt ab und wäscht den Rückstand mit eiskaltem Tetrachlormethan nach. Nach Trocknen im Vakuum erhält man 21,4 g 2,6-Dichlorphenylsulfamat

vom Schmp. 108-110 °C.

Beispiel 12: 3-Methyl-2-picolylsulfonamid

Zu einer auf 0 °C gekühlten Lösung aus 0,51 mol n-Butyllithium als 1,6N Lösung in Hexan und 500 ml abs. Diethylether tropft man bei dieser Temperatur 36,5 g 2,3-Lutidin langsam zu. Man läßt auf 10 °C aufwärmen, rührt 3 h bei dieser Temperatur nach und gibt die Lithiumsalzlösung bei -60 °C portionsweise zu einer Lösung aus 150 ml $SO_2$ und 200 ml Diethylether. Man rührt 1/2 h bei dieser Temperatur nach, läßt über Nacht auf Raumtemperatur erwärmen und destilliert das Solvens unter reduziertem Druck ab. Das rohe Lithiumsulfinat wird in 1,6 l Wasser aufgenommen, mit 66 g Natriumacetat und 50 g Hydroxylamin-O-sulfonsäure versetzt und 5 h bei Raumtemperatur gerührt. Bei pH 7 wird die wäßrige Lösung wiederholt mit Dichlormethan extrahiert, die vereinigten organischen Phasen über $Na_2SO_4$ getrocknet und das Solvens i. Vak. abdestilliert. Der Rückstand wird aus Diisopropylether/Dichlormethan rekristallisiert. Man erhält so 22,3 g 3-Methyl-2-picolylsulfonamid vom Schmp. 155-158 °C.

Beispiel 13: 2-Nitro-N-[(6-Chlor-2-methylpyrimidin-4-yl)carbonyl]benzylsulfonamid

Zur gerührten Lösung aus 2,3 g Dicyclohexylcarbodiimid, 120 mg 4-Dimethylaminopyridin und 1,9 g 6-Chlor-2-methylpyrimidin-4-carbonsäure in 80 ml abs. Dichlormethan gibt man bei 0-2 °C portionsweise 2,2 g 2-Nitrobenzylsulfonamid zu. Es wird 1/2 h bei 0 °C nachgerührt und 2 Tage bei Raumtemperatur stehen gelassen. Man saugt ab, destilliert das Solvens unter reduziertem Druck ab, löst den Rückstand mit Aceton an und verrührt 2 h mit 100 ml 10%iger wäßriger Natriumacetatlösung. Es wird abfiltriert, das Filtrat mit Diethylether extrahiert und danach mit konz. Salzsäure angesäuert. Das Produkt wird abgesaugt und bei 50 °C/20 mbar getrocknet. Man erhält so 1,55 g 2-Nitro-N-[(6-Chlor-2-methylpyrimidin-4-yl)-carbonyl]-benzylsulfonamid vom Schmp. 190-192 °C.

Beispiel 14: 2-Chlor-N[(2-Chlor-6-methylpyrimidin-4-yl)carbonyl]benzolsulfonamid

2,1 g 2-Chlor-6-methylpyrimidin-4-carbonsäure, 2,3 g Dicyclohexylcarbodiimid und 120 mg 4-Dimethylaminopyridin in 80 ml abs. Dichlormethan werden bei 0 °C portionsweise mit 1,9 g 2-Chlorbenzolsulfonamid versetzt. Man läßt während 1/2 h auf Raumtemperatur aufwärmen und rührt 4 Tage nach. Nach Aufarbeitung wie in Beispiel 13 beschrieben erhält man 1,2 g 2-Chlor-N-[(2-Chlor-6-methylpyrimidin-4-yl)-carbonyl]benzolsulfonamid vom Schmp. 170-171 °C.

Beispiel 15: 2-Trifluormethyl-N-[(6-methoxy-2-methylpyrimidin-4-yl)carbonyl]benzylsulfonamid

Zu einer Mischung aus 2,3 g Dicyclohexylcarbodiimid, 120 mg 4-Dimethylaminopyridin und 2,0 g 6-Methoxy-2-methylpyrimidin-4-carbonsäure in 80 ml abs. Dichlormethan gibt man bei 0-2 °C portionsweise 2,4 g 2-Trifluormethylbenzylsulfonamid zu. Man rührt 1/2 h bei 0 °C nach, läßt auf Raumtemperatur aufwärmen und rührt 14 h nach. Der ausgefallene Harnstoff wird abgesaugt, das Solvens unter reduziertem Druck abdestilliert, der Rückstand in Aceton angelöst und mit 100 ml 1 N wäßriger Natriumcarbonatlösung 1/2 h gerührt. Man filtriert ab, extrahiert das Filtrat mit Diethylether, säuert mit konz. Salzsäure an, saugt das Produkt ab und trocknet bei 50 °C/20 mbar. Man erhält so 2-Trifluormethyl-N-[(6-methoxy-2-methylpyrimidin-4-yl)carbonyl]benzylsulfonamid vom Schmp. 120-122 °C.

Beispiel 16: 1-(2-Trifluormethylphenyl)-3-[(2,6-dimethoxypyrimidin-4-yl)-carbonyl]sulfuryldiamid

Zur gerührten Mischung aus 2,3 g Dicyclohexylcarbodiimid, 120 mg 4-N,N-Dimethylaminopyridin und 2,2 g 2,6-Dimethoxypyrimidin-4-carbonsäure in 80 ml Dichlormethan tropft man bei 0 °C bis 2 °C eine Lösung aus 2,4 g 1-(2-Trifluormethylphenyl)sulfuryldiamid und 1,0 ml Triethylamin in 30 ml Aceton zu. Es wird noch 1/2 h bei 0 °C, dann über Nacht bei Raumtemperatur nachgerührt. Man saugt ab, destilliert das Solvens unter reduziertem Druck ab und verrührt den Rückstand mit einer Lösung aus 50 ml Aceton und 100 ml 1 M Natriumcarbonatlösung. Man filtriert vom Ungelösten ab, extrahiert die wäßrige Lösung einmal mit Diethylether und säuert sie dann mit konz. Salzsäure auf pH 2-3 an. Man läßt 1/4 h nachrühren, saugt das Produkt ab und trocknet bei 50 °C i. Vak. Man erhält so 1,9 g 1-(2-Trifluormethylphenyl)-3-[(2,6-dimethoxypyrimidin-4-yl)carbonyl]sulfuryldiamid vom Schmp. 133-135 °C.

Beispiel 17: 2-Nitro-N-[(2-methoxy-6-methylpyrimidin-4-yl)carbonyl]benzolsulfonamid

Zur gerührten Mischung aus 2,3 g Dicyclohexylcarbodiimid, 120 mg 4-N,N-Dimethylaminopyridin und 2,0 g 2-Methoxy-6-methylpyrimidin-4-carbonsäure in 80 ml abs. Dichlormethan gibt man bei 0 °C bis 2 °C portionsweise 2,0 g festes 2-Nitrobenzolsulfonamid zu. Man rührt 1/2 h bei Raumtemperatur nach und läßt dann über Nacht stehen. Man arbeitet wie in Beispiel 16 beschrieben auf und erhält 1,55 g 2-Nitro-N-[(2-methoxy-6-methyl-pyrimidin-4-yl)-carbonyl]benzolsulfonamid vom Schmp. 212-216 °C.

Beispiel 18: 2-Trifluormethyl-N-[(2-chloro-6-methylpyrimidin-4-yl)carbonyl]benzylsulfonamid

Zur gerührten Lösung von 2,4 g 2-Trifluormethylbenzylsulfonamid in 40 ml Dimethoxyethan gibt man 0,44 g Natriumhydrid-Suspension (60 %ig in Mineralöl) zu und rührt bis zum Ende der Wasserstoffentwicklung nach. Dazu tropft man eine Lösung von 2-Chlor-6-methylpyrimidin-4-carbonsäurechlorid (aus 2,1 g Carbonsäure, 20 ml Dioxan, einige Tropfen Dimethylformamid und 2,1 g Thionylchlorid) in 20 ml Dimethoxyethan schnell zu. Man rührt bei Raumtemperatur 3 h nach und arbeitet wie in Beispiel 13 beschrieben auf. Man erhält so 1,4 g 2-Trifluormethyl-N-[(2-chloro-6-methylpyrimidin-4-yl)carbonyl]benzylsulfonamid vom Schmp. 198 - 199 °C.

Die Verbindungen der nachfolgenden Tabellen 2 bis 12 lassen sich analog zu den in Beispielen 1 bis 18 beschriebenen Verfahrensweisen herstellen. Die Definitionen der Reste der Formeln L 1-1 bis L 5-29 sind der Tabelle 2 vorangestellt. Die Bedeutungen der Symbole in den Tabellen 2 bis 12 beziehen sich auf die der jeweiligen Tabelle vorangestellte allgemeine Formel.

L1 = 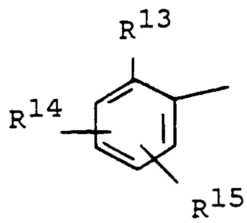

L 1-1 : $R^{13}$= $-CO_2CH_3$ ; $R^{14}, R^{15}$ = H

L 1-2 : $R^{13}$= $-CO_2CH_2CH_3$ ; "

L 1-3 : $R^{13}$= $-CO_2CH(CH_3)_2$ ; "

L 1-4 : $R^{13}$= $-CO_2CH_3$ ; $R^{14}$ = 5-$OCF_2H$ ; $R^{15}$ = H

L 1-5 : $R^{13}$= $-CO_2CH_3$ ; $R^{14}$ = 5-Cl, ; $R^{15}$ = H

L 1-6 : $R^{13}$= $-CO_2CH_3$ ; $R^{15}$ = 5-$OCH_3$ ; $R^{15}$ = H

L 1-7 : $R^{13}$= $-CON(CH_3)_2$ ; $R^{14}, R^{15}$ = H

L 1-8 : $R^{13}$= $-CF_3$ ; "

L 1-9 : $R^{13}$= 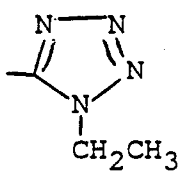 ; "

L 1-10: $R^{13}$= 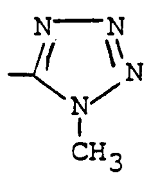 ; "

L 1-11: $R^{13}$= 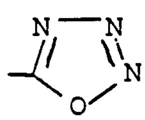 ; "

L 1-12: $R^{13}$= 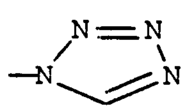 ; "

L 1-13: $R^{13}$= 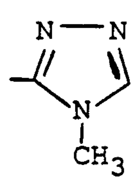 ; $R^{14}$= 6-Cl; $R^{15}$= H

| | | |
|---|---|---|
| L 1-14: $R^{13}$= | 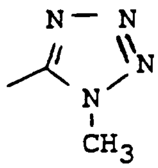 ; | $R^{14}$= 5-$OCH_3$; $R^{15}$= H |
| L 1-15: $R^{13}$= | $-OCH_3$ ; | $R^{14}$,$R^{15}$= H |
| L 1-16: $R^{13}$= | $-OC_2H_5$ ; | " |
| L 1-17: $R^{13}$ | $-OCH(CH_3)_2$ ; | " |
| L 1-18: $R^{13}$= | $-OCH_2CH_2Cl$ ; | " |
| L 1-19: $R^{13}$= | $-OCH_2CH_2-OCH_3$ ; | " |
| L 1-20: $R^{13}$= | $-OCH_2CF_3$ ; | " |
| L 1-21: $R^{13}$= | $-OCF_2CF_3$ ; | " |
| L 1-22: $R^{13}$= | $-OSO_2CH_3$ ; | " |
| L 1-23: $R^{13}$= | $-OSO_2CH_2CH_3$ ; | " |
| L 1-24: $R^{13}$= | -Br ; | " |
| L 1-25: $R^{13}$= | -F ; | " |
| L 1-26: $R^{13}$= | -Cl ; | " |
| L 1-27: $R^{13}$= | $-OCF_2CHF_2$ ; | " |
| L 1-28: $R^{13}$= | $-NO_2$ ; | " |
| L 1-29: $R^{13}$= | $-N(CH_3)_2$ ; | " |
| L 1-30: $R^{13}$= | $-N(CH_3)CH_2CH_3$ ; | " |
| L 1-31: $R^{13}$= | $-N(CH_2CH_3)_2$ | " |
| L 1-32: $R^{13}$= | -CN ; | " |
| L 1-33: $R^{13}$= | $-CO-CH_3$ ; | " |
| L 1-34: $R^{13}$= | -F | $R^{14}$= 6-F; $R^{15}$= H |
| L 1-35: $R^{13}$= | -Cl ; | $R^{14}$= 6-Cl; $R^{15}$= H |
| L 1-36: $R^{13}$= | -Br ; | $R^{14}$= 6-Br; $R^{15}$= H |
| L 1-37: $R^{13}$= | $-NO_2$ ; | $R^{14}$= 6-$CH_3$; $R^{15}$= H |
| L 1-38: $R^{13}$= | $-CH_3$ ; | $R^{14}$,$R^{15}$= H |
| L 1-39: $R^{13}$= | $-C_6H_5$ ; | " |
| L 1-40: $R^{13}$= | $-SO_2CH_3$ ; | " |
| L 1-41: $R^{13}$= | $-SO_2CH_2CH_3$ ; | " |
| L 1-42: $R^{13}$= | $-SO_2CH(CH_3)_2$ ; | " |
| L 1-43: $R^{13}$= | $-SO_2CH_2CH_2CH_3$ ; | " |
| L 1-44: $R^{13}$= | $-SO_2N(CH_3)_2$ ; | " |
| L 1-45: $R^{13}$= | $-CH_2Cl$ ; | " |

L 1-46: $R^{13}$= $-CH_2OCH_3$ ; $R^{14}$,$R^{15}$= H
L 1-47: $R^{13}$= $-CH_2N(CH_3)_2$ ; "
L 1-48: $R^{13}$= $-CH_2SCH_3$ ; "
L 1-49: $R^{13}$= $-N_3$ ; "
L 1-50: $R^{13}$= $-SCH_3$ ; "
L 1-51: $R^{13}$= $-SCH_2CH_3$ ; "
L 1-52: $R^{13}$= $-OCF_2H$ ; "
L 1-53: $R^{13}$= $-OCF_3$ ; "
L 1-54: $R^{13}$= -I ; "
L 1-55: $R^{13}$= $-NO_2$ ; $R^{14}$= 6-F ; $R^{15}$= H
L 1-56: $R^{13}$= $-NO_2$ ; $R^{14}$= 6-Cl; $R^{15}$= H
L 1-57: $R^{13}$= $-NO_2$ ; $R^{14}$= 6-I ; $R^{15}$= H
L 1-58: $R^{13}$= -Cl ; $R^{14}$= $6-CH_3$; $R^{15}$= H
L 1-59: $R^{13}$= $-CO_2CH_3$ ; $R^{14}$= $6-NO_2$; $R^{15}$= H
L 1-60: $R^{13}$= $-CO_2CH_3$ ; $R^{14}$= 6-Cl ; $R^{15}$= H
L 1-61: $R^{13}$= $-CO_2CH_3$ ; $R^{14}$= 6-I ; $R^{15}$= H
L 1-62: $R^{13}$= $-CO_2CH_3$ ; $R^{14}$= $6-SO_2N(CH_3)_2$; $R^{15}$= H
L 1-63: $R^{13}$= -F ; $R^{14}$= 6-I ; $R^{15}$= H
L 1-64: $R^{13}$= -F ; $R^{14}$= 6-Cl ; $R^{15}$= H
L 1-65: $R^{13}$= -Cl ; $R^{14}$= 6-I ; $R^{15}$= H
L 1-66: $R^{13}$= $-SO_2N(CH_3)_2$ ; $R^{14}$= $6-SO_2N(CH_3)_2$; $R^{15}$= H
L 1-67: $R^{13}$= $-SO_2N(CH_3)_2$ ; $R^{14}$= 6-Cl ; $R^{15}$= H
L 1-68: $R^{13}$= $-SO_2N(CH_3)_2$ ; $R^{14}$= $6-NO_2$; $R^{15}$= H

L 3 = 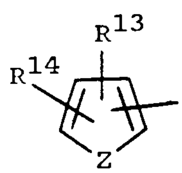

L 3-1: 3-Methoxycarbonyl-2-thienyl
L 3-2: 2-Methoxycarbonyl-3-thienyl
L 3-3: 2-Ethoxycarbonyl-3-thienyl
L 3-4: 4-Methoxycarbonyl-3-thienyl
L 3-5: 3-Chloro-2-thienyl

L 4 = 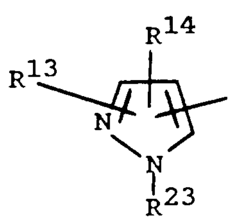

L 4-1: $R^{14}$= H; $R^{13}$= 4-$CO_2CH_3$; $R^{23}$= $CH_3$; freie Valenz an C-5
L 4-2: $R^{14}$= H; $R^{13}$= 4-$CO_2CH_2CH_3$; $R^{23}$= -$CH_3$ " C-5
L 4-3: $R^{14}$= 3-Cl; $R^{13}$= 4-$CO_2CH_3$; $R^{23}$= -$CH_3$; " C-5
L 4-4: $R^{14}$= 3-$SO_2N(CH_3)_2$; $R^{13}$= H; $R^{23}$= -$CH_3$; " C-4
L 4-5: $R^{14}$= 3-$CH_3$; $R^{13}$= 5-$CO_2CH_3$; $R^{23}$= -$CH_3$; " C-4

L 5 = 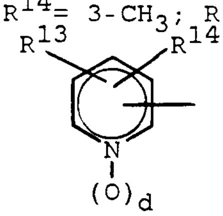

L 5-1 : 3-Methoxycarbonyl-2-pyridyl
L 5-2 : 3-Methylsulfonyl-2-pyridyl
L 5-3 : 3-Ethoxycarbonyl-2-pyridyl
L 5-4 : 3-Dimethylaminosulfonyl-2-pyridyl
L 5-5 : 3-Trifluormethyl-2-pyridyl
L 5-6 : 3-Methoxy-2-pyridyl
L 5-7 : 3-Ethoxy-2-pyridyl
L 5-8 : 3-Methyl-2-pyridyl
L 5-9 : 3-Ethyl-2-pyridyl
L 5-10 : 2-Dimethylaminosulfonyl-3-pyridyl
L 5-11 : 2-Ethoxy-3-pyridyl
L 5-12 : 2-Chlor-3-pyridyl
L 5-13 : 2-Methoxycarbonyl-3-pyridyl
L 5-14 : 2-Ethoxycarbonyl-3-pyridyl
L 5-15 : 2-Methoxy-3-pyridyl
L 5-16 : 2-Methylsulfonyl-3-pyridyl
L 5-17 : 3-Chlor-2-pyridyl
L 5-18 : 3-Brom-2-pyridyl
L 5-19 : 3-Jod-2-pyridyl
L 5-20 : 2-Brom-3-pyridyl
L 5-21 : 2-Fluor-3-pyridyl
L 5-22 : 2-Jod-3-pyridyl
L 5-23 : 3-Fluor-2-pyridyl
L 5-24 : 2-Trifluormethyl-3-pyridyl
L 5-25 : 2-Pyridyl
L 5-26 : 3-Thiomethyl-2-pyridyl
L 5-27 : 3-Thioethyl-2-pyridyl
L 5-28 : 3-Ethylsulfonyl-2-pyridyl
L 5-29 : 3-Trifluormethoxy-2-pyridyl

Tabelle 2

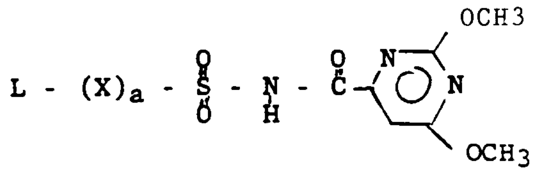

| Bsp.-Nr. | L | X | a | Schmp. [°C] |
|---|---|---|---|---|
| 1 | L1-1 | - | 0 | 124 - 126 |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | 149 - 152 |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | 170 - 174 |
| 16 | L1-16 | - | 0 | 173 - 174 |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | 172 - 180 |
| 26 | L1-26 | - | 0 | 200 - 202 |
| 27 | L1-27 | - | 0 | |
| 28 | L1-28 | - | 0 | 143 - 148 |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | 175 - 182 |
| 36 | L1-36 | - | 0 | |
| 37 | L1-37 | - | 0 | 187 - 188 |
| 38 | L1-38 | - | 0 | 188 - 191 |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | 198 - 200 |
| 45 | L1-45 | - | 0 | 154 - 156 |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 2, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | 152 - 153 |
| 54 | L1-54 | - | 0 | 183 - 185 |
| 55 | L1-55 | - | 0 | |
| 56 | L1-56 | - | 0 | |
| 57 | L1-57 | - | 0 | |
| 58 | L1-58 | - | 0 | 163 - 166 |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | 126 - 127 |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | 164 - 167 |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | |
| 84 | L1-16 | $CH_2$ | 1 | |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | 177 - 178 |
| 94 | L1-26 | $CH_2$ | 1 | 181 - 185 |
| 95 | L1-27 | $CH_2$ | 1 | |
| 96 | L1-28 | $CH_2$ | 1 | 172 - 177 |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 2, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | |
| 122 | L1-54 | $CH_2$ | 1 | 177 |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | 157 - 159 |
| 128 | L1-60 | $CH_2$ | 1 | |
| 129 | L1-61 | $CH_2$ | 1 | |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 2, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 2, Fortsetzung

| Bsp.-Nr. | L | X | a | Schmp. [°C] |
|---|---|---|---|---|
| 212 | L1-8 | NH | 1 | 133 - 135 |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | 111 -113 |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 2, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | 0 | |
| 274 | L3-2 | - | 0 | 166 - 169 |
| 275 | L3-3 | - | 0 | |
| 276 | L3-4 | - | 0 | |
| 277 | L3-5 | - | 0 | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | 0 | |
| 294 | L4-2 | - | 0 | |
| 295 | L4-3 | - | 0 | |
| 296 | L4-4 | - | 0 | |
| 297 | L4-5 | - | 0 | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-4 | NH | 1 | |

Tabelle 2, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 2, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

**Tabelle 3**

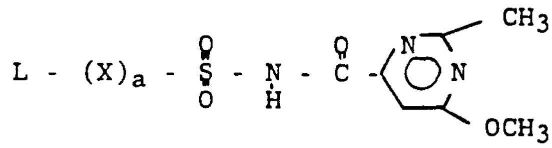

| Bsp.-Nr. | L | X | a | Schmp. [°C] |
|---|---|---|---|---|
| 1 | L1-1 | - | 0 | |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | 159 - 162 |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | |
| 27 | L1-27 | - | 0 | |
| 28 | L1-28 | - | 0 | 178 - 180 |
| 29 | L1-29 | - | 0 | |
| 30 | L1-30 | - | 0 | |
| 31 | L1-31 | - | 0 | |
| 32 | L1-32 | - | 0 | |
| 33 | L -33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 3, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | 120 - 122 |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | 142 - 144 |
| 84 | L1-16 | $CH_2$ | 1 | 117 - 119 |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | |
| 95 | L1-27 | $CH_2$ | 1 | |
| 96 | L1-28 | $CH_2$ | 1 | 141 - 142 |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 3, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | 165 - 1 6 |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | |
| 122 | L1-54 | $CH_2$ | 1 | |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | |
| 128 | L1-60 | $CH_2$ | 1 | |
| 129 | L1-61 | $CH_2$ | 1 | |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 15[illegible] | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 3, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 3, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 3, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | 0 | |
| 274 | L3-2 | - | 0 | |
| 275 | L3-3 | - | 0 | |
| 276 | L3-4 | - | 0 | |
| 277 | L3-5 | - | 0 | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | 0 | |
| 294 | L4-2 | - | 0 | |
| 295 | L4-3 | - | 0 | |
| 296 | L4-4 | - | 0 | |
| 297 | L4-5 | - | 0 | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 3, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 3, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

**Tabelle 4**

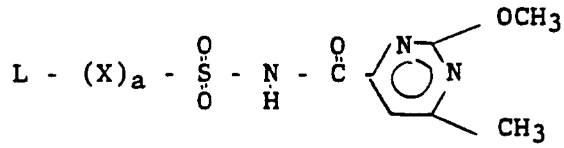

| Bsp.-Nr. | L | X | a | Schmp. [°C] |
|---|---|---|---|---|
| 1 | L1-1 | - | 0 | 145 - 146 |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | 168-169 |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | 158 - 160 |
| 27 | L1-27 | - | 0 | |
| 28 | L1-28 | - | 0 | 212 - 216 |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | 174 - 176 |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 4, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | 138 - 140 |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | 115 - 118 |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | 171 - 172 |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | 184 - 187 |
| 84 | L1-16 | $CH_2$ | 1 | 133 - 134 |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | 171 - 173 |
| 95 | L1-27 | $CH_2$ | 1 | |
| 96 | L1-28 | $CH_2$ | 1 | 178 - 179 |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 4, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | 146 - 148 |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | 163 - 166 |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | 151 - 153 |
| 121 | L1-53 | $CH_2$ | 1 | 100 - 101 |
| 122 | L1-54 | $CH_2$ | 1 | 162 - 164 |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | 170 - 172 |
| 128 | L1-60 | $CH_2$ | 1 | |
| 129 | L1-61 | $CH_2$ | 1 | 150 |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 4, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 4, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | 133 - 134 |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | 170 |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | 154 - 156 |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 4, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | O | |
| 274 | L3-2 | - | O | |
| 275 | L3-3 | - | O | |
| 276 | L3-4 | - | O | |
| 277 | L3-5 | - | O | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | O | |
| 294 | L4-2 | - | O | |
| 295 | L4-3 | - | O | |
| 296 | L4-4 | - | O | |
| 297 | L4-5 | - | O | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 4, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | 170 - 172 |
| 343 | L5-7 | $CH_2$ | 1 | 235 - 236 |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 4, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

Tabelle 5

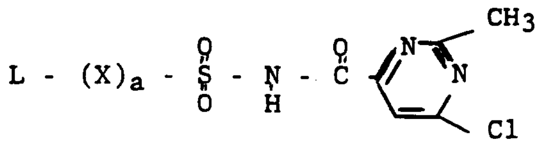

| Bsp.-Nr. | L | X | a | Schmp. [°C] |
|---|---|---|---|---|
| 1 | L1-1 | - | 0 | |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | 134 - 135 |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | |
| 27 | L1-27 | - | 0 | |
| 28 | L1-28 | - | 0 | |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 5, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | 186 - 189 |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | |
| 84 | L1-16 | $CH_2$ | 1 | |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | |
| 95 | L1-27 | $CH_2$ | 1 | |
| 96 | L1-28 | $CH_2$ | 1 | 190 - 192 |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 5, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | |
| 122 | L1-54 | $CH_2$ | 1 | |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | |
| 128 | L1-60 | $CH_2$ | 1 | |
| 129 | L1-61 | $CH_2$ | 1 | |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 13[illegible] | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 5, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 5, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 5, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | 0 | |
| 274 | L3-2 | - | 0 | |
| 275 | L3-3 | - | 0 | |
| 276 | L3-4 | - | 0 | |
| 277 | L3-5 | - | 0 | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | 0 | |
| 294 | L4-2 | - | 0 | |
| 295 | L4-3 | - | 0 | |
| 296 | L4-4 | - | 0 | |
| 297 | L4-5 | - | 0 | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 5, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 5, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

Tabelle 6

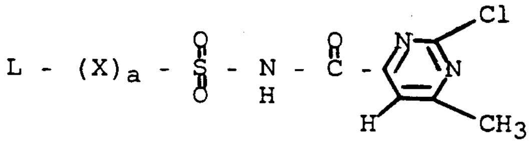

| Bsp.-Nr. | L | X | a | Schmp. [°C] |
|---|---|---|---|---|
| 1 | L1-1 | - | 0 | 144 - 145 |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | 182 - 184 |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | 125 - 128 |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | 165 - 166 |
| 27 | L1-27 | - | 0 | |
| 28 | L1-28 | - | 0 | 181 - 182 |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 6, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | 126 - 127 |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | 198 - 199 |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | 162 |
| 84 | L1-16 | $CH_2$ | 1 | 132 - 133 |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | 182 - 183 |
| 95 | L1-27 | $CH_2$ | 1 | |
| 96 | L1-28 | $CH_2$ | 1 | 184 - 186 |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 6, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | 145 - 146 |
| 122 | L1-54 | $CH_2$ | 1 | 174 - 176 |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | |
| 128 | L1-60 | $CH_2$ | 1 | |
| 129 | L1-61 | $CH_2$ | 1 | 171 - 172 |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 6, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 6, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 6, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | O | |
| 274 | L3-2 | - | O | |
| 275 | L3-3 | - | O | |
| 276 | L3-4 | - | O | |
| 277 | L3-5 | - | O | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | O | |
| 294 | L4-2 | - | O | |
| 295 | L4-3 | - | O | |
| 296 | L4-4 | - | O | |
| 297 | L4-5 | - | O | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 6, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 6, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

Tabelle 7

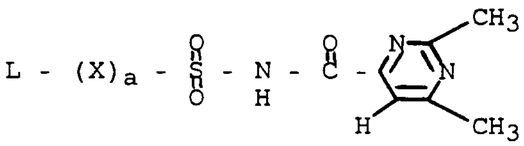

| Bsp.-Nr. | L | X | a | Schmp. [°C] |
|---|---|---|---|---|
| 1 | L1-1 | - | 0 | 152 |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | 147 - 150 |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | 164 - 165 |
| 27 | L1-27 | - | 0 | |
| 28 | L1-28 | - | 0 | 154 - 157 |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | 166 - 167 |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 7, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | 155 |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | 145 |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | |
| 84 | L1-16 | $CH_2$ | 1 | |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | 175 - 179 |
| 95 | L1-27 | $CH_2$ | 1 | |
| 96 | L1-28 | $CH_2$ | 1 | 200 - 206 |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 7, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | |
| 122 | L1-54 | $CH_2$ | 1 | 167 - 169 |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | |
| 128 | L1-60 | $CH_2$ | 1 | |
| 129 | L1-61 | $CH_2$ | 1 | |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 7, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 7, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 7, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | 0 | |
| 274 | L3-2 | - | 0 | |
| 275 | L3-3 | - | 0 | |
| 276 | L3-4 | - | 0 | |
| 277 | L3-5 | - | 0 | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | 0 | |
| 294 | L4-2 | - | 0 | |
| 295 | L4-3 | - | 0 | |
| 296 | L4-4 | - | 0 | |
| 297 | L4-5 | - | 0 | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 7, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 7, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

Tabelle 8

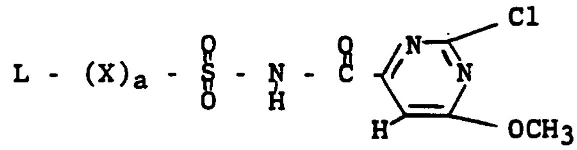

| Bsp.-Nr. | L | X | a | Schmp. [°C] |
|---|---|---|---|---|
| 1 | L1-1 | - | 0 | |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | |
| 27 | L1-27 | - | 0 | |
| 28 | L1-28 | - | 0 | |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 8, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | |
| 84 | L1-16 | $CH_2$ | 1 | |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | |
| 95 | L1-27 | $CH_2$ | 1 | |
| 96 | L1-28 | $CH_2$ | 1 | |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 8, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | |
| 122 | L1-54 | $CH_2$ | 1 | |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | |
| 128 | L1-60 | $CH_2$ | 1 | |
| 129 | L1-61 | $CH_2$ | 1 | |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | 1 | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 8, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 8, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 8, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | 0 | |
| 274 | L3-2 | - | 0 | |
| 275 | L3-3 | - | 0 | |
| 276 | L3-4 | - | 0 | |
| 277 | L3-5 | - | 0 | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | 0 | |
| 294 | L4-2 | - | 0 | |
| 295 | L4-3 | - | 0 | |
| 296 | L4-4 | - | 0 | |
| 297 | L4-5 | - | 0 | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 8, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 8, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

Tabelle 9

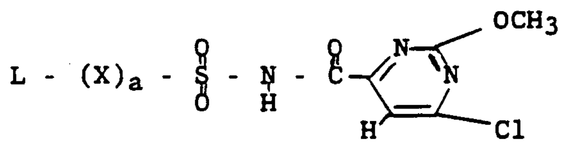

| Bsp.-Nr. | L | X | a | Schmp. [°C] |
|---|---|---|---|---|
| 1 | L1-1 | - | 0 | |
| 2 | L1-2 | - | 0 | |
| 3 | L1-3 | - | 0 | |
| 4 | L1-4 | - | 0 | |
| 5 | L1-5 | - | 0 | |
| 6 | L1-6 | - | 0 | |
| 7 | L1-7 | - | 0 | |
| 8 | L1-8 | - | 0 | |
| 9 | L1-9 | - | 0 | |
| 10 | L1-10 | - | 0 | |
| 11 | L1-11 | - | 0 | |
| 12 | L1-12 | - | 0 | |
| 13 | L1-13 | - | 0 | |
| 14 | L1-14 | - | 0 | |
| 15 | L1-15 | - | 0 | |
| 16 | L1-16 | - | 0 | |
| 17 | L1-17 | - | 0 | |
| 18 | L1-18 | - | 0 | |
| 19 | L1-19 | - | 0 | |
| 20 | L1-20 | - | 0 | |
| 21 | L1-21 | - | 0 | |
| 22 | L1-22 | - | 0 | |
| 23 | L1-23 | - | 0 | |
| 24 | L1-24 | - | 0 | |
| 25 | L1-25 | - | 0 | |
| 26 | L1-26 | - | 0 | |
| 27 | L1-27 | - | 0 | |
| 28 | L1-28 | - | 0 | |
| 29 | L1-29 | - | 0 | |
| 30 | L1-30 | - | 0 | |
| 31 | L1-31 | - | 0 | |
| 32 | L1-32 | - | 0 | |
| 33 | L1-33 | - | 0 | |
| 34 | L1-34 | - | 0 | |
| 35 | L1-35 | - | 0 | |
| 36 | L1-36 | - | 0 | |
| 37 | L1-37 | - | 0 | |
| 38 | L1-38 | - | 0 | |
| 39 | L1-39 | - | 0 | |
| 40 | L1-40 | - | 0 | |
| 41 | L1-41 | - | 0 | |
| 42 | L1-42 | - | 0 | |
| 43 | L1-43 | - | 0 | |
| 44 | L1-44 | - | 0 | |
| 45 | L1-45 | - | 0 | |
| 46 | L1-46 | - | 0 | |
| 47 | L1-47 | - | 0 | |
| 48 | L1-48 | - | 0 | |
| 49 | L1-49 | - | 0 | |
| 50 | L1-50 | - | 0 | |

Tabelle 9, Fortsetzung

| Bsp.-Nr. | L | X | a | Schmp. [°C] |
|---|---|---|---|---|
| 51 | L1-51 | - | 0 | |
| 52 | L1-52 | - | 0 | |
| 53 | L1-53 | - | 0 | |
| 54 | L1-54 | - | 0 | |
| 55 | L1-55 | - | 0 | |
| 56 | L1-56 | - | 0 | |
| 57 | L1-57 | - | 0 | |
| 58 | L1-58 | - | 0 | |
| 59 | L1-59 | - | 0 | |
| 60 | L1-60 | - | 0 | |
| 61 | L1-61 | - | 0 | |
| 62 | L1-62 | - | 0 | |
| 63 | L1-63 | - | 0 | |
| 64 | L1-64 | - | 0 | |
| 65 | L1-65 | - | 0 | |
| 66 | L1-66 | - | 0 | |
| 67 | L1-67 | - | 0 | |
| 68 | L1-68 | - | 0 | |
| 69 | L1-1 | $CH_2$ | 1 | |
| 70 | L1-2 | $CH_2$ | 1 | |
| 71 | L1-3 | $CH_2$ | 1 | |
| 72 | L1-4 | $CH_2$ | 1 | |
| 73 | L1-5 | $CH_2$ | 1 | |
| 74 | L1-6 | $CH_2$ | 1 | |
| 75 | L1-7 | $CH_2$ | 1 | |
| 76 | L1-8 | $CH_2$ | 1 | |
| 77 | L1-9 | $CH_2$ | 1 | |
| 78 | L1-10 | $CH_2$ | 1 | |
| 79 | L1-11 | $CH_2$ | 1 | |
| 80 | L1-12 | $CH_2$ | 1 | |
| 81 | L1-13 | $CH_2$ | 1 | |
| 82 | L1-14 | $CH_2$ | 1 | |
| 83 | L1-15 | $CH_2$ | 1 | |
| 84 | L1-16 | $CH_2$ | 1 | |
| 85 | L1-17 | $CH_2$ | 1 | |
| 86 | L1-18 | $CH_2$ | 1 | |
| 87 | L1-19 | $CH_2$ | 1 | |
| 88 | L1-20 | $CH_2$ | 1 | |
| 89 | L1-21 | $CH_2$ | 1 | |
| 90 | L1-22 | $CH_2$ | 1 | |
| 91 | L1-23 | $CH_2$ | 1 | |
| 92 | L1-24 | $CH_2$ | 1 | |
| 93 | L1-25 | $CH_2$ | 1 | |
| 94 | L1-26 | $CH_2$ | 1 | |
| 95 | L1-27 | $CH_2$ | 1 | |
| 96 | L1-28 | $CH_2$ | 1 | |
| 97 | L1-29 | $CH_2$ | 1 | |
| 98 | L1-30 | $CH_2$ | 1 | |
| 99 | L1-31 | $CH_2$ | 1 | |
| 100 | L1-32 | $CH_2$ | 1 | |
| 101 | L1-33 | $CH_2$ | 1 | |
| 102 | L1-34 | $CH_2$ | 1 | |
| 103 | L1-35 | $CH_2$ | 1 | |
| 104 | L1-36 | $CH_2$ | 1 | |

Tabelle 9, Fortsetzung

| Bsp.-Nr. | L | X | a | Schmp. [°C] |
|---|---|---|---|---|
| 105 | L1-37 | $CH_2$ | 1 | |
| 106 | L1-38 | $CH_2$ | 1 | |
| 107 | L1-39 | $CH_2$ | 1 | |
| 108 | L1-40 | $CH_2$ | 1 | |
| 109 | L1-41 | $CH_2$ | 1 | |
| 110 | L1-42 | $CH_2$ | 1 | |
| 111 | L1-43 | $CH_2$ | 1 | |
| 112 | L1-44 | $CH_2$ | 1 | |
| 113 | L1-45 | $CH_2$ | 1 | |
| 114 | L1-46 | $CH_2$ | 1 | |
| 115 | L1-47 | $CH_2$ | 1 | |
| 116 | L1-48 | $CH_2$ | 1 | |
| 117 | L1-49 | $CH_2$ | 1 | |
| 118 | L1-50 | $CH_2$ | 1 | |
| 119 | L1-51 | $CH_2$ | 1 | |
| 120 | L1-52 | $CH_2$ | 1 | |
| 121 | L1-53 | $CH_2$ | 1 | |
| 122 | L1-54 | $CH_2$ | 1 | |
| 123 | L1-55 | $CH_2$ | 1 | |
| 124 | L1-56 | $CH_2$ | 1 | |
| 125 | L1-57 | $CH_2$ | 1 | |
| 126 | L1-58 | $CH_2$ | 1 | |
| 127 | L1-59 | $CH_2$ | 1 | |
| 128 | L1-60 | $CH_2$ | 1 | |
| 129 | L1-61 | $CH_2$ | 1 | |
| 130 | L1-62 | $CH_2$ | 1 | |
| 131 | L1-63 | $CH_2$ | 1 | |
| 132 | L1-64 | $CH_2$ | 1 | |
| 133 | L1-65 | $CH_2$ | 1 | |
| 134 | L1-66 | $CH_2$ | 1 | |
| 135 | L1-67 | $CH_2$ | 1 | |
| 136 | L1-68 | $CH_2$ | 1 | |
| 137 | L1-1 | O | 1 | |
| 138 | L1-2 | O | 1 | |
| 139 | L1-3 | O | 1 | |
| 140 | L1-4 | O | 1 | |
| 141 | L1-5 | O | 1 | |
| 142 | L1-6 | O | 1 | |
| 143 | L1-7 | O | 1 | |
| 144 | L1-8 | O | 1 | |
| 145 | L1-9 | O | 1 | |
| 146 | L1-10 | O | 1 | |
| 147 | L1-11 | O | 1 | |
| 148 | L1-12 | O | 1 | |
| 149 | L1-13 | O | 1 | |
| 150 | L1-14 | O | 1 | |
| 151 | L1-15 | O | [illegible] | |
| 152 | L1-16 | O | 1 | |
| 153 | L1-17 | O | 1 | |
| 154 | L1-18 | O | 1 | |
| 155 | L1-19 | O | 1 | |
| 156 | L1-20 | O | 1 | |
| 157 | L1-21 | O | 1 | |
| 158 | L1-22 | O | 1 | |

Tabelle 9, Fortsetzung

| Bsp.-Nr. | L | X | a | Schmp. [°C] |
|---|---|---|---|---|
| 159 | L1-23 | O | 1 | |
| 160 | L1-24 | O | 1 | |
| 161 | L1-25 | O | 1 | |
| 162 | L1-26 | O | 1 | |
| 163 | L1-27 | O | 1 | |
| 164 | L1-28 | O | 1 | |
| 165 | L1-29 | O | 1 | |
| 166 | L1-30 | O | 1 | |
| 167 | L1-31 | O | 1 | |
| 168 | L1-32 | O | 1 | |
| 169 | L1-33 | O | 1 | |
| 170 | L1-34 | O | 1 | |
| 171 | L1-35 | O | 1 | |
| 172 | L1-36 | O | 1 | |
| 173 | L1-37 | O | 1 | |
| 174 | L1-38 | O | 1 | |
| 175 | L1-39 | O | 1 | |
| 176 | L1-40 | O | 1 | |
| 177 | L1-41 | O | 1 | |
| 178 | L1-42 | O | 1 | |
| 179 | L1-43 | O | 1 | |
| 180 | L1-44 | O | 1 | |
| 181 | L1-45 | O | 1 | |
| 182 | L1-46 | O | 1 | |
| 183 | L1-47 | O | 1 | |
| 184 | L1-48 | O | 1 | |
| 185 | L1-49 | O | 1 | |
| 186 | L1-50 | O | 1 | |
| 187 | L1-51 | O | 1 | |
| 188 | L1-52 | O | 1 | |
| 189 | L1-53 | O | 1 | |
| 190 | L1-54 | O | 1 | |
| 191 | L1-55 | O | 1 | |
| 192 | L1-56 | O | 1 | |
| 193 | L1-57 | O | 1 | |
| 194 | L1-58 | O | 1 | |
| 195 | L1-59 | O | 1 | |
| 196 | L1-60 | O | 1 | |
| 197 | L1-61 | O | 1 | |
| 198 | L1-62 | O | 1 | |
| 199 | L1-63 | O | 1 | |
| 200 | L1-64 | O | 1 | |
| 201 | L1-65 | O | 1 | |
| 202 | L1-66 | O | 1 | |
| 203 | L1-67 | O | 1 | |
| 204 | L1-68 | O | 1 | |
| 205 | L1-1 | NH | 1 | |
| 206 | L1-2 | NH | 1 | |
| 207 | L1-3 | NH | 1 | |
| 208 | L1-4 | NH | 1 | |
| 209 | L1-5 | NH | 1 | |
| 210 | L1-6 | NH | 1 | |
| 211 | L1-7 | NH | 1 | |

Tabelle 9, Fortsetzung

| Bsp.-Nr. | L | X | a | Schmp. [°C] |
|---|---|---|---|---|
| 212 | L1-8 | NH | 1 | |
| 213 | L1-9 | NH | 1 | |
| 214 | L1-10 | NH | 1 | |
| 215 | L1-11 | NH | 1 | |
| 216 | L1-12 | NH | 1 | |
| 217 | L1-13 | NH | 1 | |
| 218 | L1-14 | NH | 1 | |
| 219 | L1-15 | NH | 1 | |
| 220 | L1-16 | NH | 1 | |
| 221 | L1-17 | NH | 1 | |
| 222 | L1-18 | NH | 1 | |
| 223 | L1-19 | NH | 1 | |
| 224 | L1-20 | NH | 1 | |
| 225 | L1-21 | NH | 1 | |
| 226 | L1-22 | NH | 1 | |
| 227 | L1-23 | NH | 1 | |
| 228 | L1-24 | NH | 1 | |
| 229 | L1-25 | NH | 1 | |
| 230 | L1-26 | NH | 1 | |
| 231 | L1-27 | NH | 1 | |
| 232 | L1-28 | NH | 1 | |
| 233 | L1-29 | NH | 1 | |
| 234 | L1-30 | NH | 1 | |
| 235 | L1-31 | NH | 1 | |
| 236 | L1-32 | NH | 1 | |
| 237 | L1-33 | NH | 1 | |
| 238 | L1-34 | NH | 1 | |
| 239 | L1-35 | NH | 1 | |
| 240 | L1-36 | NH | 1 | |
| 241 | L1-37 | NH | 1 | |
| 242 | L1-38 | NH | 1 | |
| 243 | L1-39 | NH | 1 | |
| 244 | L1-40 | NH | 1 | |
| 245 | L1-41 | NH | 1 | |
| 246 | L1-42 | NH | 1 | |
| 247 | L1-43 | NH | 1 | |
| 248 | L1-44 | NH | 1 | |
| 249 | L1-45 | NH | 1 | |
| 250 | L1-46 | NH | 1 | |
| 251 | L1-47 | NH | 1 | |
| 252 | L1-48 | NH | 1 | |
| 253 | L1-49 | NH | 1 | |
| 254 | L1-50 | NH | 1 | |
| 255 | L1-51 | NH | 1 | |
| 256 | L1-52 | NH | 1 | |
| 257 | L1-53 | NH | 1 | |
| 258 | L1-54 | NH | 1 | |
| 259 | L1-55 | NH | 1 | |
| 260 | L1-56 | NH | 1 | |
| 261 | L1-57 | NH | 1 | |
| 262 | L1-58 | NH | 1 | |
| 263 | L1-59 | NH | 1 | |
| 264 | L1-60 | NH | 1 | |

Tabelle 9, Fortsetzung

| Bsp.-Nr. | L | X | a | Schmp. [°C] |
|---|---|---|---|---|
| 265 | L1-61 | NH | 0 | |
| 266 | L1-62 | NH | 0 | |
| 267 | L1-63 | NH | 1 | |
| 268 | L1-64 | NH | 1 | |
| 269 | L1-65 | NH | 1 | |
| 270 | L1-66 | NH | 1 | |
| 271 | L1-67 | NH | 1 | |
| 272 | L1-68 | NH | 1 | |
| 273 | L3-1 | - | 0 | |
| 274 | L3-2 | - | 0 | |
| 275 | L3-3 | - | 0 | |
| 276 | L3-4 | - | 0 | |
| 277 | L3-5 | - | 0 | |
| 278 | L3-1 | $CH_2$ | 1 | |
| 279 | L3-2 | $CH_2$ | 1 | |
| 280 | L3-3 | $CH_2$ | 1 | |
| 281 | L3-4 | $CH_2$ | 1 | |
| 282 | L3-5 | $CH_2$ | 1 | |
| 283 | L3-1 | O | 1 | |
| 284 | L3-2 | O | 1 | |
| 285 | L3-3 | O | 1 | |
| 286 | L3-4 | O | 1 | |
| 287 | L3-5 | NH | 1 | |
| 288 | L3-1 | NH | 1 | |
| 289 | L3-2 | NH | 1 | |
| 290 | L3-3 | NH | 1 | |
| 291 | L3-4 | NH | 1 | |
| 292 | L3-5 | NH | 1 | |
| 293 | L4-1 | - | 0 | |
| 294 | L4-2 | - | 0 | |
| 295 | L4-3 | - | 0 | |
| 296 | L4-4 | - | 0 | |
| 297 | L4-5 | - | 0 | |
| 298 | L4-1 | $CH_2$ | 1 | |
| 299 | L4-2 | $CH_2$ | 1 | |
| 300 | L4-3 | $CH_2$ | 1 | |
| 301 | L4-4 | $CH_2$ | 1 | |
| 302 | L4-5 | $CH_2$ | 1 | |
| 303 | L4-1 | O | 1 | |
| 304 | L4-2 | O | 1 | |
| 305 | L4-3 | O | 1 | |
| 306 | L4-4 | O | 1 | |
| 307 | L4-5 | O | 1 | |
| 308 | L4-1 | NH | 1 | |
| 309 | L4-2 | NH | 1 | |
| 310 | L4-3 | NH | 1 | |
| 311 | L4-4 | NH | 1 | |
| 312 | L4-5 | NH | 1 | |

Tabelle 9, Fortsetzung

| Bsp.-Nr. | L | X | a | Schmp. [°C] |
|---|---|---|---|---|
| 313 | L5-1 | - | 0 | |
| 314 | L5-2 | - | 0 | |
| 315 | L5-3 | - | 0 | |
| 316 | L5-4 | - | 0 | |
| 317 | L5-5 | - | 0 | |
| 318 | L5-6 | - | 0 | |
| 319 | L5-7 | - | 0 | |
| 320 | L5-8 | - | 0 | |
| 321 | L5-9 | - | 0 | |
| 322 | L5-10 | - | 0 | |
| 323 | L5-11 | - | 0 | |
| 324 | L5-12 | - | 0 | |
| 325 | L5-13 | - | 0 | |
| 326 | L5-14 | - | 0 | |
| 327 | L5-15 | - | 0 | |
| 328 | L5-16 | - | 0 | |
| 329 | L5-17 | - | 0 | |
| 330 | L5-18 | - | 0 | |
| 331 | L5-19 | - | 0 | |
| 332 | L5-20 | - | 0 | |
| 333 | L5-21 | - | 0 | |
| 334 | L5-22 | - | 0 | |
| 335 | L5-23 | - | 0 | |
| 336 | L5-24 | - | 0 | |
| 337 | L5-1 | $CH_2$ | 1 | |
| 338 | L5-2 | $CH_2$ | 1 | |
| 339 | L5-3 | $CH_2$ | 1 | |
| 340 | L5-4 | $CH_2$ | 1 | |
| 341 | L5-5 | $CH_2$ | 1 | |
| 342 | L5-6 | $CH_2$ | 1 | |
| 343 | L5-7 | $CH_2$ | 1 | |
| 344 | L5-8 | $CH_2$ | 1 | |
| 345 | L5-9 | $CH_2$ | 1 | |
| 346 | L5-10 | $CH_2$ | 1 | |
| 347 | L5-11 | $CH_2$ | 1 | |
| 348 | L5-12 | $CH_2$ | 1 | |
| 349 | L5-13 | $CH_2$ | 1 | |
| 350 | L5-14 | $CH_2$ | 1 | |
| 351 | L5-15 | $CH_2$ | 1 | |
| 352 | L5-16 | $CH_2$ | 1 | |
| 353 | L5-17 | $CH_2$ | 1 | |
| 354 | L5-18 | $CH_2$ | 1 | |
| 355 | L5-19 | $CH_2$ | 1 | |
| 356 | L5-20 | $CH_2$ | 1 | |
| 357 | L5-21 | $CH_2$ | 1 | |
| 358 | L5-22 | $CH_2$ | 1 | |
| 359 | L5-23 | $CH_2$ | 1 | |
| 360 | L5-24 | $CH_2$ | 1 | |

Tabelle 9, Fortsetzung

| Bsp.-Nr. | L | X | a | Schmp. [°C] |
|---|---|---|---|---|
| 361 | L5-1 | O | 1 | |
| 362 | L5-2 | O | 1 | |
| 363 | L5-3 | O | 1 | |
| 364 | L5-4 | O | 1 | |
| 365 | L5-5 | O | 1 | |
| 366 | L5-6 | O | 1 | |
| 367 | L5-7 | O | 1 | |
| 368 | L5-8 | O | 1 | |
| 369 | L5-9 | O | 1 | |
| 370 | L5-10 | O | 1 | |
| 371 | L5-11 | O | 1 | |
| 372 | L5-12 | O | 1 | |
| 373 | L5-13 | O | 1 | |
| 374 | L5-14 | O | 1 | |
| 375 | L5-15 | O | 1 | |
| 376 | L5-16 | O | 1 | |
| 377 | L5-17 | O | 1 | |
| 378 | L5-18 | O | 1 | |
| 379 | L5-19 | O | 1 | |
| 380 | L5-20 | O | 1 | |
| 381 | L5-21 | O | 1 | |
| 382 | L5-22 | O | 1 | |
| 383 | L5-23 | O | 1 | |
| 384 | L5-24 | O | 1 | |
| 385 | L5-1 | NH | 1 | |
| 386 | L5-2 | NH | 1 | |
| 387 | L5-3 | NH | 1 | |
| 388 | L5-4 | NH | 1 | |
| 389 | L5-5 | NH | 1 | |
| 390 | L5-6 | NH | 1 | |
| 391 | L5-7 | NH | 1 | |
| 192 | L5-8 | NH | 1 | |
| 393 | L5-9 | NH | 1 | |
| 394 | L5-10 | NH | 1 | |
| 395 | L5-11 | NH | 1 | |
| 396 | L5-12 | NH | 1 | |
| 397 | L5-13 | NH | 1 | |
| 398 | L5-14 | NH | 1 | |
| 399 | L5-15 | NH | 1 | |
| 400 | L5-16 | NH | 1 | |
| 401 | L5-17 | NH | 1 | |
| 402 | L5-18 | NH | 1 | |
| 403 | L5-19 | NH | 1 | |
| 404 | L5-20 | NH | 1 | |
| 405 | L5-21 | NH | 1 | |
| 406 | L5-22 | NH | 1 | |
| 407 | L5-23 | NH | 1 | |
| 408 | L5-24 | NH | 1 | |

Tabelle 10: Beispiele für Salze mit Basen

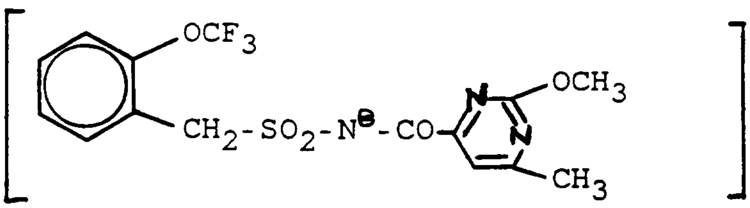

$R^{1\oplus}$

| Bsp.-Nr. | $R^1$ | Schmp. [°C] |
|---|---|---|
| 1 | Na | 188 - 189 (Zers.) |
| 2 | K | 251 - 253 |
| 3 | $(C_2H_5)_3NH$ | Öl |
| 4 | $[CH(CH_3)_2]_2NH_2$ | 108 - 111 |
| 5 | Li | 227 - 229 |

Tabelle 11: Beispiele für Säureadditionssalze

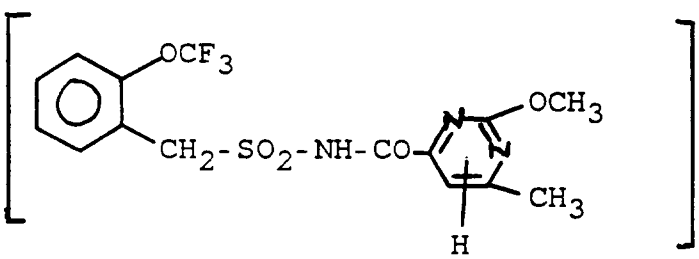

$\oplus$ $RCO_2^{\ominus}$

| Bsp.-Nr. | R | Schmp. [°C] |
|---|---|---|
| 1 | $CH_3$ | 88 - 95 |
| 2 | $n-C_{15}H_{31}$ | 114 - 116 |

Tabelle 12: Beispiele für Zwischenprodukte der Formel III ($R^1$ = H; X = $CH_2$; a = 1)

$L-CH_2-SO_2-NH_2$

| Bsp.-Nr. | L | Schmp. [°C] |
|---|---|---|
| 1 | L 5-1 | |
| 2 | L 5-2 | |
| 3 | L 5-3 | |
| 4 | L 5-4 | |
| 5 | L 5-5 | |
| 6 | L 5-6 | 245 - 246 (Zers.) |
| 7 | L 5-7 | 126 - 128 |
| 8 | L 5-8 | 160 |
| 9 | L 5-9 | |
| 10 | L 5-10 | |
| 11 | L 5-11 | |
| 12 | L 5-12 | |
| 13 | L 5-13 | |
| 14 | L 5-14 | |
| 15 | L 5-15 | |
| 16 | L 5-16 | |
| 17 | L 5-17 | |
| 18 | L 5-18 | |
| 19 | L 5-19 | |
| 20 | L 5-20 | |
| 21 | L 5-21 | |
| 22 | L 5-22 | |
| 23 | L 5-23 | |
| 24 | L 5-24 | |
| 25 | L 5-25 | 165 - 168 |
| 26 | L 5-26 | |
| 27 | L 5-27 | |
| 28 | L 5-28 | |
| 29 | L 5-29 | |

C. Biologische Beispiele

1. Herbizide Wirkung

Die Schädigung der Unkrautpflanzen bzw. die Kulturpflanzenverträglichkeit wurde gemäß einem Schlüssel bonitiert, in dem die Wirksamkeit durch Wertzahlen von 0-5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 -100 % Wirkung bzw. Schaden.

1.1 Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 - 4 Wochen im Vergleich zu unbehandelten Kontrollen.

Wie die Boniturwerte in Tabelle 13 zeigen, weisen die erfindungsgemäßen Verbindungen eine gute herbizide Vorauflaufwirksamkeit gegen ein breites Spektrum von Ungräsern und Unkräutern auf.

Tabelle 13: Herbizide Wirkung der erfindungsgemäßen Verbindungen im Vorauflauf

| Tabelle | Bsp. Nr. | Dosis (kg a.i./ha) | herbizide Wirkung STME | CRSE | SIAL | LOMU | ECCR | AVSA |
|---|---|---|---|---|---|---|---|---|
| 2 | 1 | 0,3 | 5 | 2 | 5 | 5 | 3 | 1 |
| 2 | 28 | 0,3 | 4 | 3 | 5 | 3 | 5 | 1 |
| 2 | 37 | 0,3 | 3 | 2 | 5 | 3 | 2 | 1 |
| 2 | 8 | 0,3 | 5 | 5 | 5 | 5 | 3 | 3 |
| 2 | 76 | 0,3 | 5 | 5 | 5 | 4 | 3 | 2 |
| 2 | 16 | 0,3 | 5 | 3 | 5 | 2 | 2 | 3 |
| 2 | 69 | 0,3 | 5 | 5 | 5 | 2 | 5 | 3 |
| 2 | 212 | 0,3 | 4 | 5 | 4 | 3 | 4 | 3 |
| 2 | 122 | 0,3 | 4 | 5 | 4 | 4 | 4 | 3 |
| 2 | 44 | 0,3 | 5 | 5 | 5 | 5 | 5 | 4 |
| 2 | 35 | 0,3 | 5 | 4 | 3 | 4 | 3 | 1 |
| 2 | 96 | 0,3 | 4 | 4 | 4 | 3 | 2 | 3 |
| 2 | 54 | 0,3 | 5 | 5 | 5 | 4 | 2 | 1 |
| 4 | 8 | 0,3 | 4 | 5 | 5 | 3 | 2 | 0 |
| 4 | 76 | 0,3 | 5 | 5 | 5 | 5 | 5 | 1 |
| 3 | 127 | 0,3 | 5 | 5 | 5 | 2 | 3 | 3 |
| 4 | 122 | 0,3 | 5 | 5 | 5 | 5 | 5 | 1 |
| 4 | 1 | 0,3 | 3 | 1 | 5 | 1 | 1 | 1 |
| 4 | 44 | 0,3 | 5 | 5 | 5 | 5 | 5 | 1 |
| 4 | 96 | 0,3 | 5 | 5 | 5 | 5 | 4 | 3 |
| 4 | 69 | 0,3 | 5 | 5 | 5 | 5 | 5 | 4 |
| 4 | 26 | 0,3 | 4 | 5 | 5 | 4 | 3 | 3 |
| 4 | 94 | 0,3 | 5 | 5 | 5 | 5 | 5 | 3 |
| 7 | 8 | 0,3 | 5 | 5 | 5 | 3 | 3 | 2 |
| 4 | 127 | 0,3 | 5 | 5 | 5 | 2 | 2 | 1 |

Fortsetzung von Tabelle 13:

| Tabelle | Bsp. Nr. | Dosis (kg a.i./ha) | herbizide Wirkung STME | CRSE | SIAL | LOMU | ECCR | AVSA |
|---|---|---|---|---|---|---|---|---|
| 2 | 15 | 0,3 | 4 | 2 | 3 | 2 | 2 | 1 |
| 7 | 76 | 0,3 | 5 | 5 | 5 | 5 | 5 | 2 |
| 4 | 28 | 0,3 | 4 | 3 | 4 | 4 | 3 | 3 |
| 4 | 112 | 0,3 | 2 | 2 | 5 | 1 | 1 | 1 |
| 7 | 26 | 0,3 | 5 | 5 | 5 | 4 | 4 | 2 |
| 7 | 122 | 0,3 | 5 | 5 | 5 | 5 | 5 | 2 |
| 7 | 1 | 0,3 | 1 | 2 | 4 | 2 | 2 | 2 |
| 4 | 121 | 0,3 | 5 | 5 | 5 | 5 | 5 | 3 |
| 4 | 53 | 0,3 | 3 | 3 | 3 | 3 | 2 | 3 |
| 5 | 76 | 0,3 | 3 | 4 | 5 | 2 | 3 | 2 |
| 5 | 96 | 0,3 | 5 | 3 | 5 | 3 | 2 | 3 |
| 5 | 8 | 0,3 | 1 | 2 | 2 | 2 | 2 | 1 |
| 2 | 53 | 0,3 | 5 | 5 | 5 | 5 | 2 | 2 |
| 6 | 28 | 0,3 | 2 | 3 | 2 | 2 | 2 | 3 |
| 6 | 76 | 0,3 | 5 | 5 | 5 | 5 | 5 | 3 |
| 4 | 84 | 0,3 | 5 | 5 | 5 | 5 | 5 | 4 |
| 4 | 83 | 0,3 | 5 | 5 | 5 | 5 | 5 | 4 |
| 6 | 8 | 0,3 | 5 | 4 | 5 | 4 | 4 | 3 |
| 3 | 28 | 0,3 | 5 | 4 | 5 | 3 | 5 | 2 |
| 3 | 84 | 0,3 | 5 | 5 | 5 | 4 | 4 | 3 |
| 3 | 76 | 0,3 | 5 | 4 | 4 | 3 | 4 | 3 |
| 3 | 8 | 0,3 | 5 | 5 | 5 | 3 | 5 | 3 |
| 6 | 122 | 0,3 | 5 | 5 | 5 | 5 | 5 | 4 |
| 4 | 343 | 0,3 | 5 | 5 | 5 | 5 | 5 | 5 |
| 4 | 230 | 0,3 | 4 | 4 | 4 | 5 | 2 | 2 |
| 4 | 342 | 0,3 | 5 | 5 | 5 | 5 | 5 | 5 |
| 6 | 121 | 0,3 | 5 | 5 | 5 | 5 | 5 | 3 |
| 6 | 69 | 0,3 | 5 | 5 | 5 | 5 | 5 | 3 |
| 6 | 94 | 0,3 | 4 | 5 | 5 | 5 | 4 | 3 |
| 6 | 1 | 0,3 | 3 | 4 | 4 | 2 | 3 | 3 |

Fortsetzung von Tabelle 13:

| Tabelle | Bsp. Nr. | Dosis (kg a.i./ha) | herbizide Wirkung STME | CRSE | SIAL | LOMU | ECCR | AVSA |
|---|---|---|---|---|---|---|---|---|
| 6 | 26 | 0,3 | 3 | 5 | 5 | 4 | 4 | 3 |
| 4 | 106 | 0,3 | 5 | 5 | 5 | 5 | 5 | 3 |
| 6 | 84 | 0,3 | 5 | 5 | 5 | 3 | 5 | 2 |
| 4 | 258 | 0,3 | 3 | 5 | 5 | 2 | 1 | 2 |
| 7 | 69 | 0,3 | 2 | 5 | 3 | 2 | 4 | 1 |
| 4 | 129 | 0,3 | 5 | 5 | 5 | 5 | 5 | 2 |
| 10 | 1 | 0,3 | 5 | 5 | 5 | 5 | 5 | 2 |
| 10 | 2 | 0,3 | 4 | 5 | 5 | 5 | 3 | 2 |
| 10 | 3 | 0,3 | 5 | 5 | 5 | 5 | 3 | 3 |
| 10 | 4 | 0,3 | 5 | 5 | 5 | 5 | 3 | 3 |
| 11 | 1 | 0,3 | 5 | 5 | 5 | 5 | 3 | 3 |
| 11 | 2 | 0,3 | 4 | 5 | 5 | 5 | 3 | 3 |
| 10 | 5 | 0,3 | 5 | 5 | 5 | 5 | 3 | 2 |
| 4 | 120 | 0,3 | 5 | 5 | 5 | 5 | 5 | 2 |
| 6 | 83 | 0,3 | 4 | 5 | 5 | 3 | 5 | 3 |

Abkürzungen:
STME = Stellaria media
CRSE = Chrysanthemum segetum
SIAL = Sinapis alba
LOMU = Lolium multiflorum
ECCR = Echinochloa crus-galli
AVSA = Avena sativa
a.i. = Aktivsubstanz

1.2 Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 - 800 l/ha auf die grünen Pflanzenteile gesprüht und nach ca. 3 - 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 14).

Tabelle 14: Herbizide Wirkung der erfindungsgemäßen Verbindungen im Nachauflauf

| Tabelle | Bsp. Nr. | Dosis (ka a.i./ha) | herbizide Wirkung STME | CRSE | SIAL | LOMU | ECCR | AVSA |
|---|---|---|---|---|---|---|---|---|
| 2 | 26 | 0,3 | 5 | 5 | 5 | 2 | 3 | 0 |
| 2 | 1 | 0,3 | 5 | 5 | 5 | 5 | 5 | 2 |
| 2 | 38 | 0,3 | 5 | 5 | 5 | 3 | 3 | 2 |
| 2 | 28 | 0,3 | 3 | 5 | 4 | 3 | 4 | 1 |
| 2 | 37 | 0,3 | 4 | 1 | 4 | 3 | 2 | 1 |
| 2 | 94 | 0,3 | 3 | 5 | 4 | 2 | 1 | 1 |
| 2 | 8 | 0,3 | 5 | 4 | 5 | 5 | 3 | 2 |
| 2 | 76 | 0,3 | 4 | 5 | 5 | 4 | 2 | 2 |
| 2 | 16 | 0,3 | 4 | 3 | 5 | 1 | 3 | 1 |
| 2 | 69 | 0,3 | 4 | 3 | 5 | 2 | 4 | 1 |
| 2 | 122 | 0,3 | 2 | 4 | 4 | 4 | 1 | 1 |
| 2 | 44 | 0,3 | 3 | 2 | 5 | 4 | 3 | 1 |
| 2 | 35 | 0,3 | 5 | 4 | 4 | 2 | 2 | 1 |
| 2 | 93 | 0,3 | 5 | 3 | 4 | 1 | 1 | 2 |
| 2 | 220 | 0,3 | 5 | 5 | 4 | 2 | 2 | 2 |
| 3 | 96 | 0,3 | 3 | 2 | 4 | 1 | 1 | 2 |
| 2 | 54 | 0,3 | 4 | 3 | 5 | 4 | 1 | 2 |
| 4 | 8 | 0,3 | 3 | 5 | 5 | 2 | 1 | 1 |
| 4 | 76 | 0,3 | 5 | 5 | 5 | 5 | 5 | 1 |
| 2 | 127 | 0,3 | 5 | 5 | 5 | 1 | 4 | 1 |
| 4 | 122 | 0,3 | 5 | 5 | 5 | 5 | 4 | 1 |
| 4 | 1 | 0,3 | 4 | 3 | 4 | 1 | 0 | 0 |
| 4 | 44 | 0,3 | 5 | 4 | 5 | 5 | 2 | 0 |
| 4 | 96 | 0,3 | 4 | 4 | 5 | 3 | 3 | 2 |
| 4 | 69 | 0,3 | 5 | 5 | 5 | 3 | 5 | 2 |
| 4 | 26 | 0,3 | 3 | 5 | 5 | 2 | 3 | 1 |
| 4 | 94 | 0,3 | 5 | 5 | 5 | 2 | 3 | 2 |
| 4 | 127 | 0,3 | 5 | 5 | 5 | 1 | 2 | 1 |

Fortsetzung von Tabelle 14:

| Tabelle | Bsp. Nr. | Dosis (ka a.i./ha) | herbizide Wirkung STME | CHSE | SIAL | LOMU | ECCR | AVSA |
|---|---|---|---|---|---|---|---|---|
| 2 | 15 | 0,3 | 4 | 0 | 5 | 1 | 0 | 1 |
| 7 | 76 | 0,3 | 3 | 5 | 5 | 3 | 4 | 1 |
| 4 | 28 | 0,3 | 2 | 1 | 4 | 1 | 1 | 0 |
| 4 | 112 | 0,3 | 2 | 2 | 5 | 0 | 3 | 3 |
| 7 | 26 | 0,3 | 5 | 4 | 5 | 4 | 3 | 2 |
| 7 | 122 | 0,3 | 5 | 5 | 5 | 5 | 3 | 2 |
| 7 | 1 | 0,3 | 2 | 2 | 4 | 1 | 2 | 2 |
| 4 | 121 | 0,3 | 5 | 5 | 5 | 5 | 4 | 2 |
| 4 | 53 | 0,3 | 4 | 5 | 0 | 0 | 3 | 2 |
| 5 | 76 | 0,3 | 2 | 3 | 4 | 0 | 3 | 1 |
| 5 | 96 | 0,3 | 2 | 2 | 4 | 0 | 3 | 2 |
| 5 | 8 | 0,3 | 3 | 2 | 5 | 1 | 3 | 2 |
| 2 | 53 | 0,3 | 1 | 4 | 5 | 1 | 1 | 0 |
| 6 | 28 | 0,3 | 2 | 4 | 4 | 0 | 3 | 1 |
| 6 | 76 | 0.3 | 5 | 5 | 5 | 5 | 5 | 3 |
| 4 | 84 | 0,3 | 5 | 5 | 5 | 3 | 4 | 2 |
| 4 | 83 | 0,3 | 5 | 5 | 5 | 5 | 5 | 2 |
| 6 | 8 | 0,3 | 4 | 5 | 5 | 4 | 5 | 2 |
| 3 | 28 | 0,3 | 4 | 5 | 5 | 3 | 4 | 2 |
| 3 | 84 | 0,3 | 4 | 4 | 5 | 2 | 2 | 2 |
| 3 | 76 | 0,3 | 5 | 4 | 4 | 2 | 3 | 2 |
| 3 | 8 | 0,3 | 3 | 4 | 5 | 2 | 4 | 1 |
| 6 | 122 | 0,3 | 5 | 5 | 5 | 5 | 4 | 3 |
| 4 | 343 | 0,3 | 5 | 5 | 5 | 5 | 5 | 4 |
| 4 | 230 | 0,3 | 2 | 4 | 3 | 1 | 2 | 2 |
| 4 | 342 | 0,3 | 5 | 5 | 5 | 5 | 5 | 4 |
| 6 | 121 | 0,3 | 5 | 5 | 5 | 5 | 4 | 2 |
| 6 | 69 | 0,3 | 3 | 5 | 5 | 5 | 4 | 3 |
| 6 | 94 | 0,3 | 3 | 5 | 5 | 2 | 3 | 2 |
| 6 | 1 | 0,3 | 2 | 4 | 5 | 2 | 2 | 2 |

Fortsetzung von Tabelle 14:

| Tabelle | Bsp. Nr. | Dosis (ka a.i./ha) | herbizide Wirkung STME | CHSE | SIAL | LOMU | ECCR | AVSA |
|---|---|---|---|---|---|---|---|---|
| 6 | 26 | 0,3 | 4 | 5 | 5 | 3 | 4 | 2 |
| 4 | 106 | 0,3 | 3 | 5 | 5 | 3 | 4 | 2 |
| 6 | 84 | 0,3 | 3 | 5 | 5 | 2 | 4 | 1 |
| 4 | 258 | 0,3 | 3 | 4 | 3 | 2 | 3 | 2 |
| 7 | 69 | 0,3 | 3 | 3 | 4 | 3 | 4 | 2 |
| 4 | 129 | 0,3 | 5 | 5 | 5 | 5 | 5 | 3 |
| 10 | 1 | 0,3 | 5 | 5 | 5 | 5 | 3 | 1 |
| 10 | 2 | 0,3 | 5 | 5 | 5 | 5 | 3 | 1 |
| 10 | 3 | 0,3 | 5 | 5 | 5 | 5 | 4 | 1 |
| 10 | 4 | 0,3 | 5 | 5 | 5 | 5 | 4 | 1 |
| 11 | 1 | 0,3 | 5 | 5 | 5 | 5 | 4 | 1 |
| 11 | 2 | 0,3 | 5 | 5 | 5 | 4 | 3 | 1 |
| 10 | 5 | 0,3 | 5 | 5 | 5 | 5 | 5 | 1 |
| 4 | 120 | 0,3 | 4 | 5 | 5 | 3 | 2 | 2 |
| 6 | 83 | 0,3 | 3 | 5 | 4 | 1 | 2 | 2 |

Abkürzungen: STME = Stellaria media
CRSE = Chrysanthemum segetum
SIAL = Sinapis alba
LOMU = Lolium multiflorum
ECCR = Echinochloa crus-galli
AVSA = Avena sativa
a.i. = Aktivsubstanz

2. Fungizide Wirkung

Gerstenpflanzen wurden im 2-Blattstadium mit Konidien des Gerstenmehltaus (Erysiphe graminis hordei) stark inokuliert und in einem Gewächshaus bei 20 °C und einer relativen Luftfeuchte von ca. 50 % weiterkultiviert. 1 Tag nach Inokulation wurden die Pflanzen mit den in Tabelle 15 aufgeführten Verbindungen in den angegebenen Wirkstoffkonzentrationen gleichmäßig benetzt. Nach einer Inkubationszeit von 7 - 9 Tagen wurden die Pflanzen auf Befall mit Gerstenmehltau untersucht. Der Befallsgrad wird ausgedrückt in % befallener Blattfläche, bezogen auf unbehandelte, infizierte Kontrollpflanzen (= 100 % Befall). Das Ergebnis ist in Tabelle 15 zusammengefaßt.

Tabelle 15: Fungizide Wirkung gegen Gerstenmehltau

| Verbindungen gemäß Tabelle/Beispiel | mit Gerstenmehltau befallene Blattfläche in % bei 500 bzw. 250 mg Wirkstoff/Liter Spritzbrühe | |
|---|---|---|
| | 500 | 250 |
| 10/1 | 0 | 0 |
| 10/2 | 0 | 0 |
| 10/3 | 0 | 0 |
| 10/4 | 0 | 0 |
| 11/1 | 0 | 0 |
| 11/2 | 0 | 0 |
| 10/5 | 0 | 0 |
| Kontrolle mit unbehandelten, infizierten Pflanzen | 100 | |

## Patentansprüche

1. Verbindungen der Formel I oder deren Salze

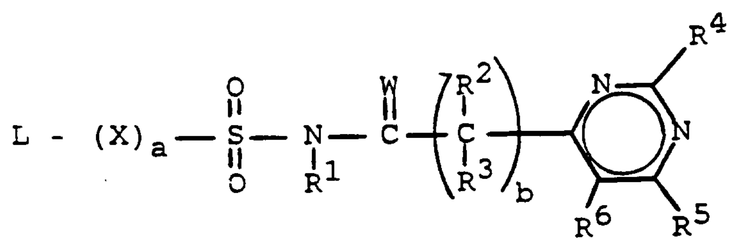

(I)

worin

| | |
|---|---|
| $R^1$ | Wasserstoff, $(C_1\text{-}C_4)$Alkyl, $(C_2\text{-}C_6)$Alkenyl oder $(C_2\text{-}C_6)$Alkinyl; |
| $R^2$, $R^3$ | unabhängig voneinander Wasserstoff, $(C_1\text{-}C_3)$Alkyl oder Phenyl; |
| W | O, S, $NR^7$ oder $NOR^7$; |
| X | $CHR^2$, O, $NR^7$ oder $NOR^7$; |
| $R^4$, $R^5$ | unabhängig voneinander Wasserstoff, Hydroxy, Halogen, $(C_1\text{-}C_6)$Alkyl, $(C_1\text{-}C_6)$Alkoxy oder $(C_1\text{-}C_6)$Alkylthio, wobei die drei vorgenannten Reste ein- oder mehrfach durch Halogen oder ein- oder zweifach durch $(C_1\text{-}C_4)$Alkoxy oder $(C_1\text{-}C_4)$Alkylthio substituiert sein können; $-NR^8R^9$, $(C_3\text{-}C_6)$Cycloalkyl, $-OCHR^8CO_2R^{10}$, $(C_2\text{-}C_5)$Alkenyl, $(C_2\text{-}$ |

$C_4$)Alkinyl, ($C_3$-$C_5$)Alkenyloxy oder ($C_3$-$C_5$)Alkinyloxy;

$R^6$ Wasserstoff, Halogen, ($C_1$-$C_3$)Alkyl, ($C_1$-$C_3$)Alkoxy, -CN, -$NO_2$, -CO-$R^{11}$, $CO_2R^{12}$, ($C_1$-$C_3$)Alkylthio, -SO-$R^{12}$, -$SO_2$-$R^{12}$ oder -CO-$NR^8R^9$;

$R^7$ Wasserstoff, ($C_1$-$C_3$)Alkyl, ($C_1$-$C_3$)Haloalkyl oder Phenyl;

$R^8$ Wasserstoff oder ($C_1$-$C_4$)Alkyl oder $R^8$ und $R^9$ zusammen -$(CH_2)_2(CH_2)_c(CH_2)_2$- oder -$(CH_2)_2O(CH_2)_2$-;

$R^9$ Wasserstoff, ($C_1$-$C_4$)Alkyl, ($C_1$-$C_4$)Alkoxy, ($C_2$-$C_4$)Alkenyl oder $R^8$ und $R^9$ zusammen -$(CH_2)_2(CH_2)_c(CH_2)_2$- oder -$(CH_2)_2O(CH_2)_2$-;

$R^{10}$ Wasserstoff, ($C_1$-$C_4$)Alkyl, das unsubstituiert oder ein- oder mehrfach durch Halogen oder ein- bis zweifach durch CN, $CO_2$-$R^{12}$, -$NR^8R^9$, $OR^{12}$ oder $Si(CH_3)_3$ substituiert ist; ($C_3$-$C_4$)Alkinyl oder ($C_3$-$C_4$)Alkenyl, wobei die zwei vorgenannten Reste unsubstituiert oder durch $CH_3$ oder -$Si(CH_3)_3$ substituiert sind; ($C_3$-$C_6$)Cycloalkyl, das unsubstituiert oder durch ($C_1$-$C_4$)Alkyl oder ($C_1$-$C_4$)Alkoxy substituiert ist; oder $Si(CH_3)_3$;

$R^{11}$ Wasserstoff, ($C_1$-$C_4$)Alkyl, ($C_1$-$C_4$)Alkyl, das ein- oder mehrfach durch Halogen oder ($C_1$-$C_3$)Alkoxy substituiert ist; ($C_3$-$C_6$)Cycloalkyl, das unsubstituiert oder durch Halogen oder $CH_3$ ein- oder mehrfach substitutiert ist, ($C_2$-$C_4$)Alkenyl, ($C_2$-$C_4$)Alkinyl;

$R^{12}$ ($C_1$-$C_3$) Alkyl;

L einen hetero- oder isocyclischen Rest der Formeln (L1) bis (L5)

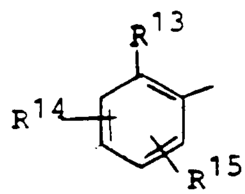

(L1)

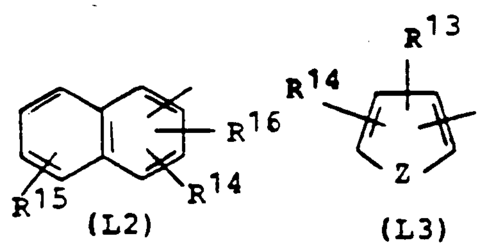

(L2) (L3)

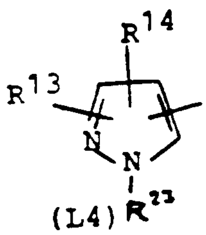

(L4)

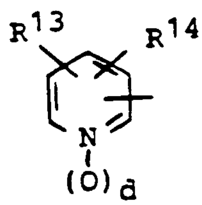

(L5)

$R^{13}$ Wasserstoff, Halogen, $NO_2$, CN, ($C_1$-$C_4$)Alkyl, das unsubstituiert oder ein- oder mehrfach durch Halogen oder einfach durch CN, $OCH_3$ oder $SCH_3$ substituiert ist; ($C_2$-$C_4$)Alkenyl, das unsubstituiert oder ein- oder mehrfach durch Halogen oder einfach durch $OCH_3$ substituiert ist; ($C_2$-$C_4$)Alkinyl, das unsubstituiert oder ein- oder mehrfach durch Halogen oder einfach durch $OCH_3$ oder $Si(CH_3)_3$ substituiert ist; ($C_3$-$C_6$)Cycloalkyl, das unsubstituiert oder ein- oder mehrfach durch Halogen oder $CH_3$ substituiert ist; -CO-$R^{11}$, -$OCH_2CH_2OR^{11}$, -OH, -$C(R^{11})(OR^{17})(OR^{18})$; -$CO_2R^{10}$, -CO-$NR^8R^9$, -$N_3$, $SO_2NR^8R^9$, -$SO_3R^{19}$, -$OSO_2R^{20}$;Phenyl, das unsubstituiert oder ein- oder mehrfach durch Halogen, $CH_3$ oder $OCH_3$ substituiert ist; E-$R^{21}$ oder -$(CH_2)_e$-G;

$R^{14}$ Wasserstoff, Halogen; CN, $NO_2$, ($C_1$-$C_4$)Alkyl, das unsubstituiert oder ein- oder mehrfach durch Halogen oder ein- bis zweifach durch -$CO_2R^{10}$, -$SO_2NR^8R^9$, -$NR^8R^9$, ($C_1$-$C_2$)Alkoxy, -E-$R^{22}$, ($C_1$-$C_2$)Haloalkoxy, ($C_1$-$C_2$)Alkylthio, ($C_1$-$C_2$)-Haloalkylthio, -CN, -OH oder SH substituiert ist; -$CO_2R^{10}$, -$SO_2NR^8R^9$, -$NR^8R^9$ oder -E-$R^{22}$;

$R^{15}$ Wasserstoff, ($C_1$-$C_4$)Alkyl, ($C_1$-$C_4$)Haloalkyl, ($C_2$-$C_4$)Alkenyl, Phenyl oder Phenyl, das ein- oder mehrfach durch Halogen oder -E-$R^{22}$ substituiert ist; -E-$R^{22}$ oder Halogen;

| | |
|---|---|
| $R^{16}$ | Wasserstoff, $(C_1-C_4)$Alkyl, $(C_1-C_4)$Haloalkyl, Halogen, $-CO_2R^{10}$, $-SO_2NR^8R^9$, $-OSO_2R^{20}$, $-E-R^{22}$, -CN oder $-NO_2$; |
| E, Z | unabhängig voneinander O oder $S(O)_f$; |
| a, b, c, d, e | unabhängig voneinander 0 oder 1; |
| f | 0, 1 oder 2; |
| $R^{17}$, $R^{18}$ | unabhängig voneinander $(C_1-C_4)$Alkyl, oder $R^{17}$ und $R^{18}$ zusammen $-CH_2CH_2-$, $-CH_2OCH_2-$ oder $-CH_2-C(CH_3)_2CH_2-$; |
| $R^{19}$ | $(C_1-C_4)$Alkyl oder $(C_1-C_4)$Haloalkyl, |
| $R^{20}$ | $(C_1-C_4)$Alkyl, $-NR^8R^9$ oder $(C_1-C_4)$Haloalkyl |
| $R^{21}$ | $(C_1-C_4)$Alkyl, $(C_1-C_4)$Haloalkyl, $(C_2-C_4)$Alkoxyalkyl, $(C_2-C_4)$Alkenyl, $(C_3-C_4)$Alkinyl, Phenyl oder Phenyl, das ein- oder mehrfach durch Halogen $(C_1-C_3)$Alkyl, $(C_1-C_3)$-Alkoxy oder $(C_1-C_3)$Haloalkyl substituiert ist; $(C_2-C_4)$Haloalkenyl; |
| $R^{22}$ | $(C_1-C_4)$Alkyl oder $(C_1-C_4)$Alkyl, das ein- oder mehrfach durch Halogen oder einfach durch $OR^{19}$ substituiert ist; |
| $R^{23}$ | Wasserstoff, $(C_1-C_4)$Alkyl, das unsubstituiert oder ein- oder mehrfach durch Halogen oder einfach durch Phenyl substituiert ist, $(C_2-C_4)$Alkenyl, Phenyl oder Phenyl, das ein- oder mehrfach durch Halogen, $(C_1-C_4)$Alkyl, $(C_1-C_4)$Haloalkyl, $-NO_2$, -CN oder $(C_1-C_4)$Alkoxy substituiert ist, |
| G | einen heterocyclischen Rest aus der Gruppe (G1)-(G25); |

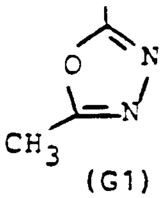
(G1)

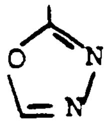
(G2)

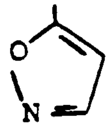
(G3)

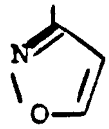
(G4)

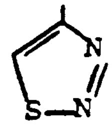
(G5)

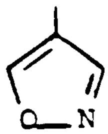
(G6)

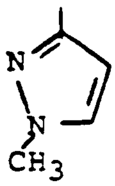
(G7)

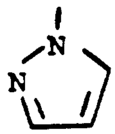
(G8)

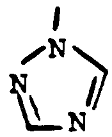
(G9)

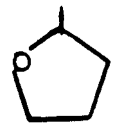
(G10)

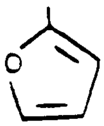
(G11)

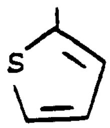
(G12)

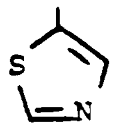
(G13)

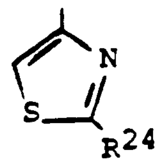
(G14)

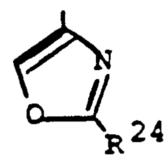
(G15)

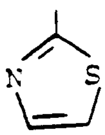
(G16)

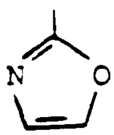
(G17)

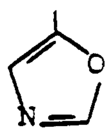
(G18)

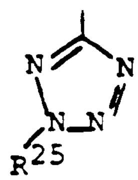
(G19)

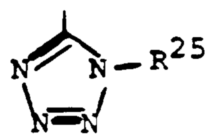
(G20)

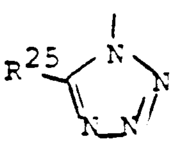
(G21)

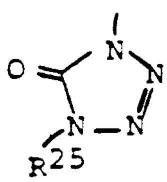
(G22)

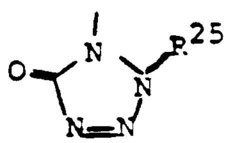
(G23)

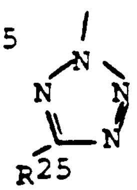
(G24)

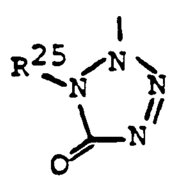
(G25)

und

$R^{24}$ Wasserstoff oder $(C_1\text{-}C_4)$Alkyl;

$R^{25}$ Wasserstoff, $(C_1\text{-}C_3)$Alkyl oder $(C_2\text{-}C_4)$Alkenyl;

bedeuten.

**2.** Verbindungen oder deren Salze nach Anspruch 1, dadurch gekennzeichnet, daß

L einen Rest der Formeln (L1), (L3), (L4) oder (L5);

X $CH_2$, $CH(CH_3)$, O, NH, $NCH_3$, $NC_2H_5$, $NOCH_3$, insbesondere $CH_2$, O oder NH;

W Sauerstoff;

$R^1$ Wasserstoff;

$R^2$, $R^3$ unabhängig voneinander Wasserstoff oder $(C_1\text{-}C_3)$Alkyl, insbesonders Wasserstoff;

| | |
|---|---|
| $R^4$, $R^5$ | unabhängig voneinander Wasserstoff, $(C_1\text{-}C_2)$Alkyl, $(C_1\text{-}C_2)$Alkoxy, $-OCH_2OCH_3$, $-CH_2OCH_3$, F, Cl, Br, I, $-CH_2OCH_2CH_3$, $-NHCH_3$, $-N(CH_3)_2$, $-N(CH_3)OCH_3$, $-CF_3$, $-SCH_3$, $-CH(OCH_3)_2$, $-OCH_2CH=CH_2$, $-OCH_2C\equiv CH$, $-OCH_2CH_2OCH_3$, $-CH_2SCH_3$, $-OCHF_2$, $-SCHF_2$, Cyclopropyl, $-C\equiv CH$ oder $-C\equiv C\text{-}CH_3$; |
| $R^6$ | Wasserstoff, Halogen, $-CH_3$, $-OCH_3$, $-NO_2$, -CN, -CHO, $-CO_2CH_3$, $CO_2C_2H_5$ oder $-SCH_3$; |
| $R^8$ | Wasserstoff oder $-CH_3$ oder $R^8$ und $R^9$ zusammen $-(CH_2)_4-$, $-(CH_2)_5-$ oder $-(CH_2)_2O(CH_2)_2-$; |
| $R^9$ | $-CH_3$, $-CH_2CH_3$, $-OCH_3$ oder $R^8$ und $R^9$ zusammen $-(CH_2)_4-$, $-(CH_2)_5-$ oder $-(CH_2)_2O(CH_2)_2-$; |
| $R^{10}$ | $(C_1\text{-}C_3)$Alkyl, $(C_3)$Alkenyl, $(C_3)$Alkinyl, $-CH_2CH_2F$, $-CH_2CH_2Cl$, $-CH_2CH_2OCH_3$, $-CH_2CH_2Si(CH_3)_3$ oder Cyclopropylmethyl; |
| $R^{11}$ | $(C_1\text{-}C_3)$Alkyl, Wasserstoff, Cyclopropyl oder $(C_3)$Alkenyl; |
| $R^{12}$ | $-CH_3$, $-CH_2CH_3$, $-CH_2CH_2CH_3$ oder $-CH(CH_3)_2$; |
| $R^{13}$ | Halogen, $-NO_2$, -CN, $(C_1\text{-}C_3)$Alkyl, das unsubstituiert oder durch F, Cl, Br, CN, $OCH_3$ oder $SCH_3$ substituiert ist; $(C_3)$Alkenyl, das unsubstituiert oder durch F, Cl oder Br substituiert ist, $(C_3)$Alkinyl, Cyclopropyl, das unsubstituiert oder durch F, Cl oder $CH_3$ substituiert ist; $-C(O)R^{11}$, $-OCH_2CH_2OR^{11}$, -OH, $C(R^{11})(OR^{17})(OR^{18})$, $-CO_2R^{10}$, $-CO\text{-}NR^8R^9$, $-N_3$, $-SO_2NR^8R^9$, $-OSO_2R^{20}$, $-E\text{-}R^{21}$ oder $-(CH_2)_eG$; |
| $R^{14}$ | Wasserstoff, Halogen, -CN, $-NO_2$, $-CH_3$, $-CF_3$, $-E\text{-}R^{22}$, oder $(C_1\text{-}C_2)$Alkoxy-$(C_1\text{-}C_2)$alkyl, $-CO_2R^{10}$ oder $SO_2NR^8R^9$; |
| $R^{15}$ | Wasserstoff; |
| $R^{17}$, $R^{18}$ | unabhängig voneinander $(C_1\text{-}C_2)$Alkyl oder $R^{17}$ und $R^{18}$ zusammen $-CH_2CH_2-$; |
| $R^{20}$ | $(C_1\text{-}C_3)$Alkyl, $(C_1\text{-}C_3)$Haloalkyl; |
| $R^{21}$ | $(C_1\text{-}C_3)$Alkyl, $(C_1\text{-}C_3)$Haloalkyl, $(C_2\text{-}C_3)$Alkoxyalkyl, Allyl, Propargyl, $(C_2\text{-}C_3)$-Haloalkenyl; |
| $R^{22}$ | $(C_1\text{-}C_2)$Alkyl, das unsubstituiert oder durch F, Cl oder $OCH_3$ substituiert ist; |
| $R^{23}$ | Wasserstoff, $(C_1\text{-}C_3)$Alkyl, Phenyl oder Phenyl, das ein- oder mehrfach durch Halogen, $-NO_2$, $(C_1\text{-}C_3)$Alkyl, $(C_1\text{-}C_3)$Haloalkyl oder $(C_1\text{-}C_3)$Alkoxy substituiert ist; |
| $R^{24}$ | Wasserstoff oder $(C_1\text{-}C_3)$Alkyl; |
| $R^{25}$ | Wasserstoff oder $(C_1\text{-}C_3)$Alkyl; |
| Z | S; |
| d | 0 und |
| e | 0 bedeuten |
| und E, a, b, f und G | wie in Anspruch 1 definiert sind. |

**3.** Verbindungen oder deren Salze nach Anspruch 1 oder 2, dadurch gekennzeichnet, daß $R^4$ und $R^5$ unabhängig voneinander Wasserstoff, $CH_3$, $OCH_3$, $CH_2CH_3$, $OCH_2CH_3$, $OCHF_2$, $OCH_2OCH_3$,$CH_2OCH_3$, $NHCH_3$, $CH(OCH_3)_2$, Cl oder Cyclopropyl bedeuten.

**4.** Verbindungen oder deren Salze nach einem der Ansprüche 1 bis 3, dadurch gekennzeichnet, daß L ein isocyclischer Rest der Formel L1 ist.

**5.** Verfahren zur Herstellung der nach Anspruch 1 definierten Verbindungen oder deren Salze, dadurch gekennzeichnet, daß man

a) zur Herstellung von Verbindungen mit W = O

$a_1$) eine Verbindung der Formel II mit

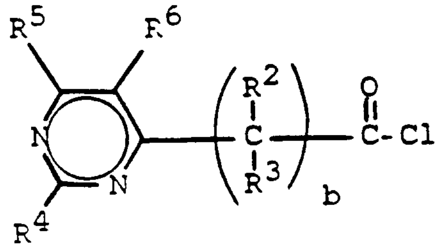

(II)

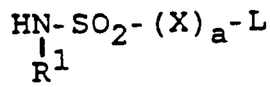

(III)

einer Verbindung der Formel III in Gegenwart einer Base umsetzt oder

$a_2$) eine Verbindung der Formel (IIa)

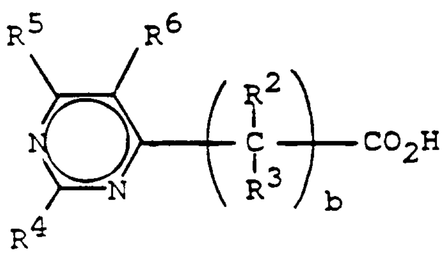

(IIa)

in Gegenwart von aktivierenden Reagentien und gegebenenfalls in Gegenwart einer Base mit einer Verbindung der Formel III umsetzt oder

$a_3$) zur Herstellung von Verbindungen mit W = O und $R^1$ = H eine Verbindung der Formel (V),

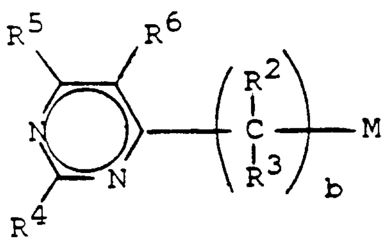

(V)

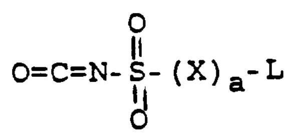

(VI)

worin M Wasserstoff (für b = 0) oder Lithium bedeutet, mit einer Verbindung der Formel (VI) umsetzt oder

b) zur Herstellung von Verbindungen mit W = S eine unter a) erhaltene Verbindung der Formel I mit $P_4S_{10}$ (VIIa) oder der Verbindung der Formel VIIb umsetzt

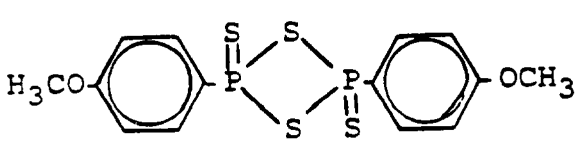

(VIIb)

c) zur Herstellung von Verbindungen mit W = $NR^7$ und $R^1$ = H eine Verbindung der Formel VIII

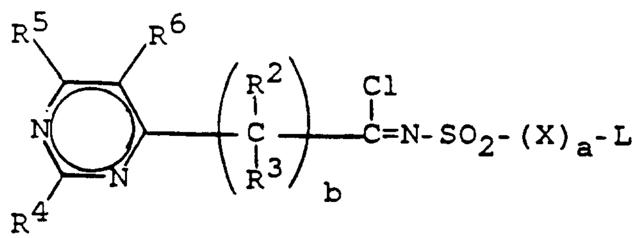

(VIII)

mit einem Amin der Formel $H_2N-R^7$ umsetzt,

d) zur Herstellung von Verbindungen mit W = $NOR^7$ und $R^1$ = H

$d_1$) im Falle $R^7$ = H eine Verbindung der Formel IX mit

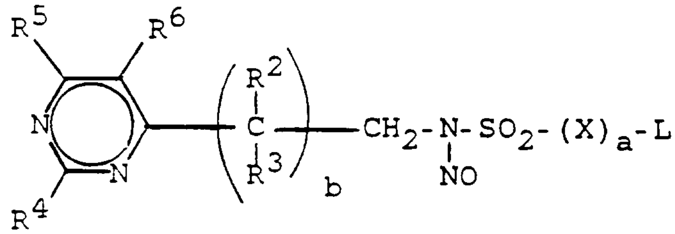

(IX)

einem Alkali- oder Erdalkalihydroxid oder Ammoniumhydroxid umsetzt oder

$d_2$) im Falle daß $R^7$ wie in Anspruch 1 definiert ist, eine Verbindung der Formel VIII in Gegenwart einer Base mit einem Hydroxylamin der Formel $H_2NOR^7$ umsetzt oder

e) zur Herstellung von Verbindungen der Formel I, worin $R^1$ ungleich Wasserstoff ist, eine Verbindung der Formel I mit $R^1$ = H in Gegenwart einer Base mit einem Alkylhalogenid der Formel $R^{1'}$-$X^1$, worin $R^{1'}$ die Bedeutung von $R^1$ außer Wasserstoff besitzt und $X^1$ Chlor, Brom oder Iod bedeutet, umsetzt.

**6.** Verwendung der nach einem oder mehreren der Ansprüche 1 bis 4 definierten Verbindungen der Formel I oder deren Salze als Herbizide oder Wachstumsregulatoren.

**7.** Verfahren zur Bekämpfung von unerwünschten Pflanzen oder zur Wachstumsregulation von Pflanzen, dadurch gekennzeichnet, daß man auf diese oder die Kulturböden eine wirksame Menge einer Verbindung der Formel I oder deren Salz nach einem oder mehreren der Ansprüche 1 bis 4 appliziert.

**8.** Herbizide oder pflanzenwachstumsregulierende Mittel, dadurch gekennzeichnet, daß sie eine Verbindung der Formel I oder deren Salz nach einem oder mehreren der Ansprüche 1 bis 4 und Formulierungshilfsmittel enthalten.

**9.** Verwendung der nach einem oder mehreren der Ansprüche 1 bis 4 definierten Verbindungen der Formel I oder deren Salze als Fungizide.

**10.** Fungizide Mittel, dadurch gekennzeichnet, daß sie eine wirksame Menge einer Verbindung der Formel I oder deren Salz nach einem oder mehreren der Ansprüche 1 bis 4 und Formulierungshilfsmittel enthalten.

**11.** Verfahren zur Bekämpfung von Schadpilzen, dadurch gekennzeichnet, daß man auf die von ihnen befallenen Pflanzen, Flächen oder Substrate eine fungizid wirksame Menge einer Verbindung der Formel I oder deren Salz gemäß einem oder mehreren der Ansprüche 1 bis 4 appliziert.

**12.** Verbindungen der Formel (II')

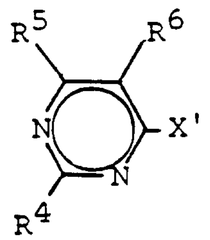

(II')

worin

X' -CO-Halogen, $-CO_2R^{10}$, $COO^-Me^+$, wobei $Me^+$ das Äquivalent eines Alkali- oder Erdalkalimetalls ist, oder $CO_2CH_2C_6H_5$,

$R^4$ Wasserstoff, Hydroxy, Halogen, $(C_1-C_6)$Alkyl, $(C_1-C_6)$Alkoxy oder $(C_1-C_6)$Alkylthio, wobei die drei vorgenannten Reste ein- oder mehrfach durch Halogen oder ein- oder zweifach durch $(C_1-C_4)$Alkoxy oder $(C_1-C_4)$Alkylthio substituiert sein können; $-NR^8R^9$, $(C_3-C_6)$Cycloalkyl, $-OCHR^8CO_2R^{10}$, $(C_2-C_5)$Alkenyl, $(C_2-C_4)$Alkinyl, $(C_3-C_5)$Alkenyloxy oder $(C_3-C_5)$Alkinyloxy, wobei $R^4$ nicht $NR^8R^9$ oder Halogen ist, wenn $R^5$ Halogen oder $NR^8R^9$ ist,

$R^5$ Hydroxy, Halogen, $(C_1-C_6)$Alkyl, $(C_1-C_6)$Alkoxy oder $(C_1-C_6)$Alkylthio, wobei die drei vorgenannten Reste ein- oder mehrfach durch Halogen oder ein- oder zweifach durch $(C_1-C_4)$Alkoxy oder $(C_1-C_4)$Alkylthio substituiert sein können; $-NR^8R^9$, $(C_3-C_6)$-Cycloalkyl, $-OCHR^8CO_2R^{10}$, $(C_2-C_5)$Alkenyl, $(C_2-C_4)$Alkinyl, $(C_3-C_5)$Alkenyloxy oder $(C_3-C_5)$Alkinyloxy; wobei $R^5$ nicht $NR^8R^9$ oder Halogen ist, wenn $R^4$ Halogen oder $NR^8R^9$ ist.

$R^6$ Wasserstoff, Halogen, $(C_1-C_3)$Alkyl, $(C_1-C_3)$Alkoxy, -CN, $-NO_2$, $-CO-R^{11}$, $CO_2R^{12}$, $(C_1-C_3)$Alkylthio, $-SO-R^{12}$, $-SO_2-R^{12}$ oder $-CO-NR^8R^9$;

$R^8$ Wasserstoff oder $(C_1-C_4)$Alkyl oder $R^8$ und $R^9$ zusammen $-(CH_2)_2(CH_2)_c(CH_2)_2-$ oder $-(CH_2)_2O(CH_2)_2-$;

$R^9$ Wasserstoff, $(C_1-C_4)$Alkyl, $(C_1-C_4)$Alkoxy, $(C_2-C_4)$Alkenyl oder $R^8$ und $R^9$ zusammen $-(CH_2)_2(CH_2)_c(CH_2)_2-$ oder $-(CH_2)_2O(CH_2)_2-$;

$R^{10}$ Wasserstoff, $(C_1-C_4)$Alkyl, das unsubstituiert oder ein- oder mehrfach durch Halogen oder ein- bis zweifach durch CN, $CO_2-R^{12}$, $-NR^8R^9$, $OR^{12}$ oder $Si(CH_3)_3$ substituiert ist; $(C_3-C_4)$Alkinyl oder $(C_3-C_4)$Alkenyl, wobei die zwei vorgenannten Reste unsubstituiert oder durch $CH_3$ oder $-Si(CH_3)_3$ substituiert sind; $(C_3-C_6)$Cycloalkyl, das unsubstituiert oder durch $(C_1-C_4)$Alkyl oder $(C_1-C_4)$Alkoxy substituiert ist; oder $Si(CH_3)_3$;

$R^{11}$ Wasserstoff, $(C_1-C_4)$Alkyl, $(C_1-C_4)$Alkyl, das ein- oder mehrfach durch Halogen oder $(C_1-C_3)$Alkoxy substituiert ist; $(C_3-C_6)$Cycloalkyl, das unsubstituiert oder durch Halogen oder $CH_3$ ein- oder mehrfach substitutiert ist, $(C_2-C_4)$Alkenyl, $(C_2-C_4)$Alkinyl;

$R^{12}$ $(C_1-C_3)$Alkyl; und

c 0, 1 oder 2 bedeuten, ausgenommen die Verbindungen der Formel (II'), in denen

X' = $-CO_2H$ oder $CO_2CH_3$, $R^4$ = $OCH_3$, $R^5$ = $OCH_3$ und $R^6$ = H;

X' = $CO_2CH_3$, $R^4 = R^5$ = $OCH_3$ und $R^6$ = I;

X' = COOH, $CO-OCH_3$ oder $CO-OC_2H_5$, $R^4 = R^5$ = $CH^3$ und $R^6$ = H;

X' = $CO-OC_2H_5$, $R^4$ = $CH_3$, $R^5$ = Cl und $R^6$ = H oder Fluor;

X' = $CO-OCH_3$ oder COOH, $R^4$ = Cl $R^5$ = $CH_3$ und $R^6$ = H;

X' = $CO-OCH_3$ oder COOH, $R^4$ = H, $R^5$ = $CH_3$ oder Cl und $R^6$ = H;

oder X' = COOH, $R^4$ = $R^6$ = H und $R^5$ = $OCH_3$

bedeuten.

**13.** Verwendung der Verbindungen nach Anspruch 12 zur Herstellung von Herbiziden, Pflanzenwachstumsregulatoren oder Fungiziden.

**14.** Verbindungen der Formel III,

$R^1 - NH - SO_2 - (X)_a - L$ (III)

worin
$R^1$ = H, $(C_1\text{-}C_4)$Alkyl, $(C_2\text{-}C_6)$Alkenyl oder $(C_2\text{-}C_6)$Alkinyl,
X = $CH_2$ und a = 1 bedeuten und L = L5 nach Anspruch 1 oder 2 bedeutet, und ihre Alkali- oder Erdalkalimetallsalze.

**15.** Verbindungen und ihre Salze flach Anspruch 14, dadurch gekennzeichnet, daß $R^1$ = H, X = $CH_2$, a = 1 und L ein Rest der Formel

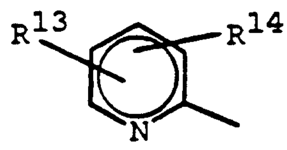

worin
$R^{13}$ und $R^{14}$ die in Anspruch 1 definierte Bedeutung haben, sind.

## Claims

**1.** A compound of the formula I or its salts

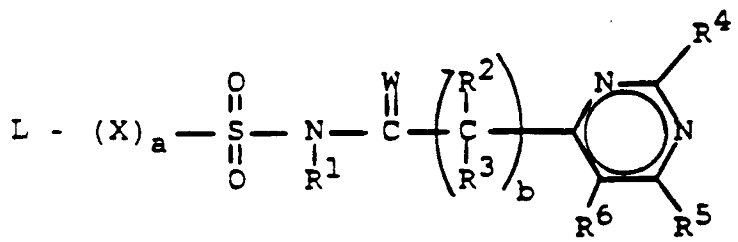

(I)

in which

| | |
|---|---|
| $R^1$ | is hydrogen, $(C_1\text{-}C_4)$alkyl, $(C_2\text{-}C_6)$alkenyl or $(C_2\text{-}C_6)$alkynyl; |
| $R^2$, $R^3$ | independently of one another are hydrogen, $(C_1\text{-}C_3)$alkyl or phenyl; |
| W | is O, S, $NR^7$ or $NOR^7$; |
| X | is $CHR^2$, O, $NR^7$ or $NOR^7$; |
| $R^4$, $R^5$ | independently of one another are hydrogen, hydroxyl, halogen, $(C_1\text{-}C_6)$alkyl, $(C_1\text{-}C_6)$-alkoxy or $(C_1\text{-}C_6)$alkylthio, where the three abovementioned radicals can be monosubstituted or polysubstituted by halogen or monosubstituted or disubstituted by $(C_1\text{-}C_4)$alkoxy or $(C_1\text{-}C_4)$alkylthio; $-NR^8R^9$, $(C_3\text{-}C_6)$cycloalkyl, $-OCHR^8CO_2R^{10}$, $(C_2\text{-}C_5)$alkenyl, $(C_2\text{-}C_4)$alkynyl, $(C_3\text{-}C_5)$alkenyloxy or $(C_3\text{-}C_5)$alkynyloxy; |
| $R^6$ | is hydrogen, halogen, $(C_1\text{-}C_3)$alkyl, $(C_1\text{-}C_3)$alkoxy, -CN, $-NO_2$, $-CO\text{-}R^{11}$, $-CO_2R^{12}$, $(C_1\text{-}C_3)$alkylthio, $-SO\text{-}R^{12}$, $-SO_2\text{-}R^{12}$ or $-CO\text{-}NR^8R^9$; |
| $R^7$ | is hydrogen, $(C_1\text{-}C_3)$alkyl, $(C_1\text{-}C_3)$haloalkyl or phenyl; |
| $R^8$ | is hydrogen or $(C_1\text{-}C_4)$alkyl or $R^8$ and $R^9$ together are $-(CH_2)_2(CH_2)_c(CH_2)_2-$ or $-(CH_2)_2O(CH_2)_2-$; |
| $R^9$ | is hydrogen, $(C_1\text{-}C_4)$alkyl, $(C_1\text{-}C_4)$alkoxy, $(C_2\text{-}C_4)$ alkenyl or $R^8$ and $R^9$ together are $-(CH_2)_2(CH_2)_c(CH_2)_2-$ or $-(CH_2)_2O(CH_2)_2-$; |
| $R^{10}$ | is hydrogen, $(C_1\text{-}C_4)$alkyl which is unsubstituted, or monosubstituted or polysubstituted by halogen or monosubstituted or disubstituted by CN, $CO_2\text{-}R^{12}$, $-NR^8R^9$, $OR^{12}$ or $Si(CH_3)_3$; $(C_3\text{-}C_4)$alkynyl or $(C_3\text{-}C_4)$alkenyl, where the two abovementioned radicals are unsubstituted or substituted by $CH_3$ or $-Si(CH_3)_3$; $(C_3\text{-}C_6)$cycloalkyl which is unsubstituted or substituted by $(C_1\text{-}C_4)$alkyl or $(C_1\text{-}C_4)$alkoxy; or $Si(CH_3)_3$; |
| $R^{11}$ | is hydrogen, $(C_1\text{-}C_4)$alkyl, $(C_1\text{-}C_4)$alkyl which is monosubstituted or polysubstituted by halogen or $(C_1\text{-}C_3)$alkoxy; $(C_3\text{-}C_6)$cycloalkyl which is unsubstituted, or monosubstituted or polysubstituted by halogen or $CH_3$; $(C_2\text{-}C_4)$alkenyl or $(C_2\text{-}C_4)$alkynyl; |

| | |
|---|---|
| $R^{12}$ | is $(C_1\text{-}C_3)$alkyl; |
| L | is a hetero- or isocyclic radical of the formulae (L1) to (L5) |

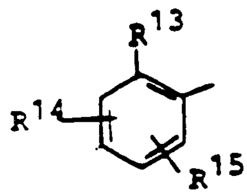

(L1)

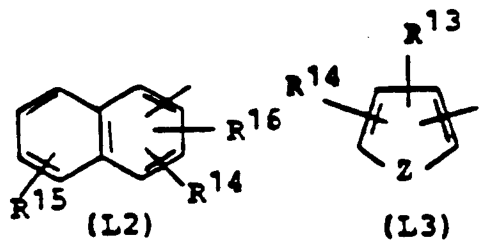

(L2) (L3)

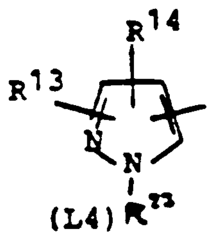

(L4)

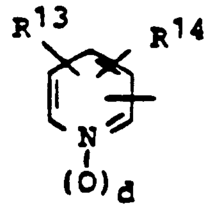

(L5)

| | |
|---|---|
| $R^{13}$ | is hydrogen, halogen, $NO_2$, CN, $(C_1\text{-}C_4)$alkyl which is unsubstituted, or monosubstituted or polysubstituted by halogen, or monosubstituted by CN, $OCH_3$ or $SCH_3$; $(C_2\text{-}C_4)$alkenyl which is unsubstituted, or monosubstituted or polysubstituted by halogen, or monosubstituted by $OCH_3$; $(C_2\text{-}C_4)$alkynyl which is unsubstituted, or monosubstituted or polysubstituted by halogen, or monosubstituted by OCH, or $Si(CH_3)_3$; $(C_3\text{-}C_6)$cycloalkyl which is unsubstituted, or monosubstituted or polysubstituted by halogen or $CH_3$; $-CO-R^{11}$, $-OCH_2CH_2OR^{11}$, -OH, $-C(R^{11})(OR^{17})(OR^{18})$; $-CO_2R^{10}$, $-CO-NR^8R^9$, $-N_3$, $SO_2NR^8R^9$, $-SO_3R^{19}$, $-OSO_2R^{20}$; phenyl which is unsubstituted, or monosubstituted or polysubstituted by halogen, $CH_3$ or $OCH_3$; $E-R^{21}$ or $-(CH_2)_e-G$; |
| $R^{14}$ | is hydrogen, halogen; CN, $NO_2$, $(C_1\text{-}C_4)$alkyl which is unsubstituted, or monosubstituted or polysubstituted by halogen or monosubstituted or disubstituted by $-CO_2R^{10}$, $-SO_2NR^8R^9$, $-NR^8R^9$, $(C_1\text{-}C_2)$alkoxy, $-E-R^{22}$, $(C_1\text{-}C_2)$haloalkoxy, $(C_1\text{-}C_2)$-alkylthio, $(C_1\text{-}C_2)$haloalkylthio, -CN, -OH or SH; $-CO_2R^{10}$, $-SO_2NR^8R^9$, $-NR^8R^9$ or $-E-R^{22}$; |
| $R^{15}$ | is hydrogen, $(C_1\text{-}C_4)$alkyl, $(C_1\text{-}C_4)$haloalkyl, $(C_2\text{-}C_4)$alkenyl, phenyl or phenyl which is monosubstituted or polysubstituted by halogen or $-E-R^{22}$; $-E-R^{22}$ or halogen; |
| $R^{16}$ | is hydrogen, $(C_1\text{-}C_4)$alkyl, $(C_1\text{-}C_4)$haloalkyl, halogen, $-CO_2R^{10}$, $-SO_2NR^8R^9$, $-OSO_2R^{20}$, $-E-R^{22}$, -CN or $-NO_2$; |
| E, Z | independently of one another are O or $S(O)_f$; |
| a, b, c, d, e | independently of one another are 0 or 1; |
| f | is 0, 1 or 2; |
| $R^{17}$, $R^{18}$ | independently of one another are $(C_1\text{-}C_4)$alkyl, or $R^{17}$ and $R^{16}$ together are $-CH_2CH_2-$, $-CH_2OCH_2-$ or $-CH_2-C(CH_3)_2CH_2-$; |
| $R^{19}$ | is $(C_1\text{-}C_4)$alkyl or $(C_1\text{-}C_4)$haloalkyl, |
| $R^{20}$ | is $(C_1\text{-}C_4)$alkyl, $-NR^8R^9$ or $(C_1\text{-}C_4)$haloalkyl, |
| $R^{21}$ | is $(C_1\text{-}C_4)$alkyl, $(C_1\text{-}C_4)$haloalkyl, $(C_2\text{-}C_4)$alkoxyalkyl, $(C_2\text{-}C_4)$alkenyl, $(C_3\text{-}C_4)$alkynyl, phenyl or phenyl which is monosubstituted or polysubstituted by halogen, $(C_1\text{-}C_3)$-alkyl, $(C_1\text{-}c_3)$alkoxy or $(C_1\text{-}C_3)$haloalkyl; or $(C_2\text{-}C_4)$haloalkenyl; |
| $R^{22}$ | is $(C_1\text{-}C_4)$alkyl or $(C_1\text{-}C_4)$alkyl which is monosubstituted or polysubstituted by halogen or monosubstituted by $OR^{19}$; |
| $R^{23}$ | is hydrogen, $(C_1\text{-}C_4)$alkyl which is unsubstituted, or monosubstituted or polysubstituted by halogen or monosubstituted by phenyl, or $(C_2\text{-}C_4)$alkenyl, phenyl or phenyl which is monosubstituted or polysubstituted by halogen, $(C_1\text{-}C_4)$alkyl, $(C_1\text{-}C_4)$haloalkyl, $-NO_2$, -CN or $(C_1\text{-}C_4)$alkoxy, |

G is a heterocyclic radical from the group comprising (G1)-(G25);

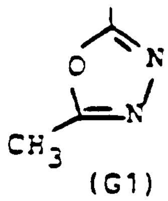
(G1)

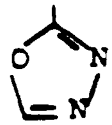
(G2)

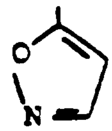
(G3)

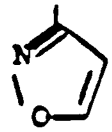
(G4)

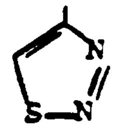
(G5)

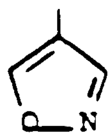
(G6)

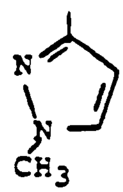
(G7)

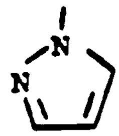
(G8)

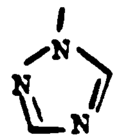
(G9)

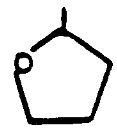
(G10)

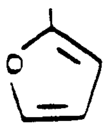
(G11)

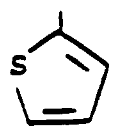
(G12)

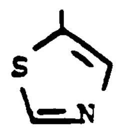
(G13)

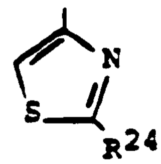
(G14)

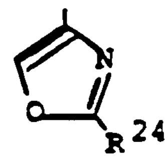
(G15)

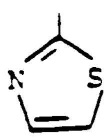
(G16)

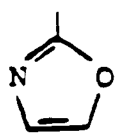
(G17)

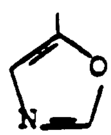
(G18)

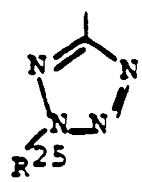
(G19)

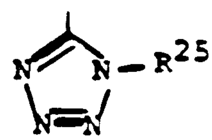
(G20)

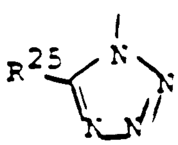
(G21)

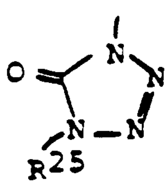
(G22)

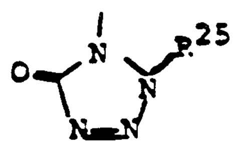
(G23)

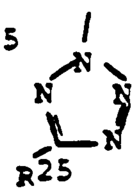
(G24)

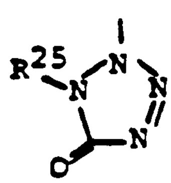
(G25)

and

$R^{24}$ is hydrogen or ($C_1$-$C_4$)alkyl;

$R^{25}$ is hydrogen, ($C_1$-$C_3$)alkyl or ($C_2$-$C_4$)alkenyl.

**2.** A compound or its salts as claimed in claim 1, wherein

L is a radical of the formulae (L1), (L3), (L4) or (L5);

X is $CH_2$, $CH(CH_3)$, O, NH, NCH,, $NC_2H_5$, $NOCH_3$, in particular $CH_2$, O or NH;

W is oxygen;

$R^1$ is hydrogen;

| | |
|---|---|
| $R^2$, $R^3$ | independently of one another are hydrogen or $(C_1-C_3)$alkyl, in particular hydrogen; |
| $R^4$, $R^5$ | independently of one another are hydrogen, $(C_1-C_2)$alkyl, $(C_1-C_2)$alkoxy, $-OCH_2OCH_3$, $-CH_2OCH_3$, F, Cl, Br, I, $-CH_2OCH_2CH_3$, $-NHCH_3$, $-N(CH_3)_2$, -N-$(-CH_3)OCH_3$, $-CF_3$, $-SCH_3$, $-CH(OCH_3)_2$, $-OCH_2CH=CR_2$, $-OCH_2C{\equiv}CH$, $-OCH_2-CH_2OCH_3$, $-CH_2SCH_3$, $-OCHF_2$, $-SCH-F_2$, cyclopropyl, $-C{\equiv}CH$ or $-C{\equiv}C-CH_3$; |
| $R^6$ | is hydrogen, halogen, $-CH_3$, $-OCH_3$, $-NO_2$, -CN, -CHO, $-CO_2CH_3$, $CO_2C_2R_5$ or $-SCH_3$; |
| $R^8$ | is hydrogen or $-CH_3$ or $R^8$ and $R^9$ together are $-(CH_2)_4-$, $-(CH_2)_5-$ or $-(CH_2)_2O(CH_2)_2-$; |
| $R^9$ | is $-CH_3$, $-CH_2CH_3$, $-OCH_3$ or $R^8$ and $R^9$ together are $-(CH_2)_4-$, $-(CH_2)_5-$ or $-(CH_2)_2O(CH_2)_2-$; |
| $R^{10}$ | is $(C_1-C_3)$alkyl, $(C_3)$alkenyl, $(C_3)$alkynyl, $-CH_2CH_2F$, $-CH_2CH_2Cl$, $-CH_2CH_2OCH_3$, $-CH_2CH_2Si(CH_3)_3$ or cyclopropylmethyl; |
| $R^{11}$ | is $(C_1-C_3)$alkyl, hydrogen, cyclopropyl or $(C_3)$alkenyl; |
| $R^{12}$ | is $-CH_3$, $-CH_2CH_3$, $-CH_2CH_2CH_3$ or $-CH(CH_3)_2$; |
| $R^{13}$ | is halogen, $-NO_2$, -CN, $(C_1-C_3)$alkyl which is unsubstituted or substituted by F, Cl, Br, CN, $OCH_3$ or $SCH_3$; $(C_3)$alkenyl which is unsubstituted or substituted by F, Cl or Br, $(C_3)$alkynyl, cyclopropyl which is unsubstituted or substituted by F, Cl or $CH_3$; $-C(O)R^{11}$, $-OCH_2CH_2OR^{11}$, -OH, $C(R^{11})(OR^{17})(OR^{18})$, $-CO_2R^{10}$, $-CO-NR^8R^9$, $-N_3$, $-SO_2NR^8R^9$, $-OSO_2R^{20}$, $-E-R^{21}$ or $-(CR_2)_eG$; |
| $R^{14}$ | is hydrogen, halogen, -CN, $-NO_2$, $-CH_3$, $-CF_3$, $-E-R^{22}$, or $(C_1-C_2)$alkoxy-$(C_1-C_2)$alkyl, $-CO_2R^{10}$ or $SO_2NR^8R^9$; |
| $R^{15}$ | is hydrogen; |
| $R^{17}$, $R^{18}$ | independently of one another are $(C_1-C_2)$alkyl or $R^{17}$ and $R^{18}$ together are $-CH_2CH_2-$; |
| $R^{20}$ | is $(C_1-C_3)$alkyl or $(C_1-C_3)$haloalkyl; |
| $R^{21}$ | is $(C_1-C_3)$alkyl, $(C_1-C_3)$haloalkyl, $(C_2-C_3)$alkoxyalkyl, allyl, propargyl or $(C_2-C_3)$haloalkenyl; |
| $R^{22}$ | is $(C_1-C_2)$alkyl which is unsubstituted or substituted by F, Cl or $OCH_3$; |
| $R^{23}$ | is hydrogen, $(C_1-C_3)$alkyl, phenyl or phenyl which is monosubstituted or polysubstituted by halogen, $-NO_2$, $(C_1-C_3)$alkyl, $(C_1-C_3)$haloalkyl or $(C_1-C_3)$-alkoxy; |
| $R^{24}$ | is hydrogen or $(C_1-C_3)$alkyl; |
| $R^{25}$ | is hydrogen or $(C_1-C_3)$alkyl; |
| Z | is S; |
| d | is 0; |
| e | is 0; |
| and E, a, b, f and G | are as defined in claim 1. |

**3.** A compound or its salts as claimed in claim 1 or 2, wherein $R^4$ and $R^5$ independently of one another are hydrogen, $CH_3$, $OCH_3$, $CH_2CH_3$, $OCH_2CH_3$, $OCHF_2$, $OCH_2OCH_3$, $CH_2OCH_3$, $NHCH_3$, $CH(OCH_3)_2$, Cl or cyclopropyl.

**4.** A compound or its salts as claimed in any one of claims 1 to 3, wherein L is an isocyclic radical of the formula L1.

**5.** A process for the preparation of a compound or its salts as defined in claim 1, wherein
a) for the preparation of compounds where W = O

$a_1$) a compound of the formula II

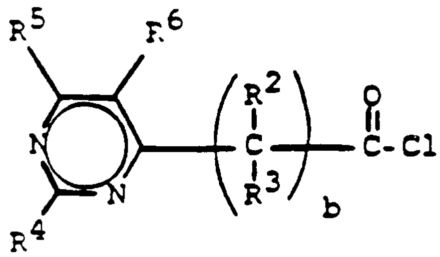

(II)

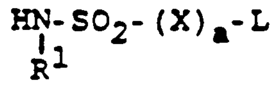

(III)

is reacted with a compound of the formula III in the presence of a base or
$a_2$) a compound of the formula (IIa)

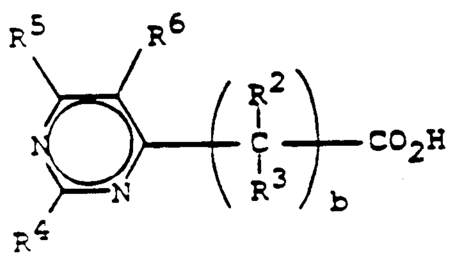

(IIa)

is reacted in the presence of activating reagents and optionally in the presence of a base with a compound of the formula III or
$a_3$) for the preparation of compounds where W = O and $R^1$ = H, a compound of the formula (V)

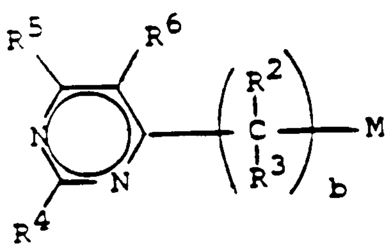

(V)

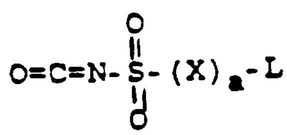

(VI)

in which M is hydrogen (for b = 0) or lithium is reacted with a compound of the formula (VI) or
b) for the preparation of compounds where W = S, a compound of the formula I obtained under a) is reacted with $P_4S_{10}$ (VIIa) or the compound of the formula VIIb

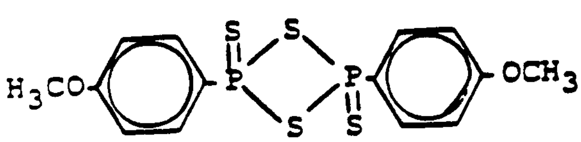

(VIIb)

c) for the preparation of compounds where W = $NR^7$ and $R^1$ = H, a compound of the formula VIII

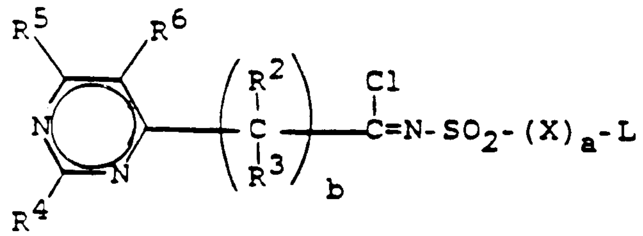

(VIII)

is reacted with an amine of the formula $H_2N\text{-}R^7$,

d) for the preparation of compounds where W = $NOR^7$ and $R^1$ = H

$d_1$) when $R^7$ = H a compound of the formula IX

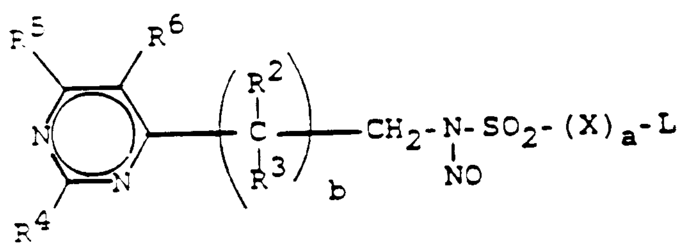

(IX)

is reacted with an alkali metal or alkaline earth metal hydroxide or ammonium hydroxide or

$d_2$) when $R^7$ is as defined in claim 1 a compound of the formula VIII is reacted in the presence of a base with a hydroxylamine of the formula $H_2NOR^7$ or

e) for the preparation of compounds of the formula I in which $R^1$ is not equal to hydrogen, a compound of the formula I where $R^1$ = H is reacted in the presence of a base with an alkyl halide of the formula $R^{1'}\text{-}X^1$, in which $R^{1'}$ has the meaning of $R^1$ apart from hydrogen and $X^1$ is chlorine, bromine or iodine.

**6.** The use of a compound of the formula I or its salts as defined in one or more of claims 1 to 4 as a herbicide or growth regulator.

**7.** A process for controlling undesired plants or regulating the growth of plants, which comprises applying an effective amount of a compound of the formula I or its salt as claimed in one or more of claims 1 to 4 to these plants or the cultivation soil.

**8.** A herbicidal or plant growth-regulating agent which contains a compound of the formula I or its salt as claimed in one or more of claims 1 to 4 and formulation auxiliaries.

**9.** The use of a compound of the formula I or its salts as defined in one or more of claims 1 to 4 as a fungicide.

**10.** A fungicidal agent which contains an effective amount of a compound of the formula I or its salt as claimed in one or more of claims 1 to 4 and formulation auxiliaries.

**11.** A process for controlling harmful fungi, which comprises applying a fungicidally effective amount of a compound of the formula I or its salt as claimed in one or more of claims 1 to 4 to the plants, surfaces or substrates attacked by these fungi.

**12.** A compound of the formula (II')

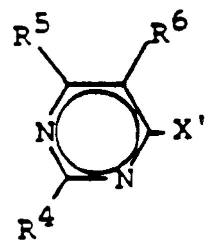

(II')

in which

X' is -CO-halogen, $-CO_2R^{10}$, $COO^-Me^+$, where $Me^+$ is the equivalent of an alkali metal or alkaline earth metal, or $CO_2CH_2C_6H_5$,

$R^4$ is hydrogen, hydroxyl, halogen, $(C_1-C_6)$alkyl, $(C_1-C_6)$alkoxy or $(C_1-C_6)$alkylthio, where the three abovementioned radicals can be monosubstituted or polysubstituted by halogen or monosubstituted or disubstituted by $(C_1-C_4)$alkoxy or $(C_1-C_4)$alkylthio; $-NR^8R^9$, $(C_3-C_6)$-cycloalkyl, $-OCHR^8CO_2R^{10}$, $(C_2-C_5)$alkenyl, $(C_2-C_4)$alkynyl, $(C_3-C_5)$alkenyloxy or $(C_3-C_5)$-alkynyloxy,
where $R^4$ is not $NR^8R^9$ or halogen if $R^5$ is halogen or $NR^8R^9$,

$R^5$ is hydroxyl, halogen, $(C_1-C_6)$alkyl, $(C_1-C_6)$alkoxy or $(C_1-C_6)$alkylthio, where the three abovementioned radicals can be monosubstituted or polysubstituted by halogen or monosubstituted or disubstituted by $(C_1-C_4)$alkoxy or $(C_1-C_4)$alkylthio; $-NR^8R^9$, $(C_3-C_6)$-cycloalkyl, $-OCHR^8CO_2R^{10}$, $(C_2-C_5)$alkenyl, $(C_2-C_4)$alkynyl, $(C_3-C_5)$alkenyloxy or $(C_3-C_5)$-alkynyloxy,
where $R^5$ is not $NR^8R^9$ or halogen if $R^4$ is halogen or $NR^8R^9$,

$R^6$ is hydrogen, halogen, $(C_1-C_3)$alkyl, $(C_1-C_3)$alkoxy, -CN, $-NO_2$, $-CO-R^{11}$, $-CO_2R^{12}$, $(C_1-C_3)$-alkylthio, $-SO-R^{12}$, $-SO_2-R^{12}$ or $-CO-NR^8R^9$;

$R^8$ is hydrogen or $(C_1-C_4)$alkyl or $R^8$ and $R^9$ together are $-(CH_2)_2(CH_2)_c(CH_2)_2-$ or $-(CH_2)_2O-(CH_2)_2-$;

$R^9$ is hydrogen, $(C_1-C_4)$alkyl, $(C_1-C_4)$alkoxy, $(C_2-C_4)$alkenyl or $R^8$ and $R^9$ together are $-(CH_2)_2-(CH_2)_c(CH_2)_2-$ or $-(CH_2)_2O(CH_2)_2-$;

$R^{10}$ is hydrogen, $(C_1-C_4)$alkyl which is unsubstituted, or monosubstituted or polysubstituted by halogen or monosubstituted or disubstituted by CN, $CO_2-R^{12}$, $-NR^8R^9$, $OR^{12}$ or $Si(CH_3)_3$; $(C_3-C_4)$alkynyl or $(C_3-C_4)$alkenyl, where the two abovementioned radicals are unsubstituted or substituted by CH, or $-Si(CH_3)_3$; $(C_3-C_6)$cycloalkyl which is unsubstituted or substituted by $(C_1-C_4)$alkyl or $(C_1-C_4)$alkoxy; or $Si(CH_3)_3$;

$R^{11}$ is hydrogen, $(C_1-C_4)$alkyl, $(C_1-C_4)$alkyl which is monosubstituted or polysubstituted by halogen or $(C_1-C_3)$alkoxy; $(C_3-C_6)$cycloalkyl which is unsubstituted, or monosubstituted or polysubstituted by halogen or $CH_3$; $(C_2-C_4)$alkenyl or $(C_2-C_4)$alkynyl;

$R^{12}$ is $(C_1-C_3)$alkyl;
and

c is 0, 1 or 2, excluding the compounds of the formula (II') in which

X' = $-CO_2H$ or $CO_2CH_3$, $R^4 = OCH_3$, $R^5 = OCH_3$ and $R^6 = H$;
X' = $CO_2CH_3$, $R^4 = R^5 = OCH_3$ and $R^6 = I$;
X' = COOH, $CO-OCH_3$ or $CO-OC_2H_5$, $R^4 = R^5 = CH_3$ and $R^6 = H$;
X' = $CO-OC_2H_5$, $R^4 = CH_3$, $R^5 = Cl$ and $R^6 = H$ or fluorine;
X' = $CO-OCH_3$ or COOH, $R^4 = Cl$, $R^5 = CH_3$ and $R^6 = H$;
X' = $CO-OCH_3$ or COOH, $R^4 = H$, $R^5 = CH_3$ or Cl and $R^6 = H$; or
X' = COOH, $R^4 = R^6 = H$ and $R^5 = OCH_3$.

**13.** The use of a compound as claimed in claim 12 for the production of herbicides, plant growth regulators or fungicides.

**14.** A compound of the formula III,

$R^1$ - NH - $SO_2$ - $(X)_a$ - L (III)

in which
$R^1$ = H, $(C_1\text{-}C_4)$alkyl, $(C_2\text{-}C_6)$alkenyl or $(C_2\text{-}C_6)$alkynyl,
X = $CH_2$ and a = 1 and L = L5 as claimed in claim 1 or 2, and its alkali metal or alkaline earth metal salts.

**15.** A compound or its salts as claimed in claim 14, wherein $R^1$ = H, X = $CH_2$, a = 1 and L is a radical of the formula

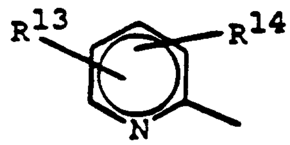

in which
$R^{13}$ and $R^{14}$ have the meaning defined in claim 1.

**Revendications**

**1.** Composés de formule I ou leurs sels

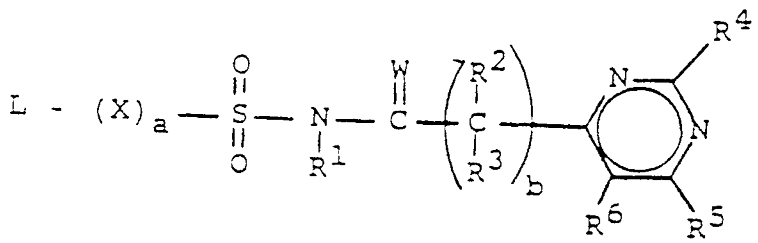

(I)

où

| | |
|---|---|
| $R^1$ | représente l'hydrogène, les alkyle en $(C_1\text{-}C_4)$,alcényle en $(C_2\text{-}C_6)$ ou alcynyle en $(C_2\text{-}C_6)$ ; |
| $R^2$, $R^3$ | représentent indépendamment l'un de l'autre, l'hydrogène, les alkyle en $(C_1\text{-}C_3)$ ou phényle ; |
| W | représente O, S, $NR^7$ ou $NOR^7$ ; |
| X | représente $CHR^2$, O, $NR^7$ ou $NOR^7$ ; |
| $R^4$, $R^5$ | représentent, indépendamment l'un de l'autre, l'hydrogène, les hydroxy, halogène, alkyle en $(C_1\text{-}C_6)$, alcoxy en $(C_1\text{-}C_6)$ ou alkylthio en $(C_1\text{-}C_6)$, les trois radicaux précités pouvant être substitués une ou plusieurs fois par des halogènes ou une ou deux fois par des alcoxy en $(C_1\text{-}C_4)$ ou des alkylthio en $(C_1\text{-}C_4)$ ; $-NR^8R^9$, cycloalkyle en $(C_3\text{-}C_6)$, $-OCHR^8CO_2R^{10}$, alcényle en $(C_2\text{-}C_5)$, alcynyle en $(C_2\text{-}C_4)$, alcényloxy en $(C_3\text{-}C_5)$ ou alcynyloxy en $(C_3\text{-}C_5)$ ; |
| $R^6$ | représente l'hydrogène, les halogènes, alkyle en $(C_1\text{-}C_3)$, alcoxy en $(C_1\text{-}C_3)$, -CN, $-NO_2$, $-CO\text{-}R^{11}$, $-CO_2R^{12}$, alkylthio en $(C_1\text{-}C_3)$, $-SO\text{-}R^{12}$, $-SO_2\text{-}R^{12}$ ou $-CO\text{-}NR^8R^9$ ; |
| $R^7$ | représente l'hydrogène, les alkyle en $(C_1\text{-}C_3)$, halogènoalkyle en $(C_1\text{-}C_3)$ ou phényle ; |
| $R^8$ | représente l'hydrogène ou les alkyle en $(C_1\text{-}C_4)$ ou $R^8$ et $R^9$ ensemble représentent $-(CH_2)_2(CH_2)_c(CH_2)_2$-ou $-(CH_2)_2O(CH_2)_2$- ; |
| $R^9$ | représente l'hydrogène, les alkyle en $(C_1\text{-}C_4)$, alcoxy en $(C_1\text{-}C_4)$, alcényle en $(C_2\text{-}C_4)$ ou $R^8$ et $R^9$ ensemble $-(CH_2)_2(CH_2)_c(CH_2)_2$- ou $-(CH_2)_2O(CH_2)_2$- ; |

$R^{10}$ représente l'hydrogène, alkyle en ($C_1$-$C_4$), qui peut être non substitué ou substitué une ou plusieurs fois par les halogènes ou une à deux fois par CN, $-CO_2-R^{12}$, $-NR^8R^9$, $OR^{12}$ ou $Si(CH_3)_3$ ; alcynyle en ($C_3$-$C_4$) ou alcényle en ($C_3$-$C_4$), les deux radicaux précités étant non substitués ou substitués par $CH_3$ ou $-Si(CH_3)_3$ ; cycloalkyle en ($C_3$-$C_6$) qui est non substitué ou substitué par des alkyle en ($C_1$-$C_4$) ou alcoxy en ($C_1$-$C_4$) ; ou représente $-Si(CH_3)_3$ ;

$R^{11}$ représente l'hydrogène, les alkyle en ($C_1$-$C_4$),alkyle en ($C_1$-$C_4$) qui est substitué une ou plusieurs fois par les halogène ou alcoxy en ($C_1$-$C_3$) ; cycloalkyle en ($C_3$-$C_6$), qui est non substitué ou qui est substitué une ou plusieurs fois par les halogènes comme par exemple F, Cl et Br, ou $CH_3$, ou représente aussi les alcényle en ($C_2$-$C_4$), alcynyle en ($C_2$-$C_4$) ;

$R^{12}$ représente un alkyle en ($C_1$-$C_3$) ;

L représente un radical hétéro- ou isocyclique des formules (L1) à (L5)

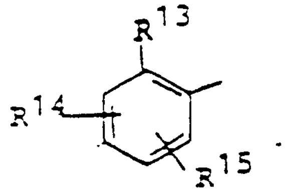

(L1)

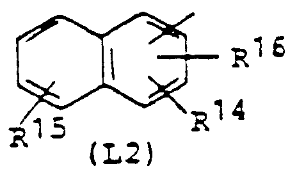

(L2)

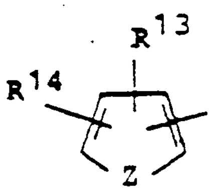

(L3)

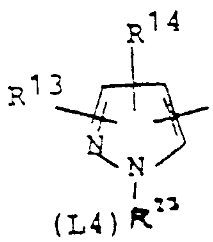

(L4)

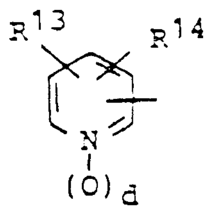

(L5)

$R^{13}$ représente l'hydrogène, les halogènes, $NO_2$, CN, alkyle en ($C_1$-$C_4$) qui est non substitué ou une ou plusieurs fois substitué par les halogènes, comme par exemple F, Cl et Br, ou une fois par CN, $OCH_3$, ou $SCH_3$ ; alcényle en ($C_2$-$C_4$) qui est non substitué ou substitué une ou plusieurs fois par des halogènes, comme par exemple F, Cl et Br, ou une fois par $OCH_3$ ; alcynyle en $C_2$-$C_4$) qui est non substitué ou substitué une ou plusieurs fois par les halogène, comme par exemple F, Cl et Br, ou une fois par $OCH_3$ ou $Si(CH_3)_3$ ; cycloalkyle en ($C_3$-$C_6$) qui est non substitué ou une ou plusieurs fois substitué par les halogènes, comme par exemple F et Cl, ou $CH_3$ ; $-CO-R^{11}$, $-OCH_2CH_2OR^{11}$, -OH, $-C(R^{11})(OR^{17})(OR^{18})$ ; $-CO_2R^{10}$, $-CO-NR^8R^9$, $-N_3$, $SO_2NR^8R^9$, $-SO_3R^{19}$, $-OSO_2R^{20}$ ; phényle non substitué ou substitué une ou plusieurs fois par les halogènes, comme par exemple F, Cl et Br, $CH_3$ ou $OCH_3$ ; $-E-R^{21}$ ou $-(CH_2)_e-G$;

$R^{14}$ représente l'hydrogène, un halogène ; CN, $NO_2$, les alkyle en ($C_1$-$C_4$) qui est non substitué une ou plusieurs fois par les halogènes ou une à deux fois par $-CO_2R^{10}$, $-SO_2NR^8R^9$, $-NR^8R^9$, alcoxy en ($C_1$-$C_2$), $-E-R^{22}$, halogénoalcoxy en ($C_1$-$C_2$), alkylthio en ($C_1$-$C_2$), halogénoalkylthio en ($C_1$-$C_2$), -CN, -OH ou SH ; $-CO_2R^{10}$, $-SO_2NR^8R^9$, $-NR^8R^9$ ou $-E-R^{22}$ ;

$R^{15}$ représente l'hydrogène, les alkyle en ($C_1$-$C_4$), halogénoalkyle en ($C_1$-$C_4$), alcényle en ($C_2$-$C_4$), phényle ou phényle qui est substitué une ou plusieurs fois par des halogènes ou $-E-R^{22}$ ; $-E-R^{22}$ ou un halogène ;

$R^{16}$ représente l'hydrogène, les alkyle en ($C_1$-$C_4$), halogénoalkyle en ($C_1$-$C_4$), halogène,

| | |
|---|---|
| | $-CO_2R^{10}$, $-SO_2NR^8R^9$, $-OSO_2R^{20}$, $-E-R^{22}$, -CN ou $-NO_2$ ; |
| E, Z | représentent indépendamment l'un de l'autre O ou $S(O)_f$ ; |
| a, b, c, d, e, | représentent indépendamment l'un de l'autre, 0 ou 1 ; |
| f | vaut 0, 1 ou 2 ; |
| $R^{17}$, $R^{18}$ | représentent indépendamment l'un de l'autre les alkyle en ($C_1$-$C_4$), ou $R^{17}$ et $R^{18}$ ensemble, représentent $-CH_2CH_2-$, $-CH_2OCH_2-$ ou $-CH_2-C(CH_3)_2CH_2-$ ; |
| $R^{19}$ | représente les alkyle en ($C_1$-$C_4$) ou halogénoalkyle en $C_1$-$C_4$ ; |
| $R^{20}$ | représente les alkyle en ($C_1$-$C_4$), $-NR^8R^9$ ou halogénoalkyle en ($C_1$-$C_4$) ; |
| $R^{21}$ | représente les alkyle en ($C_1$-$C_4$), halogénoalkyle en ($C_1$-$C_4$), alcoxyalkyle en ($C_2$-$C_4$), alcényle en ($C_2$-$C_4$), alcynyle en ($C_3$-$C_4$), phényle ou phényle qui est substitué une ou plusieurs fois par les halogènes, alkyle en ($C_1$-$C_3$), alcoxy en ($C_1$-$C_3$) ou halogénoalkyle en ($C_1$-$C_3$) ; halogénoalkyle en ($C_2$-$C_4$) ; |
| $R^{22}$ | représente les alkyle en ($C_1$-$C_4$) ou alkyle en ($C_1$-$C_4$) qui est substitué une plusieurs fois par les halogènes, comme par exemple F, Cl, ou une fois par $OR^{19}$ ; |
| $R^{23}$ | représente l'hydrogène, les alkyle en ($C_1$-$C_4$) qui est non substitué ou substitué une ou plusieurs fois par des halogènes ou une fois par le phényle, représente aussi les alcényle en ($C_2$-$C_4$), phényle ou phényle qui est substitué une ou plusieurs fois par les halogènes, alkyle en ($C_1$-$C_4$), halogénoalkyle en ($C_1$-$C_4$), $-NO_2$, -CN ou alcoxy en $C_1$-$C_4$ ; |
| G | représente un radical hétérocyclique pris dans le groupe des radicaux de formules (G1) à (G25) ; |

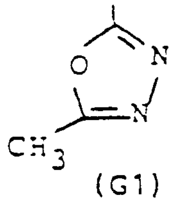
(G1)

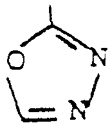
(G2)

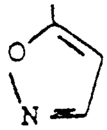
(G3)

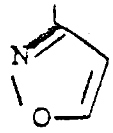
(G4)

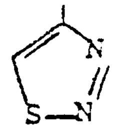
(G5)

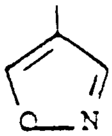
(G6)

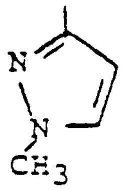
(G7)

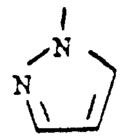
(G8)

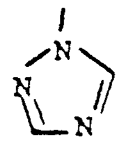
(G9)

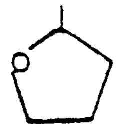
(G10)

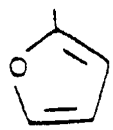
(G11)

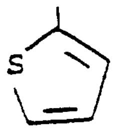
(G12)

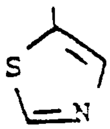
(G13)

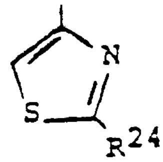
(G14)

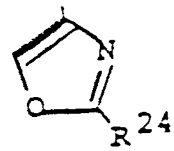
(G15)

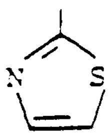
(G16)

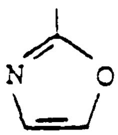
(G17)

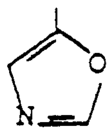
(G18)

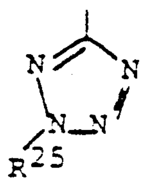
(G19)

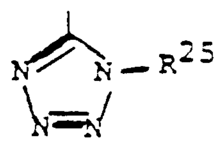
(G20)

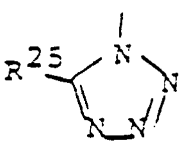
(G21)

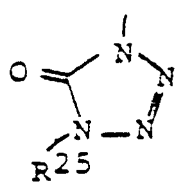
(G22)

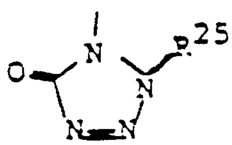
(G23)

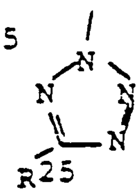
(G24)

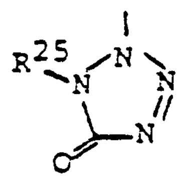
(G25)

et

$R^{24}$ représente l'hydrogène ou un alkyle en ($C_1$-$C_4$), et

$R^{25}$ représente l'hydrogène, les alkyle en ($C_1$-$C_3$) ou alcényle ($C_2$-$C_4$).

**2.** Composés ou leurs sels selon la revendication 1, caractérisés en ce que

| | |
|---|---|
| L | représente un radical de formules (L1), (L3), (L4) ou (L5) ; |
| X | représente $CH_2$, $CH(CH_3)$, O, NH, $NCH_3$, $NC_2H_5$, $NOCH_3$, notamment $CH_2$, O ou NH ; |
| W | représente l'oxygène ; |
| $R^1$ | représente l'hydrogène ; |
| $R^2$, $R^3$ | représente indépendamment l'un de l'autre l'hydrogène ou un alkyle en ($C_1$-$C_3$), plus particulièrement l'hydrogène ; |
| $R^4$, $R^5$ | représentent indépendamment l'un de l'autre l'hydrogène, les alkyle en ($C_1$-$C_2$), alcoxy en ($C_1$-$C_2$), $-OCH_2OCH_3$, $-CH_2OCH_3$, F, Cl, Br, I, $-CH_2OCH_2CH_3$, $-NHCH_3$, $-N(CH_3)_2$, $-N(CH_3)OCH_3$, $-CF_3$, $-SCH_3$, $-CH(OCH_3)_2$, $-OCH_2CH=CH_2$, $-OCH_2C\equiv CH$, $-OCH_2CH_2OCH_3$, $-CH_2SCH_3$, $-OCHF_2$, $-SCHF_2$, cyclopropyle, $-C\equiv CH$ ou $-C\equiv C-CH_3$ ; |
| $R^6$ | représente l'hydrogène, les halogènes, $-CH_3$, $-OCH_3$, $-NO_2$, -CN, -CHO, $-CO_2CH_3$, $CO_2C_2H_5$ ou $-SCH_3$, plus particulièrement l'hydrogène, les halogènes, |

| | |
|---|---|
| | $-NO_2$, -CN, -CHO, $-CO_2CH_3$ ou $-CO_2C_2H_5$ ; |
| $R^8$ | représente l'hydrogène ou $-CH_3$ ou $R_8$ et $R_9$ ensemble représentent $-(CH_2)_4-$, $-(CH_2)_5-$ ou $(CH_2)_2O(CH_2)_2-$ ; |
| $R^9$ | représente $-CH_3$, $-CH_2CH_3$, $-OCH_3$ ou $R^8$ et $R^9$ ensemble $-(CH_2)_4-$, $-(CH_2)_5-$ ou $-(CH_2)_2O(CH_2)_2-$ ; |
| $R^{10}$ | représente les alkyle en $(C_1-C_3)$, alcényle en $(C_3)$, alcynyle en $(C_3)$, $-CH_2CH_2F$, $-CH_2CH_2Cl$, $-CH_2CH_2OCH_3$, $-CH_2CH_2Si(CH_3)_3$ ou cyclopropylméthyle ; |
| $R^{11}$ | représente les alkyle en $(C_1-C_3)$ , hydrogène, cyclopropyle ou un alcényle en $(C_3)$ ; |
| $R^{12}$ | représente $-CH_3$, $-CH_2CH_3$, $-CH_2CH_2CH_3$ ou $-CH(CH_3)_2$ ; |
| $R^{13}$ | représente les halogène, $-NO_2$, -CN, alkyle en $(C_1-C_3)$ qui est non substitué ou substitué par F, Cl, Br, CN, $OCH_3$ ou $SCH_3$ ; alcényle en $(C_3)$ qui est non substitué ou substitué par F, Cl ou Br ; alcynyle en $(C_3)$, cyclopropyle qui est non substitué ou substitué par F, Cl ou $CH_3$ ; $-C(O)R^{11}$, $-OCH_2CH_2OR^{11}$, -OH, C-$(R^{11})(OR^{17})(OR^{18})$, $-CO_2R^{10}$, $-CO-NR^8R^9$, $-N_3$, $-SO_2NR^8R^9$, $-OSO_2R^{20}$, $-E-R^{21}$ ou $-(CH_2)_e-G$ ; |
| $R^{14}$ | représente l'hydrogène, halogène -CN, $-NO_2$, $-CH_3$, $-CF_3$, $-E-R^{22}$, ou alcoxy en $(C_1-C_2)$-alkyle en $(C_1-C_2)$, $-CO_2R^{10}$ ou $SO_2NR^8R^9$ ; |
| $R^{15}$ | représente l'hydrogène ; |
| $R^{17}$, $R^{18}$ | représentent indépendamment l'un de l'autre un alkyle en $(C_1-C_2)$ ou $R^{17}$ et $R^{18}$ ensemble représentent $-CH_2CH_2-$ ; |
| $R^{20}$ | représente les alkyle en $(C_1-C_3)$, halogénoalkyle en $(C_1-C_3)$ ; |
| $R^{21}$ | représente les alkyle en $(C_1-C_3)$, halogénoalkyle en $(C_1-C_3)$, alcoxyalkyle en $(C_2-C_3)$, allyle, propargyle, halogénoalkyle en $(C_1-C_3)$ ; |
| $R^{22}$ | représente un alkyle en $(C_1-C_2)$ qui est non substitué ou substitué par F, Cl ou $OCH_3$ ; |
| $R^{23}$ | représente l'hydrogène, les alkyle en $(C_1-C_3)$,phényle ou phényle qui est une ou plusieurs fois substitué par les halogènes, $-NO_2$, alkyle en $(C_1-C_3)$, halogénoalkyle en $(C_1-C_3)$ ou alcoxy en $(C_1-C_3)$ ; |
| $R^{24}$ | représente l'hydrogène ou un alkyle en $(C_1-C_3)$ ; |
| $R^{25}$ | représente l'hydrogène ou un alkyle en $(C_1-C_3)$ ; |
| Z | représente S ; |
| d | vaut 0 et |
| e | vaut 0 |
| et E, a, b, f et G | ont les définitions données ci-dessus. |

3. Composés ou leurs sels selon la revendication 1 ou 2, caractérisés en ce que $R^4$ et $R^5$ indépendamment l'un de l'autre représentent l'hydrogène, $CH_3$, $OCH_3$, $CH_2CH_3$, $OCH_2CH_3$, $OCHF_2$, $OCH_2CH_3$, $CH_2CH_3$, $NHCH_3$, $CH(OCH_3)_2$, Cl ou le cyclopropyle.

4. Composés ou leurs sels selon l'une des revendications 1 à 3, caractérisés en ce que L représente un radical isocyclique de formule L1.

5. Procédé pour la préparation des composés définis selon la revendication 1 ou de leurs sels, caractérisé en ce que

a) pour la préparation des composés avec W = O

$a_1$) on fait réagir un composé de formule II avec

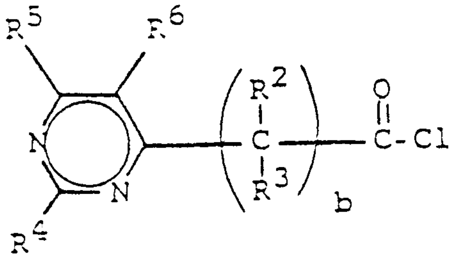

(II)

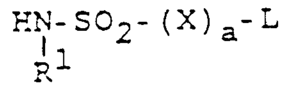

(III)

un composé de formule III en présence d'une base ou

$a_2$) un composé de formule IIa

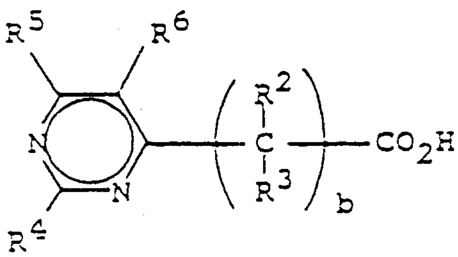

(IIa)

en présence de réactifs activateurs tels que le chlorure de 2-chloro-1-méthylpyridinium (IVa), le dicyclohexylcarbodiimide (IVb) ou le 1,1-carbonyldiimidazole (IVc) et éventuellement en présence d'une base avec un composé de formule III

ou

$a_3$) pour la préparation de composés avec W = O et $R^1$ = H, on fait réagir un composé de formule (V)

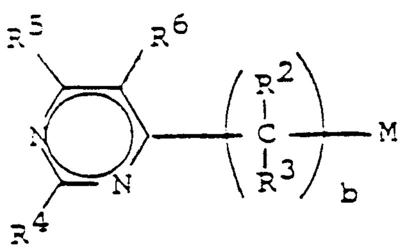

(V)

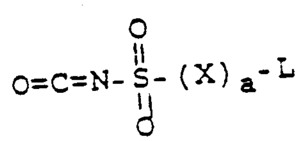

(VI)

où M représente l'hydrogène (pour b = 0) ou le lithium, avec un composé de formule (VI) ou

b) pour la préparation de composés avec W = S, on fait réagir un composé de formule I obtenu en a) avec $P_4S_{10}$ (VIIa) ou le composé de formule VIIb

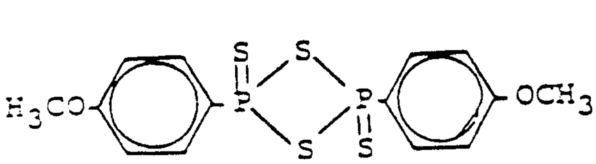

(VIIb)

c) pour la préparation de composés avec W = $NR^7$ et $R^1$ = H, on fait réagir un composé de formule VIII

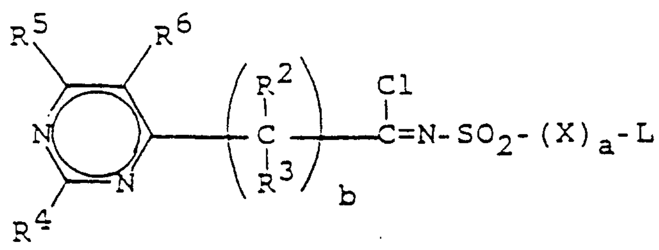

(VIII)

avec une amine de formule $H_2N-R^7$,

d) pour la préparation de composés avec W = $NOR^7$ et $R^1$ = H,

$d_1$) dans le cas où $R^7$ = H on fait réagir un composé de formule IX avec

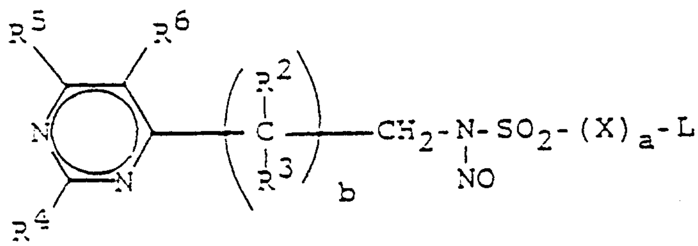

(IX)

un hydroxyde alcalin- ou alcalino-terreux ou l'hydroxyde d'ammonium ou

$d_2$) dans le cas où $R^7$ est défini comme dans la revendication 1, on fait réagir un composé de formule VIII en présence d'une base avec une hydroxylamine de formule $H_2NOR^7$ ou

e) pour la préparation de composés de formule I où $R^1$ n'est pas égal à l'hydrogène, on fait réagir un composé de formule I avec $R^1$ = H en présence d'une base avec un halogénoalkyle de formule $R^{1'}$-$X^1$, où $R^{1'}$ a la signification de $R^1$ à l'exception de l'hydrogène, et $X^1$ représente les chlore, brome ou iode.

**6.** Utilisation des composés de formule I définis selon une ou plusieurs des revendications 1 à 4 ou de leurs sels en tant qu'herbicides ou régulateurs de croissance.

**7.** Procédé pour la lutte contre la végétation non désirée ou pour la régulation de croissance des plantes, caractérisé en ce qu'on applique sur celles-ci ou sur les sols cultivables une quantité efficace d'un composé de formule I ou de ses sels selon une ou plusieurs des revendications 1 à 4.

**8.** Agent herbicide ou régulateur de croissance de plantes, caractérisé en ce qu'il contient un composé de formule I ou ses sels selon une ou plusieurs des revendications 1 à 4 et un agent auxiliaire de formulation.

**9.** Utilisation des composés de formule I définis selon une ou plusieurs des revendications 1 à 4 ou de leurs sels en tant que fongicides.

**10.** Agent fongicide caractérisé en ce qu'il contient une quantité efficace d'un composé de formule I ou de ses sels selon une ou plusieurs des revendications 1 à 4 et un agent auxiliaire de formulation.

**11.** Procédé pour la lutte contre les champignons pathogènes, caractérisé en ce qu'on applique sur les plantes, superficies ou substrats envahis de champignns une quantité efficace fongicide d'un composé de formule I ou de ses sels selon une ou plusieurs des revendications 1 à 4.

**12.** Composés de formule (II')

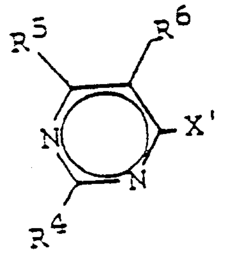

(II')

où

X' représente les -CO-halogène, $-CO_2R^{10}$, $COO^-Me^+$, où $Me^+$ représente l'équivalent d'un métal alcalin ou alcalino-terreux, ou $CO_2CH_2C_6H_5$ ;

$R^4$ représente l'hydrogène, les hydroxy, halogène, alkyle en ($C_1$-$C_6$), alcoxy en ($C_1$-$C_6$) ou alkylthio en ($C_1$-$C_6$), les trois radicaux précités pouvant être substitués une ou plusieurs fois par des halogènes ou une ou deux fois par des alcoxy en ($C_1$-$C_4$) ou alkylthio en ($C_1$-$C_4$) ; $-NR^8R^9$, cycloalkyle en ($C_3$-$C_6$), $-OCHR^8CO_2R^{10}$, alcényle en ($C_2$-$C_5$), alcynyle en ($C_2$-$C_4$), alcényloxy en ($C_3$-$C_5$) ou alcynyloxy en ($C_3$-$C_5$), $R^4$ n'étant pas $-NR^8R^9$ ou un halogène lorsque $R^5$ est un halogène ou $-NR^8R^9$ ;

$R^5$ représente les hydroxy, halogène, alkyle en ($C_1$-C), alcoxy en ($C_1$-$C_6$) ou alkylthio en ($C_1$-$C_6$), les trois radicaux précités pouvant être substitués une ou plusieurs fois par des halogènes, une ou deux fois par des alcoxy en ($C_1$-$C_4$) ou alkylthio en ($C_1$-$C_4$) ; $-NR^8R^9$, cycloalkyle en ($C_3$-$C_6$), $-OCHR^8CO_2R^{10}$, alcényle en ($C_2$-$C_5$), alcynyle en ($C_2$-$C_4$), alcényloxy en ($C_3$-$C_5$) ou alcynyloxy en ($C_3$-$C_5$), $R^5$ n'étant pas $-NR^8R^9$ ou un halogène lorsque $R^4$ est un halogéne ou $-NR^8R^9$ ;

$R^6$ représente l'hydrogène, les halogènes, alkyle en ($C_1$-$C_3$), alcoxy en ($C_1$-$C_3$), -CN, $-NO_2$, $-CO-R^{11}$, $-CO_2R^{12}$, alkylthio en ($C_1$-$C_3$), $-SO-R^{12}$, $-SO_2-R^{12}$ ou $-CO-NR^8-R^9$ ;

$R^8$ représente l'hydrogène ou alkyle en ($C_1$-$C_4$) ou $R^8$ et $R^9$ ensemble représentent $-(CH_2)_2-(CH_2)_c(CH_2)_2-$ ou $-(CH_2)_2O(CH_2)_2-$ ;

$R^9$ représente l'hydrogène, les alkyle en ($C_1$-$C_4$), alcoxy en ($C_1$-$C_4$), alcényle en ($C_2$-$C_4$) ou $R^8$ et $R^9$ ensemble représentent $-(CH_2)_2(CH_2)_c(CH_2)_2-$ ou $-(CH_2)_2O(CH_2)_2-$ ;

$R^{10}$ représente l'hydrogène, un alkyle en ($C_1$-$C_4$), qui est non substitué ou substitué une ou plusieurs fois par des halogènes ou une ou deux fois par CN, $CO_2-R^{12}$, $-NR^8R^9$, $OR^{12}$, ou $Si(CH_3)_3$ ; alcynyle en ($C_3$-$C_4$) ou alcényle en ($C_3$-$C_4$), les deux radicaux précités étant non substitués ou substitués par $CH_3$ ou $-Si(CH_3)_3$ ; cycloalkyle en $C_3$-$C_6$ qui est non substitué ou substitué par un alkyle en ($C_1$-$C_4$) ou alcoxy en ($C_1$-$C_4$) ; ou $-Si(CH_3)_3$ ;

$R^{11}$ représente l'hydrogène, les alkyle en ($C_1$-$C_4$),alkyle en ($C_1$-$C_4$) qui est substitué une ou plusieurs fois par des halogène ou alcoxy en ($C_1$-$C_3$) ; cycloalkyle en ($C_3$-$C_6$), qui est non substitué ou substitué une ou plusieurs fois par des halogènes ou $CH_3$, alcényle en ($C_2$-$C_4$),alcynyle en ($C_2$-$C_4$) ;

$R^{12}$ représente un alkyle en ($C_1$-$C_3$) ; et

c vaut 0, 1 ou 2, à l'exception des composés de formule (II') dans lesquels

X' = $-CO_2H$ ou $CO_2CH_3$, $R^4 = OCH_3$, $R^5 = OCH_3$ et $R^6 = H$ ;

X' = $CO_2CH_3$, $R^4 = R^5 = OCH_3$ et $R^6 = I$ ;

X' = COOH, $CO-OCH_3$ ou $CO-OC_2H_5$, $R^4 = R^5 = CH_3$ et $R^6 = H$ ;

X' = $CO-OC_2CH_5$, $R^4 = CH_3$, $R^5 = Cl$ et $R^6 = H$ ou fluor ;

X' = $CO-OCH_3$ ou COOH, $R^4 = Cl$, $R^5 = CH_3$ et $R^6 = H$ ;

X' = $CO-OCH_3$ ou COOH, $R^4 = H$, $R^5 = CH_3$ ou Cl et $R^6 = H$ ; ou

X' = COOH, $R^4 = R^6 = H$ et $R^5 = OCH_3$.

**13.** Utilisation des composés selon la revendication 12 pour la préparation d'herbicides, de régulateurs de croissance de plantes ou de fongicides.

**14.** Composé de formule III,

$R^1$ - NH - $SO_2$ - $(X)_a$ - L (III)

où
$R^1$ = H, alkyle en ($C_1$-$C_4$), alcényle en ($C_2$-$C_6$) ou alcynyle en ($C_2$-$C_6$), X = $CH_2$ et a = 1 et L = L5 selon la revendication 1 ou 2, et leurs sels de métaux alcalins ou alcalino-terreux.

**15.** Composés et leurs sels selon la revendication 14, caractérisés en ce que $R^1$ = H, X = $CH_2$, a = 1 et L représente un radical de formule

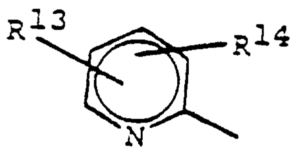

où
$R^{13}$ et $R^{14}$ ont la signification donnée dans la revendication 1.